# EUROPEAN PATENT SPECIFICATION

(11) **EP 0 639 584 B1**
(45) Date of publication and mention of the grant of the patent: **01.04.1998**
(21) Application number: 94109577.0
(22) Date of filing: 21.06.1994
(51) Int. Cl.: C07K 1/04, C07H 21/00, C07H 13/04, G01N 33/68

(54) **Preparation and screening of highly diverse peptide libraries for binding activity**
Herstellung und Durchmusterung von hochgradig diversen Peptidbanken hinschtlich Bindungsaktivität
Préparation et triage de librairie peptidiques qui présentent une haute diversification pour en dépister la capacité de former une fixation

(30) Priority: 22.06.1993 IL 10610693
(43) Date of publication of application: 22.02.1995
(73) Proprietor: INTERPHARM LABORATORIES LTD., Ness-Ziona 76110 (IL)
(72) Inventor: Hadas, Eran, Kiryat Ganim, Rishon LeZion (IL); Hornik, Vered, Rehovot (IL)
(74) Representative: VOSSIUS & PARTNER

(56) References cited:
- INTERNATIONAL JOURNAL OF PEPTIDE AND PROTEIN RESEARCH, vol.35, no.2, February 1990, COPENHAGEN DK pages 141 - 146 F S TJOENG ET AL. 'Multiple peptide synthesis using a single support (MPS3)'
- INTERNATIONAL JOURNAL OF PEPTIDE AND PROTEIN RESEARCH, vol.37, no.6, June 1991, COPENHAGEN DK pages 487 - 493 A FURKA ET AL. 'General method for rapid synthesis of multicomponent peptide mixtures'
- CHEMICAL ABSTRACTS, vol. 121, no. 7, 15 August 1994, Columbus, Ohio, US; abstract no. 77731p, V HORNIK & E HADAS 'Self-encoded, highly condensed solid phase-supported peptide library for identification of ligand-specific peptides' page 541 ;

## Description

The present invention describes a novel method for preparation of high density peptide (or other polymer) libraries, and for screening such libraries for peptide having the capacity to recognize targets of choice.

Many biological phenomena are known to involve the interaction of peptides with a macromolecular target, such as a protein. Such peptides include the vasoactive intestinal peptide, the angiotensins, and the endothelins.

For such an interaction to occur, the peptide must be able to fold into a conformation in which it presents a surface which is complementary to a critical region, e.g., a catalytic pocket, of the target molecule. While short peptides that interact with proteins or other biomolecules are used in research and clinical therapy (Magazine, 1991; Bischoff, 1992; Baumbach, 1992), the rational design of peptides to bind to new targets, or to enhance or render more specific their binding to a known target, is difficult.

In peptides, the number of total chemical structures is determined by the number of different amino acids used and by the peptide length, e.g., from 20 amino acids it is possible to synthesize 20⁹ different peptides with a length of 9 amino acids.

If one cannot rationally predict the peptides which will have the desired binding activities, it is desirable to be able to screen, simultaneously, a large number of peptides of different sequences, which are prepared and presented in a manner which facilitates identification of binding peptides. Such a collection of peptides is called a "peptide library." (Birbaum, 1992; Amato, 1992).

One method for construction of a peptide library is by genetic engineering (Scott 1990, Cwirla 1990, Devlin 1990). Peptides are expressed as part of a surface protein on the pIII protein on the outer surface of a filamentous phage. Phages reacting specifically with a target of choice are selected by panning and expanded. The relevant DNA sequence of the selected phages is then determined. The identified peptide sequences are deduced from the identified DNA sequences. The advantages of this approach to peptide library are speed and convenience. The major disadvantage is that the peptide library only contains linear (non-branched) peptides composed of the 20 native, L-amino acids.

Another approach for the generation of a peptide library is chemical synthesis. The major advantage of the chemical approach is the ability to produce peptides whose composition is not limited to the twenty genetically encoded amino acids. The use of a large number of different amino acids increases the extent of diversity it is possible to obtain from short peptide sequences. The chemical approach also facilitates the synthesis of cyclic and branched peptides. Besides increasing the diversity of the library, the use of non-native amino acids enables better control of peptide properties: e.g., lipid solubility of peptides may be largely increased by use of sulfoxide derivatives of methionine.

The "addressable library" approach, practiced by Affimax (Fodor 1991) is as follows: peptides are synthesized in squares as small as 10x10 µm on a piece of glass. The peptide sequence formed in each square is known by virtue of its position. On a surface of about 1 cm² it is possible to pack as many as 100x100=10,000 different peptides. The peptides are reacted with a fluorescent ligand and the stained squares are identified under the microscope. In this way it is possible to immediately identify peptides binding to a specific ligand. The major disadvantage of this approach is the relative small number of peptides it is possible to screen.

In the variation presented by Houghten (1991), hexapeptide mixtures were synthesized from 18 L-native amino acids. Position 6 corresponds to the C terminal and position 1 corresponds to the N terminal. A complete mixture of all 18 amino acids was introduced in each of positions 3-6. The peptide mixture was then separated into 18x18=324 different tubes, and in each tube a specific dipeptide was introduced in positions 1-2. The 324 peptide populations were screened for activity (e.g., inhibition of antibody-antigen interaction) and the most active peptide mixture was identified. Next, 18 new peptide mixtures were synthesized. In positions 1 and 2 all of the 18 new peptide mixtures contained the dipeptide identified in the previous step. The 3rd position of each of the mixtures had a single amino acid. Positions 4-6 contained a mixture of all 18 amino acids. The 18 peptide mixtures were screened for activity and the best reacting mixture was selected for further characterization. The process was repeated until all 6 positions in the peptide were identified.

More recently, Houghten (Oral presentation and abstract, European Peptide Society (EPS) 92 symposium, Interlaken, Switzerland) suggested a different approach. Starting from 18 amino acids, a total of 18x6=108 peptide mixtures were synthesized. In 18 mixtures, position 6 contained a unique amino acid, and positions 1-5 contained a mixture of all amino acids. In another 18 mixtures position 5 contained a unique amino acid and all other positions contained a mixture of all 18 amino acids, etc.... Once synthesized, all the 108 peptide mixtures were tested simultaneously and the most active mixture out of each of the 18 mixture representing each position was identified. The desired sequence was thus identified in a single day.

The major disadvantage of both Houghten approaches is the limited ability to incorporate a large number of different amino acids. When the number of different amino acids is increased, the relative amount of each unique peptide within the mixture is reduced and thus its effects become more difficult to identify. Houghten compensates by testing his peptide mixtures for activity at a concentration of 5 mg/ml. However, testing libraries constructed of more than 40 or so different amino acids would be difficult to conceive.

The present invention overcomes the aforementioned deficiencies of the Background Art. In particular, it permits the screening of much larger peptide libraries.

Conventionally, when a peptide library is synthesized on beads, on any given bead, all of the peptide molecules have the same sequence. Consequently, as previously explained, the diversity of the peptide library is limited by the total number of beads employed, which in turn is limited by human factors. We have estimated this limit to be on the order of 10⁸ different beads, and hence 10⁸ different peptide sequences. By the method of the present invention, it is believed that the delivery of the library may be increased by as much as seven orders of magnitude, i.e., to as many as 10¹⁵ different peptide sequences.

In the present invention, each bead bears, not a single peptide sequences, but a single family of related peptide sequences. (Normally, the family trait is a common (or low degeneracy) amino terminal portion of one or more amino acids.) The peptide library, in turn, includes many different families of peptides, with each family being found on one or more beads. Because the peptide library is arranged so that the peptide complement of each bead is constrained, the library is said to be "structured." This structured library is then subjected to a round of screening.

If a bead is marked by an affinity reagent, it indicates that one or more of the peptides in its family are bound by the affinity reagent. The peptide mixture on the bead is then sequenced to determine the common (or low degeneracy) amino terminal portion, the familial "marker."

In the next round of screening, a sublibrary of the library of the prior round is constructed, in which all peptides possess the familial marker of the successful family in the last library. Each bead of this new library carries only peptides belonging to a subfamily of the aforementioned family. When this sublibrary is screened with an affinity reagent, the beads which are bound are those whose subfamilies include a binding peptide. The process is then repeated, with each successful family of the library of one screening round becoming, in the next round, a new library, which in turn is divided into families. Eventually, the entire sequence of the binding peptide is known.

While, for convenience, the description refers to synthesis, screening and sequencing of peptide libraries, it applies, mutatis mutandis, to libraries displaying other heteropolymers whose ability to bind specifically to a target is related to their specific sequence of monomeric units. Such polymers include peptoids, nucleic acids, and carbohydrates. It should further be noted that the term "polymer" is intended to include "oligomers".

The present invention involves preparation of a peptide (or other polymer) library in which a highly diverse collection of peptides are synthesized on beads by solid phase peptide synthesis techniques and then presented to potential targets. The library is structured so that each bead itself offers a detectable number of molecules of essentially each of a family of different yet related peptides. Because of this family relationship, once a "bead" is identified as "positive" by affinity screening, one or more amino acids of the part of the sequence which is "common" to all peptide sequences borne by that bead can be identified. A new peptide library is then prepared whose members correspond to the "positive family" of the prior library. This daughter library is in turn structured into families, one family per bead, so that screening and sequencing lead to the identification of additional residues of the actual binding peptide(s). The process is continued until the binding peptides have been fully sequenced.

The number of different peptides of length k which are possibly synthesized from N different amino acids is N^{K}. The number of beads it is possible to use per single library is practically limited by the amount of the peptides we are willing to synthesize and by our ability to screen the library. Each ml of packed beads contains from several hundred thousand to several millions of beads. Manually, we might screen a library of about 100 ml of beads, containing about 10⁷ - 10⁹ beads. If, as is conventional, each bead carried a single peptide, the number of beads in the library would be sufficient to screen for all the hexapeptides which could be synthesized from up to 30 different amino acids (720x10⁶). Screening of all the hexapeptides which are possibly synthesized from larger number of amino acids would be technically difficult or impossible.

The amount of peptide found on a single bead of about 100 µm diameter can be about 100 pmole, or about 6x10¹³ molecules. This number of peptide molecules is much larger than the number of molecules needed for assaying of ligand binding to the beads. Using enzyme detection or fluorescence detection methods it is possible to monitor the binding of antibodies with binding constants of about K_{A}=10⁹ to as few as 1000 receptor molecules appearing on the cell membrane of mammalian cells with diameter of about 10 µm. The diameter of the beads used for synthesis of the peptide is about 100 µm and so their volume is about (100/10)³=1000 times larger than mammalian cells. We therefore conclude that using enzyme or fluorescence detection methods we should be able to monitor the binding of ligands with K_{A}=10⁹ to beads containing at least 1000x1000=10⁶ target molecules. Since the beads contain about 6x10¹³ peptide molecules we concluded that we can pack each bead with many peptides. In fact, theoretically, we could pack 6x10¹³/10⁶=6x10⁷ different peptide molecules per bead and still be able to detect the binding of a ligand to a single peptide type.

Once a certain bead is identified as containing an interesting peptide we need to determine the peptide structure. This is achieved by N-terminal sequencing (Edmann degradation). The procedure is routinely carried out by an automatic machine. Following degradation the resulting PTH-amino acid is analyzed by reverse phase chromatography. The state of the art machines are capable of analyzing the sequence of about 10 pmole of peptide. Thus, the 100 pmole of peptide found on each bead are ample amount for sequencing. However, as compared to the immunological staining process the sequencing step is relatively insensitive. Thus, even though we could synthesize on each bead over 10⁷ different peptides and still be able to select beads based upon the interaction of the ligand with the peptides on the beads, using current machinery we would not have been able to retrieve the sequence information. In fact, even if we synthesized only 20 different peptides on a single bead, the amount of amino acid obtained by each Edmann degradation step would have been only 100/20=5 pmole, which is too low to allow precise analysis of the sequence.

We suggest that in accordance with the present invention it is possible to tackle the problem of elucidating the structure of an active peptide, in a library expressing many peptides per bead, in an iterative manner. According to our strategy, each step of the iteration is designed to allow identification of the desired bead via interaction to one or several of the peptides expressed on the bead, and at the same time enable the at least partial determination of the identity of one or more amino acids of the peptide. Several iterations are needed in order to allow the complete elucidation of the desired structure.

### Target

The purpose of constructing a peptide library is to identify peptides which bind to a target of interest. The target may be any kind of substance, whatsoever. It may be inorganic or organic; crystalline or amorphous; micromolecular or macromolecular; naturally occurring or artificial. Typical targets include proteins (including enzymes, hormones and receptors), carbohydrates, and lipids. Suitable targets include human tumor necrosis factor (or its p55 and p75 receptors), and interleukin - 6, its cell surface receptor, the IL - 6/receptor complex, and its transducer, gp130.
The novel binding molecules which may be obtained from peptide (or other polymer) libraries, include the following (the categories recited in italics are not mutually exclusive):

*Molecules that inhibit cell-cell or cell substratum recognition*. Possible applications could be: inhibition of tumor metastasis formation, and inhibition of platelet aggregation. The cell surface contains groups of molecules that mediate binding of one cell to another or of cells to extracellular matrix components. These molecules are e.g. the integrins. See Ferguson, T.A., Mizutani, H. and Kupper, T.S. "Two integrin-binding peptides abrogate T cell-mediated immune responses *in vivo*.", *Proc. Natl. Acad. Sci. USA* 88:8072-8076 (1991); Skubitz, A.P.N., Letourneau, P.D., Wayner, E. and Furcht, L.T., "Synthetic peptides from the carboxy-terminal globular domain of the A chain of laminin: Their ability to promote cell adhesion and neurite outgrowth, and interact with heparin and the b1 integrin subunit," *J. Cell Biol*. 115:1137-1148 (1991); Hynes, R.O., "Integrins: Versatility, modulation, and signaling in cell adhesion." *Cell* 69:11-25 (1992).

Peptides capable of inhibiting the cellular activities mediated by the integrins could be discovered as follows: An integrin would be cloned. The recombinant protein would be produced and purified. The purified proteins would be labeled with biotin and used to screen the library. Avidin conjugated with alkaline phosphatase would be used for staining of the beads which bind the biotinylated proteins. Following identification of the peptides that bind to the proteins, the peptides would be synthesized in soluble form and tested for their ability to modify the desired biological activity, e.g., as described in the cited papers.

*Inhibitors of viral adhesion to cell surface receptors, e.g. molecules that will mimic the activity of the soluble CD4*. The membrane bound form of the CD4 serves as a receptor for HIV1. Discovery of peptides capable of inhibiting the binding of viruses to cells could be done in one of two approaches. In one approach we could use complete virions. The binding of the virions to the beads could be monitored by using antibodies specific to the virus. Once the structure of the peptide binding to the virus is know it could be synthesized in a soluble form and tested directly in viral inhibition assays. In a second approach, viral proteins known of mediating the binding of the virus to the cell could be cloned, expressed and purified and used as described above for section 1.

*Inhibitors of viral-specific enzyme activities*.
-- Inhibition of viral protease activity.
-- Binding to and inhibition of the viral reverse transcriptase activity.

*Bactericidal or bacteriostatic molecules:*
-- Molecules that would produce a hole in bacterial membranes
-- Molecules that would block the bacterial protein synthesis machinery by interaction with the bacterial ribosomes. The screening approach would be to test the binding of purified bacterial ribosomes (or polyribosomes) or any other protein which is participating in the synthesis of polypeptides (e.g. enzymes) to the beads and later test the activity of the synthesized soluble peptides in inhibition of the ribosome activity.
-- Molecules that would interfere with bacterial cell wall construction. In this case we would screen for beads containing peptides that bind to at least one of the enzymes participating in cell wall synthesis. Later the identified peptides would be synthesized in soluble form and tested for their ability to inhibit the enzyme activity and consequently the cell wall synthesis.
-- Molecules that would inhibit the adsorption of the bacteria to specific targets. In this case we shall look for beads containing peptides that bind whole bacterial cells or, more preferably, cloned and purified bacterial cells known as participating in recognition of bacterial targets. In a second step the identified peptides would be tested for their ability to inhibit bacterial binding to the target.
-- Molecules that would interfere with bacterial DNA synthesis. We shall look for peptides which bind to cloned and purified enzyme which participate in DNA synthesis or in production of DNA precursors.

*Inhibitors of bacterial exo-and endotoxins*. The approach would include finding of peptides that bind to the toxin and later testing the ability of the identified peptide to inhibit the toxic activity.

*Molecules that have enzymatic activity*. We may screen for such peptides with a colorimetric (color generating) assay for the enzyme where the final product of the reaction would be insoluble, thereby staining the beads. e.g. Reduction of NAD to NADH could be monitored by binding on the beads of enzyme capable of using the reduced NADH to reduce a tetrazolium slat to insoluble colored formazan. Detection of other type of reactions may necessitate coupling of several enzymatic reactions until the desired color product is obtained. See Tawfik, D.S., Green, B.S., Chap, R., Sela, M. and Eshhar, Z. "catELISA: A facile general route to catalytic antibodies," *Proc. Natl. Acad*. *Sci*. *USA* 90: 373-377, (1993).

*Inhibitors of enzymatic activity, e.g. of proteolytic enzymes*. We shall first look for peptide capable of binding to the enzyme of choice. The peptides would then be synthesized in a soluble form and their ability to modify the enzymatic activity would be determined.

*Molecules that would modify enzyme activity in an allosteric manner*.

*Molecules that would bind DNA at specific sequences or sites and inhibit transcription*. For screening, beads could be stained by specific DNA segments labeled with biotin or an enzyme. In a second stage, soluble peptides could be tested for their ability to modify transcription. Binding at a specific site may indicate binding at loops, hairpins or other structures.

*Molecules that interfere with the interaction of proteins with nucleic acids by interaction with the proteins*. Screening for the ability of the peptide to interfere with the interaction of the protein with DNA or RNA could be done in the second stage, once peptide capable of binding to the protein are identified.

*Molecules that serve as adjuvants in vaccines*. Such structures may be used alone or as parts of constructs that express both the antigen and adjuvant on a single molecule.

*Molecules that serve as vaccines*, *i.e*., *molecules that mimic antigenic epitopes of natural antigens*. The current approach for preparation of peptide vaccines is to prepare peptides that contain B and T cell determinants of the antigenic protein. The preferred T cell determinant(s) are promiscuous (reactive with many MHC isotypes), so as to allow generation of immune response in as large proportion as possible of the population. The difficulty is that the peptides representing the major antigenic determinants are not necessarily immunogenic when removed from the protein. We would use antibodies generated against the antigen as targets in order to identify peptides capable of binding with the antibodies. These selected peptides would mimic the immunogenic structure of the antigenic protein. Thus, the approach would allow preparation of vaccines from parts of the protein which are immunogenic when the protein is intact, but not in isolation. In a second step we would couple the identified peptides to a T cell determinant and test their ability to serve as immunogens.

*Molecules that interact with the T cell receptor and serve for induction of T cell suppression*. Peptides that bind to the T cell receptor could bind at the active (recognition site) or outside of it, on the other part(s) of the receptor polypeptide chains. Initial screening could be for peptides that bind to purified receptor. A second screening could verify if the bound peptide has the ability to activate specific T clone (antigen mimetic) to activate many type of T cells (superantigen mimetic) or to inhibit the activation of the T cells by known T cell determinants. See Drake, D.G. and Kotzin, B.L., "Superantigens: Biology, immunology, and potential role in disease," Journal of Clinical Immunology 12:149-162 (1992); Esser, U. and Parham, P. "Superantigens: Playing upon both sides," Nature 359:19-20 (1992).

*Molecules that interfere in the antigenic presentation of other molecules by interacting with surface receptors on the antigen presenting cells (APCs)*. It is presently believed that protein antigens undergo cleavage into small peptides in the APC. These processed peptides bind to MHC (major histocompatibility antigen) molecules at the cell surface and are thus presented to T cells. Some types of MHC molecules bind the peptide during their own synthesis and migrate with the bound peptides to the cell surface. In order to compete with this mechanism we would need an inhibitory peptide which penetrates into the cells to the specific site where "loading" of the MHC molecule with peptides occurs.

Other types of MHC molecules bind peptide extra-cellularly. The binding of these peptides could be competitively inhibited with peptides in the circulation. The current dogma states that the binding of the peptide to the MHC is via two or more anchoring residues (sites) of the peptide (e.g. the second residue from the amino terminal and the free carboxy terminal) to specific sites on the MHC molecule. Theoretically, we could design stronger binding peptides that would prevent binding of the natural peptides. The application would be mainly in controlling autoimmune disorders.

*Molecules that enhance the immunogenicity of other molecule by targeting them to antigen presenting cells*. Many peptide epitopes are not good immunogens since they can not bind to MHC molecules and are thus not "presentable". It is possible that if such peptides would be coupled to peptide mimetics that would bind the MHC molecules the target peptides would become immunogenic. The library approach can be used for discovering peptides that bind to the different MHC isotypes. As stated above, MHC molecules bind peptides via "anchoring" residues/sites. We could discover peptide mimetics that bind to the MHC via non-conventual structures/sites that would be more efficient as compared with pure peptides.

*Molecules that inhibit the IgE mediated immediate type hypersensitivity response by preventing the occupation of the Fc*^{*ε*} *receptor by specific IgE antibodies*. During the screening we would look for peptides capable of binding to the Fc_{ε} receptor. This receptor is responsible for binding of the IgE. When the IgE which is bound by the receptor encounters the antigen, the cell bearing the receptor is activated. Activation of such cell is the primary element in the immediate type hypersensitivity also know as allergic response. If we could prevent the cells from binding the IgE we could abrogate the activation of the cells by the antigen thereby preventing the allergic response. In a first step we could use the library to look for peptides binding to the Fc_{ε} receptor. In a second step we would test the ability of the identified peptides to block the binding of the IgE to the receptor. The main application of such peptides would be the prevention of allergic reactions.

*Molecules that inhibit the binding of complement components to immune complexes thereby inhibiting complement activation*. We can screen the library for peptides that bind to immunoglobulin which participate in formation of immune complexes. The Fc of these immunoglobulins is conformationally modified as compared to the Fc of free immunoglobulin. Only the Fc of immunoglobulins participating in immune complex formation is capable of activating the complement. We could screen the library for peptides that bind to the Fc of immunoglobulins in an immune complex. In a second step we could test the identified peptides in a soluble form for their ability to inhibit complement activation.

*Molecules that bind to soluble immune complexes and prevent their accumulation in the kidneys*. Immune complexes tend to accumulate in the basal membrane of the kidney. Non-specific activation of complement at the site may eventually cause kidney failure. Some of the peptides that bind to the immune complexes (via the Fc of the participating immunoglobulins) may inhibit the specific binding of the immune complexes to the basal membranes. Alternatively, peptides may be found that prevent the activation of the complement by the immune complex by preventing the binding of the complement component C3 to the immune complex.

*Molecules that serve as target antigens or pseudo-antibodies in immunoassays*. Peptides may replace antigens or antibodies. Replacing of antigenic reagents used in immunoassays for the presence of cognate antibodies in the serum (e.g. measurement of antibodies to the AIDS virus) with a peptide might improve specificity. Currently, many such assays tend to pick up false positives that have to be further evaluated to make sure they are truly positives. Some people have tried to use peptides derived from the virus. This approach resulted in a more specific assays. Using peptide mimetics and the library approach it would be possible to further narrow down the specificity.

The difficulties associated with use of antibodies in immunoassays are numerous: (1) Instability of the antibody protein. (2) Changes in the specificity of the antibodies from batch to batch and upon storage. (3) Difficulties of labeling with tracer. (4) High price. (5) Difficulties in disinfection e.g. when preparing probes. (6) Denaturation upon repeated elution of the analyte. (7) Difficulties in immobilization onto a solid support. (8) Bivalency of the antibodies is sometime disadvantageous.

Replacing of the antibodies with peptides might overcome some of the above difficulties. (1) As compared with the antibody, the peptide has no "conformation" which may be destroyed (leading to an inactive protein). (2) The batch to batch consistency of the peptide is much easier to control as compared with antibodies. (3) The tracer may be synthesized together with the binding part of the peptide. (4) The price of synthetic peptides is typically lower than that of antibodies. (5) The peptides are stable to high concentration of organic solvent which may be used in disinfection. These solvent would destroy antibodies. (6) Having no "fixed" conformation, the peptides would not be destroyed by extended use. (7) The peptide does not need to be immobilized. It can be synthesized in situ on many type of supports. (8) The peptide may be mono, di- or poly-valent as required.

*Inhibition of immune cell migration by molecules that bind to and block the cell surface receptors responsible or chemotaxis*. Some of the molecules that are expressed on the cell surface control the ability of the cells to respond to stimuli, These cell surface receptors control, among other phenomena, also chemotaxis. These molecules need to be identified, cloned and purified. Once purified the molecules could be used for selection of peptides which would bind to them. Some of the peptides might have the ability to specifically block the receptor and thus abrogate the ability of the cells to respond to specific stimuli. Such peptides could be used e.g. in prevention of inflammation or prevention of graft rejection.

*Inhibition of cytotoxic T lymphocyte (CTL) function by molecules that interfere with binding of the CTL with the target cell*. The first step in the lysis of target cells by CTL is binding of the CTL receptor to a specific receptor on the target cells. Cloned and purified polypeptide chains of the CTL receptor could be used for screening of a peptide library for peptides that bind to the receptor. It is expected that some of the peptides selected would be capable of inhibiting the ability of the CTL to bind to their targets. Peptides that bind at the antigen binding site would inhibit the activity of specific CTL clones. However, peptides may be found that would bind outside the active site but still inhibit the ability of the receptor to bind to its target. Such peptides could be e.g. inhibition of autoimmune responses.

*Molecules that temporarily inhibit the multiplication of stem cells*. Can be used in order to minimize damage to the stem cells during chemotherapy.

*Molecules that interact with both tumor cells and cytotoxic T lymphocytes (CTLs) thereby targeting the CTLs into tumors and enhancing tumor cell killing*. The first step in the attack of CTL on tumor cells is binding of the CTL to tumor cells. The binding is mediated by specific cell surface receptors found on the CTL. The CTL receptor bind to tumor antigens, expressed on the surface of tumor cells. Recent results indicated that it is possible to induce specific lytic activity also when the CTLs bind to the tumor cell via a bridging molecule e.g. a bifunctional antibody that recognizes the CTL receptor and the tumor antigen. A similar activity could be mediated by peptides having two sites: one site that would bind to a tumor cell surface antigen and another that would bind to the CTL receptor. Screening of a peptide library for peptides capable of binding each of the structures could be performed as described. Once such peptides are found, they would be synthesized into a single polypeptide chain, or other wise chemically conjugated and tested for their ability to induce specific lysis of the target tumor cells.

*Molecules that interact with and inhibit the activity of angiogenic factors or their receptors*. Used for inhibition of angiogenic activity of tumors. The approach here would be similar to discovery of peptides which bind to and inhibit other cytokines.

*Molecules that inhibit multiplication of tumor cells*. Peptides may be discovered that inhibit the activity of enzymes that participate in DNA synthesis or the synthesis of any of the precursors. The screening for such peptides would be based upon screening for peptides that bind to the desired enzyme and inhibit its activity. The specificity to tumor cells amy be mediated by another peptide that would bind to a cell surface molecule that is internalized following binding of the peptides thereby allowing the enzyme inhibitor peptide access specifically into tumor cells. Such approach is currently under investigation using natural toxic peptides conjugated to antibody fragments, e.g. see, Better, M., Bernhard, S.L. Lei, S.P., Fishwild, D.M., Lane, J.A., Carroll, S.F., and Horwitz, A.H., "Potent anti-DC5 Ricin A Chain Immunoconguates for Bacterially Produced Fab' and F(ab'')₂," Proc. Natl. Acad. Sci., (USA), 90:457-461, (1993).

*Molecules that enable passage through the cell membrane or the membrane of endosomal vesicles*.
-- Enzyme inhibitors.
-- Antisense polynucleotide or PNAs.

*Molecules that enable passage through the blood-brain barrier*.

*Molecules that target functional groups into tumors by way of binding to cellular components*. The functional groups may include:
-- toxins
-- radio nuclides: For imaging and therapy.
-- Neutron capturing agents
-- Enzymes: e.g. glucose oxidase.

*Molecules that enable enhanced passage through skin, upper respiratory tract or lungs*.

*Sorting of cells following their fluorescent surface labeling or magnetic sorting, e.g. Purging of tumor cells from bone marrow cells in vitro*. Like antibodies, peptides may be used as specific labels for cells. The use of labeled peptides would allow analysis and sorting of the desired cells.

### Inhibitors of embryo implantation or development

*Molecules that would serve for affinity purification of other molecules*. One popular method for purification of recombinant (and native) proteins is affinity chromatography. The affinity ligands usually are mimetic dyes and sometimes also monoclonal antibodies. Peptides could be selected that specifically bind to a target of choice, thus allowing its purification by affinity chromatography. They might allow a combination of the specificity of antibodies coupled with the ease of use of mimetic dye columns, e.g. depyrogenization with 1 N NaOH.

*Enzymatic catalysis*. In some processes (e.g. immunoassay) it is needed to immobilize proteins or other molecules on surfaces. Peptides that bind the desired ligand could be used for such immobilization. Immobilization of cells (adherence of cells to peptide containing surfaces) is a specific example that has already been demonstrated. See Fernandez, M.C., Mullenix, M.S., Christner, R.B. and Mortensen, R.F., "A Cell Attachment Peptide From Human C-Reactive Protein," J. Cell Biochem. 50:83-92, (1992); Chen, Y.-C.J., Danon, T., Sastry, L., Mubaraki, M., Janda, K.D. and Lerner, R.A., "Catalytic Antibodies from Combinatorial Libraries," J. Amer. Chem. Soc. 115:357-358, (1993); and Lesley, S.A., Patten, P.A. and Schultz, P.G., "A Genetic Approach to the Generation of Antibodies with Enhanced Catalytic Activities," Proc. Natl. Acad. Sci. (USA), 90:1160-1165, (1993).

*Molecules that would serve as mammalian tissue culture additives*. Recently it has been found that some proteins can be used to replace serum in propagation of mammalian cells in tissue culture. Among the proteins are insulin and some growth factors. When the receptors of these proteins are known, peptides could be selected from the library that would bind to the receptor, e.g., the insulin receptor. The ability of the peptide to activate the receptor and thus replace the proteins could be tested at a second stage.
The advantages for the use of peptides would be both economical (peptide are cheaper than proteins) and regulatory (it is basically safer to use peptide as compared to proteins from any source, natural or recombinant).

*Molecules which are antagonists (partial or complete) of ligands (hormones, cytokines, neurotransmitters, toxins, etc.)* (Note: The term "Partial antagonist" means that only some of the biological activities of the ligand would be inhibited while other activities would not be impaired.) Activity is mediated by interaction with any of the following:
-- The ligand (hormone, cytokine, neurotransmitter, steroid, leukotrienes, releasing factor, etc.): Prevention of the ligand interaction with the receptor.
-- The receptor: Binding to the receptor and thereby prevention of the interaction of receptor with the ligand or with signal transduction molecules.
--The signal transducing molecule(s): Prevention of the activation of the molecule by the receptor or prevention of the signal transduction by prevention of the activation of the signal transducer.

With regard to agonists (complete or partial) or antagonists (complete or partial) of natural peptides, the following peptides may be targets:
Angiotensin (types I and II), Biberotoxin; Endothelin; Sarafotoxin; Bombesin; Calcitonin; Calpain; Cholescystokinins; Cecropin; Corticotropin releasing hormone (CRH); Defensin; Galanine; Gelsolin; Glucagon; GnRH - Gonadotropin releasing hormone; Leupeptin; MSH - alpha melanotropin; NPY - Neuropeptide Y; Peptide Leukotrienes; Somatostatin; Substance P; Tachykinin; Vasopresin; VIP; Opiate family. Natural peptides are not convenient for use as drugs. They are relatively unstable, are difficult to deliver, tend to have short half lives in the circulation and sometimes lack desired specificity. Peptide mimetics could be selected that bind to the receptors of the natural peptides and mimic or antagonize their biological activities. Since the library would probably yield several possible lead structures, it is possible that some of them would be more suitable for use as drugs as compared with the original native peptide.

### Affinity Reagent

Peptides which bind to a target of interest are identified by their binding to an affinity reagent. An affinity reagent is a chemical entity whose binding characteristics are similar to those of the target of interest, and whose binding to a peptide (or to another reagent which is bound to the peptide) causes a detectable physical or chemical change to occur. Typically, the affinity reagent is a target molecule (or an analogue thereof), conjugated with a label, such as a radioisotope, a fluorophore, a colorophore, an enzyme, an enzyme substrate, or an electron-dense moiety. The label may be observable directly, or it may be detectable only by virtue of further processing. For example, a biotinylated target molecule may be bound to the peptide, then an enzyme-labeled avidin bound to the biotin tag, and finally the enzyme provided with a substrate, the enzymatic reaction product having a distinctive color. The preferred reagents have fluorescent or enzymatic labels. If the label is fluorescent, it is desirably rhodamine. If the label is enzymatic, alkaline phosphatase is preferred.

### Amino Acids and Peptides

Amino acids are the basic building blocks with which peptides and proteins are constructed. Amino acids possess both an amino group (-NH₂) and a carboxylic acid group (-COOH). Many amino acids, but not all, have the structure NH₂-CHR-COOH, where R is hydrogen, or any of a variety of functional groups.

Twenty amino acids are genetically encoded: Alanine, Arginine, Asparagine, Aspartic Acid, Cysteine, Glutamic Acid, Glutamine, Glycine, Histidine, Isoleucine, Leucine, Lysine, Methionine, Phenylalanine, Proline, Serine, Threonine, Tryptophan, Tyrosine, and Valine. Of these, all save Glycine are optically isomeric, however, only the L-form is found in humans. Nevertheless, the D-forms of these amino acids do have biological significance; D-Phe, for example, is a known analgesic.

Many other amino acids are also known, including: 2-Aminoadipic acid; 3-Aminoadipic acid; beta-Aminopropionic acid; 2-Aminobutyric acid; 4-Aminobutyric acid (Piperidinic acid); 6-Aminocaproic acid; 2-Aminoheptanoic acid; 2-Aminoisobutyric acid, 3-Aminoisobutyric acid; 2-Aminopimelic acid; 2,4-Diaminobutyric acid; Desmosine; 2,2'-Diaminopimelic acid; 2,3-Diaminopropionic acid; N-Ethylglycine; N-Ethylasparagine; Hydroxylysine; allo-Hydroxylysine; 3-Hydroxyproline; 4-Hydroxyproline; Isodesmosine; allo-Isoleucine; N-Methylglycine (Sarcosine); N-Methylisoleucine; N-Methylvaline; Norvaline; Norleucine; and Ornithine.

*It has been found to be convenient to assign each of the amino acids used in the libraries disclosed herein an ID number for greater simplicity of reference. These ID numbers appear in Table 101*.

Peptides are constructed by condensation of amino acids and/or smaller peptides. The amino group of one amino acid (or peptide) reacts with the carboxylic acid group of a second amino acid (or peptide) to form a peptide (-NHCO-) bond, releasing one molecule of water. Therefore, when an amino acid is incorporated into a peptide, it should, technically speaking, be referred to as an amino acid residue.

### Peptide Synthesis: An Overview

In a standard "Merrifield" synthesis, a side chain-protected amino acid is coupled by its carboxy terminal to a support material,such as a resin. A side chain and amino terminal protected amino acid reagent is added, and its carboxy terminal reacts with the exposed amino terminal of the insolubilized amino acid to form a peptide bond. The amino terminal of the resulting peptide is then deprotected, and a new amino acid reagent is added. The cycle is repeated until the desired peptide has been synthesized. For an overview of techniques, see Geisaw, Trends. Biotechnol., 9:294-95 (1991).

In the conventional application of this procedure, the amino acid reagent is made as pure as possible. However, if a mixture of peptides is desired, the amino acid reagent employed in one or more of the cycles may be a mixture of amino acids, and this mixture may be the same or different, from cycle to cycle. Thus, if Ala were coupled to the resin, and a mixture of Glu, Cys, His and Phe were added, the dipeptides Ala-Glu, Ala-Cys, Ala-His and Ala-Phe will be formed.

When, during a synthetic cycle, a pure amino acid is added, the resulting residue in the peptide molecules being synthesized is called a constant residue. If a mixture of amino acids is employed, the added residue is called a variable residue. The component amino acids of the mixture, which are the only amino acids which can occupy that variable residue position, are called the "set" of that variable residue. The set for one variable residue may be different from that of the next one. When any of the residues added during the synthesis of a peptide is a variable residue, so that the synthesis deliberately produces a mixture of peptides, the mixture is termed a peptide library. The differences among the peptide molecules of the library will lie, essentially at, and only at, the predetermined variable residue positions.

### Peptide Library

A peptide library may consist essentially only of peptides of the same length, or it may include peptides of different length. The peptides of the library may include, at any variable residue position, any desired amino acid. Possible sets include, but are not limited to: (a) all of the genetically encoded amino acids, (b) all of the genetically encoded amino acids except cysteine (because of its ability to form disulfide crosslinks), (c) all of the genetically encoded amino acids, as well as their D-forms; (d) all naturally occurring amino acids (including, e.g., hydroxyproline); (e) all hydrophilic amino acids; (f) all hydrophobic amino acids; (g) all charged amino acids; (h) all uncharged amino acids; etc. The peptide library may include branched and/or cyclic peptides.

The "size" of the library is the estimated number of peptide molecules in it. Preferably the size of the library is at least 10¹⁴, more preferably at least 10¹⁶, still more preferably at least 10¹⁸, most preferably at least 10²⁰ molecules. If there are 6 x 10¹³ peptides per bead, even 10⁶ beads would provide 6 x 10¹⁹ peptides, while 10⁸ beads would carry 6 x 10²¹ peptides.

The "diversity ("degeneracy") of the library is the expected number of unique peptide sequences in the library. The method of the present invention does not have a technically imposed lower limit on library diversity. However, there would be no point to constructing a library with a diversity of only two. Desirably, the library should be sufficiently diverse so that it would be advantageous to simultaneously synthesize and screen the library, rather than prepare and test each peptide individually. For this reason, the library will ordinarily have a diversity of at least 10³. A further consideration is whether the library has a diversity comparable to, or greater than, that of the libraries described in the Background Art. Preferably, the diversity of the library is at least 10⁸, more preferably at least 10¹⁰, still more preferably at least 10¹², and most preferably at least 10¹⁴ unique sequences. A diversity of 10¹⁴ would be achieved with 10⁸ beads each bearing 10⁶ sequences. The "sequence set" of the library is the set of sequences which, given the choice of constant and variable residues, and the sets for each variable residue, could theoretically be presented in the library.

The "average sampling level" of the library is the size divided by the diversity, i.e., the average number of molecules having the same peptide sequence. Preferably, the average sampling level is sufficient for detection and at least partial sequencing. It is preferably at least 10⁶ molecules per sequence, more preferably at least 10⁷, still more preferably at least 10⁸. The average sampling level should be at least equal to the peptide-per bead detection limit (assumed to be presently 10⁶), more preferably 10 times said limit to provide a margin of safety.

While the peptide library may include di-, tri-, and tetrapeptides, the preferred minimum length of the peptides is five amino acids. There is no definite maximum length.

### Structured Peptide Library

A structured peptide library is one in which peptide synthesis on a collection of beads (or equivalents) is controlled so that the repertoire of sequence variation on a single bead is limited to a predetermined subset of the allowed universe of sequence variation for the entire library.

Such a library is formed by stepwise synthesis of the peptides on the beads by a protocol which includes one or more "structured random" addition cycles. (Optionally, one or more "unstructured random", or "nonrandom", cycles may be utilized as well.)

A "structured random" cycle is one in which a variable residue is added, but some degree of control is exercised as to which beads receive which amino acids of the set. A "nonrandom" cycle is one in which all growing peptides of the library are reacted with a pure amino acid addition reagent. An "unstructured random" cycle 1s one in which they are all reacted with a "mixed amino acid addition reagent", the mixture including all amino acids belonging to the set defined for that variable residue position.

In the simplest form of "structured random cycle," the beads are divided into N aliquots, where N is the number of amino acids in the set of that variable residue. Each aliquot receives a different one, and only one, of those N different amino acids. As a result, all peptides on a bead in a given aliquot have the identical amino acid at the variable residue position, in question. This is called a "fully structured" cycle.

There are circumstances, however, when it is appropriate to react each aliquot with a mixture of a unique subset of the amino acids in the set of the variable residue in question. For example, the set for the residue position, considering the library as a whole, may be 100 amino acids. The beads may be divided into aliquots, A, B, C and D, which are reacted with mixtures A' (AAs 1-25), B' (AAs 26-50), C' (amino acids 51-75) and D' (amino acids 76-100), respectively. This is an example of a "partly structured" cycle.

The number of different aliquots to which a bead may be assigned during a particular structured cycle is the "partitioning factor" for that cycle. The number of different permutations of aliquot assignments which an individual bead may experience as the library is synthesized is the "library partitioning factor", the product of the partitioning factors for the individual cycles (the partitioning factor for an unstructured cycle is unity). The expected number of beads in the library that will have been subject to a particular sequence of aliquot assignments in the partitioning steps (B_{L}')is the total number of beads in the library (B_{L}), divided by the library partitioning factor. B_{L}' is preferably at least one, more preferably at least two, still more preferably at least ten.

If there are 10⁷ beads in the library (B_{L}) and there are three structured cycles, each with a partitioning factor of 100, the library partitioning factor is 10⁶, and B_{L}'is 10. If four structured cycles were employed, a partitioning factor of 100 per cycle would be too high; a factor of about 90 would be acceptable (90⁴=7.43 x 10⁶). If a larger number of beads could be screened, the library partitioning factor could be increased.

The expected number of identical peptide molecules on a single bead (M_{B}') is equal to the expected number of peptide molecules on the bead (M_{B}) divided by the expected diversity of the bead (D_{B}). The diversity factor (D_{B}) for the bead is the product of the diversity factors for that bead for each residue of the peptide. In an unstructured random cycle, the cycle's diversity factor is the same for both bead and the library, i.e., the number of different amino acids in the corresponding reagent. In a structured random cycle, the cycle's diversity factor for a bead is the number of different amino acids in the reagent reacted at that time with that bead (for a fully structured cycle, it is unity).

The number of peptide molecules which may be carried by a single bead is a function of the surface area of the bead, and the number of potential simultaneous peptide attachment sites on that surface. The method of the present invention requires that this number (M_{B}) be at least two (which would be technically feasible only if a single peptide molecule could be detected and sequenced, and which would allow only two different sequences per bead). For practical reasons, M_{B} is at least 10², preferably at least 10³, more preferably at least 10⁶, still more preferably at least 10⁹, even more preferably at least 10¹². Examples 1-4 assume a value of 6x10¹³ molecules/bead.

The number of beads in the library (B_{L}) is limited only by the number of beads which may be screened. Preferably, at least 10⁷ beads, more preferably 10⁸ or 10⁹ beads, are screened. It is likely that mechanical assistance would be required to effectively screen a larger number of beads in a single library.

The number of binding peptides which must be carried by a single bead for the binding assay to be able to determine whether those molecules specifically bind the affinity reagent is a function of both the degree reagent, and the sensitivity of the assay. Preferably, the assay requires no more than 10⁷, no more than 10⁶ binding molecules, per bead, for identification. Also, it is preferable, that no more than ten, more preferably no more than two, still more preferably no more than one such bead is needed for detection.

The maximum potential diversity of the peptide library is a function of
(a) the number of peptide molecules which may be carried by a single bead,
(b) the number of beads which may be screened,
(c) the number of binding peptide molecules which must be carried by a single bead for the binding assay to be able to determine whether those molecules specifically bind the affinity reagent,
(d) the number of at least partially identical peptide molecules which must be carried by a single bead for the common portion of their amino acid sequence to be sequenceable, and
(e) the required level of statistical confidence that essentially all theoretically synthesized peptides are actually present in detectable and sequenceable amounts.

*It will be recognized that the person of ordinary skill will take advantage of advances in the binding assay, peptide synthesis, and peptide sequencing arts so as to achieve a higher level of diversity in the library*. *Consequently, while for the purpose of calculations demonstrating the feasibility of the present invention, it may be assumed that 10*^{*8*} *- 10*^{*9*} *beads may be* s*creened manually, that 6 X 10*^{*13*} *peptide molecules may be packed on a single bead, that 10*^{*6*} *molecules are required for detection of binding, and that 10 pmoles of peptide (about 1.5 X 10*^{*13*} *hexapeptide molecules) are required for sequencing, these limitations should not be imposed on the scope of the present* i*nvention if they become technologically obsolete.*

The first limitation on the diversity of the library is the number of peptide molecules in it. This is equal to the number of beads in the library, multiplied by the number of molecules per bead. Thus, if there are 10⁷ beads, and 6 x 10¹³ peptides per bead, there are 6 x 10²⁰ peptide molecules in the library.

The second limitation is imposed by the detection technology. For detection to occur, there must be one or more beads each of which bears a minimum number of identical peptide molecules which have the desired binding property. For example, if the detection technology requires one positive bead, and at least 10⁶ molecules on that bead which have the appropriate sequence, the maximum permissible diversity of the library is (6 x 10²⁰/10⁶=)6 x 10¹⁴ different peptide sequences. Thus, such a library might be a pentapeptide library, with 500 different amino acids at each position (500⁵<6 x 10¹³), a hexapeptide library, with nearly 200 different amino acids at each position (200⁶= 6.4 x 10¹³), an octapeptide library with over 40 different amino acids at each position (40⁸ = 6.6 x 10¹²).

The diversity of a single bead is also limited. If there are 6x10¹³ attachment sites, and 10⁶ identical peptide molecules are required for detection, the maximum bead diversity is 6x10⁷.

Each unstructured random cycle increases the diversity of a single bead, as well as of the library, by its diversity factor. Each fully structured random cycle increases the diversity of the library, but not the diversity of the bead. A bead diversity limit of 6x10⁷ would be approached by three unstructured cycles of a little less than 400 amino acids each, four unstructured cycles of almost 90 amino acids each, and so forth.

In the example above, there would be no advantage to adjusting the relative number of structured and unstructured cycles. With two structured and four unstructured cycles of 100 AAs each, there would only be (6x10¹³/100⁴=)6x10⁵ molecules of each peptide sequence on each bead, below the assumed detection limit of 10⁶. With four structured and two unstructured cycles of 100 AAs each, there would be 100⁴(=10⁸) different permutations of bead partitions, but only 10⁷ beads, so that the expected number of beads subjected to a given series of four aliquot assignments would be only 0.1, not at least 1.0 as is desirable.

However, if our underlying assumptions are changed, the relative merits of structured and unstructured cycles also change. If, for example, the peptide density on the bead were higher, or the detection limit lower, the number of unstructured cycles could be increased. If the number of beads were higher, more structured cycles would be feasible. And finally, if fewer amino acids were used in each cycle, there could be more cycles, structured or unstructured.

The amount of peptide required for sequencing by present technology is 10 pmoles, which corresponds to about 6x10¹² molecules (hexapeptides). If the diversity on a single bead were limited to that required for sequencing the entire peptide at once, the approach would be of marginal value. With 6x10¹³ molecules per bead, the diversity would be limited to (6x1013/6x10¹²=)10. However, the present method contemplates that only a partial sequence is determined initially.

Thus, the peptides of the initial library consist of a first familial portion and of a second individual portion. The first portion, which usually comprises one to five, preferably three amino acids, is common to (or of limited variability among) all peptides on a given bead. The remainder of the peptide sequence is the portion which fully or primarily distinguishes it from the different peptide sequences carried by the same bead.

In the subsequent sublibraries, each peptide may be characterized as having a first portion which is "universal," i.e., possessed by all peptides in that sublibrary, a second portion which is familial to all peptides on a single bead of that sublibrary, and a third, individual portion. It is only necessary that each possible residue of the familial subsequence on the active bead be present in a sequenceable amount. If there are 100 pmole per bead, and 10 pmole is sequenceable, there could be up to ten different amino acids (each at 10 pmole) in a given residue position among the peptides on a single bead. If the residues of the familial subsequence are variable residues, several secondary will be studied to determine which of the familial subsequences belonged to an active peptide of the primary library.

During synthesis of the familial portion of the peptide, in each cycle in which a variable residue is to be added, the beads are divided into N aliquots, where N is the number of amino acids in the set of that variable residue, i.e., the number of different amino acid reagents used in that cycle if the cycle is fully structured. Each aliquot of beads is reacted with an amino acid reagent providing one, and only one, of the amino acids of the set. This is conveniently done in N different reactors. The aliquots are then pooled. More typically, an encoding factor of two is used, so each aliquot is reacted with a mixture of two different amino acid reagents.

If, however, the variable residue to be added is within the individual portion of the peptide, a mixture of all the amino acids of the set is added to all of the beads.

A library may include peptides of different lengths. Such a library may be constructed by modifying one or more structured cycles so that one of the aliquots is not reacted with an amino acid. Alternatively, in any random cycle, the reagent may comprise a mixture of amino acids and oligopeptides. Either way, a library may be formed having peptides of different lengths but with a common familial portion.

### Beads

The term "beads" is not intended to be limited to spherical particles, but includes any small, discrete, solid elements upon which a structured peptide library may be synthesized and screened. Thus, the "beads" must be formed of a material with which peptides can be conjugated, and which is not substantially bound by the affinity reagent. In addition, the beads must be capable of being divided into aliquots and pooled back together, as described above, and of being separated later, during the screening process, according to the ability of their conjugated peptides to bind an affinity reagent.

Preferably, the beads are made of aminomethylated polystyrene crosslinked with divinyl benzene. Other potentially suitable materials include Tentagel (polyethyleneglycol modified polystyrene cross linked with divinyl benzene). The suitability of other support materials for use in the present invention may be evaluated against the following criteria:
a. The ability to synthesize peptides on the beads: The beads should be stable for all the solvents used in the peptide synthesis.
b. They should contain a free amino group, or a suitable stable but cleavable linker. However, it should be noted that a cleavable linker is not required.
c. The beads should be mechanically stable during synthesis, screening and handling.
d. The size of the beads should be large enough to allow manual handling, or whatever alternative handling means is contemplated.
e. The peptide capacity of the bead should be at least 10 pmole of peptide per bead, or whatever lower limit is rendered feasible by advances in sequencing technology. A capacity of about 100 pmole is preferable.
f. The beads should display a low degree of non-specific adsorption of ligands of choice and of proteins in general. (These criteria should not be considered absolute requirements.)

Beads which may be tested for suitability include:
Amino methyl PERSEPTIVE beads: (Perseptive, Cambridge, Massachusetts, USA).
Beads based on the polymer TSK gel (TosoHaas, in Stuttgart, Germany).
Matrix based upon FastFlow Sepharose (Pharmacia Uppsala, Sweden).

The number of peptide molecules which may be placed on a given bead is a function of the surface area of the bead and of the number of reactive sites per unit area. While there is no definite lower limit on carrying capacity, the number of peptide molecules per bead is one of the factors limiting the potential diversity of the library which can be reliably screened (i.e., so that one may with reasonable confidence assume that all of the peptides which were theoretically expected to be produced were in fact produced in detectable amounts, and, if screening were negative, assert that none of the expected peptides had the desired affinity for the target). Nor is there a definite upper limit on carrying capacity, however, if the reactive sites are too closely spaced, it is possible that there would be stearic hindrance of binding. Preferably, the bead has a diameter of 50 to 500 microns, (e.g., 100 microns). The bead is preferably able to carry at least 25 pmole peptide, more preferably at least 100 pmole peptide.

Since the packing efficiency (beads per unit volume of reactor) decreases with increasing bead diameter, it is desirable to use smaller beads. The number of beads per unit volume of reactor is directly dependent upon the volume of each bead, assuming that all the beads in question are spherical. The volume of each bead is proportional to the third power of the bead diameter. The beads are porous, and the peptide is expected to be synthesized throughout the bead volume. The capacity of the beads (the amount of peptide on the bead) is therefore expected to be directly proportional to the bead volume and thus directly proportional to the third power of the diameter. Thus, if you increase the diameter of the bead from 100 to 200 microns, it is expected that:
a. The number of beads per unit volume of the reactor would be 8 fold lower.
b. The peptide capacity of each bead would be 8 fold higher.
The total peptide capacity per reactor volume would therefore be constant.

### Alternative Supports

The structured oligomer libraries of the present invention may be adapted to libraries in which peptides are displayed on supports other than beads. However, the supports must be individually addressable, so that an individual support element displays a known family of oligomers, having a substantially common subsequence whose sequence is determinable when the individual support element is examined.

One example is an adaptation of the light-directed, spatially addressable parallel chemical synthesis method of Fodor, et al., Science, 251:767 (1991). In Fodor's method, synthesis occurs on a solid support sheet. The pattern of exposure to light (or other forms of energy) through a mask (or other spatially addressable means) determine which regions of the support are activated for chemical coupling. This activation results from the removal of photolabile protecting groups from the illuminated area. The support is exposed to the addition reagent, which reacts only in the region which underlay the window of the mask. The substrate is then illuminated through a second mask, with a different window. Combinatorial masking strategies are used to form a large number of compounds in a small number of chemical steps. For example, in the first round, the support surface may be divided into twenty vertical stripes, each of which (in separate illumination-reaction cycles) receives one of the twenty genetically encoded amino acids. The surface is then divided into twenty horizontal stripes, which are similarly treated in the second round. All 400 dipeptides are thereby synthesized. Obviously, by using 400 vertical stripes and 400 horizontal stripes, all 160,000 tetrapeptides could be synthesized. A resolution of 50 microns was achieved by Fodor, et al. (His detection system was said to have a sensitivity limit of about 100 fluorescein molecules in a 10 square micron region.)

The result of the combinatorial masking strategy is the formation of a large number of compounds, distributed across the support. However, in any given "synthesis area", i.e., a commonly treated area of the support, one product predominates.

In the present modification of Fodor's method, the entire support is subjected to one or more rounds of reaction with a mixture of amino acids. Then one or more additional amino acids are added to each growing peptide, using Fodor's method. The result is that within a synthesis area, one finds not a single peptide sequence, but rather a family of related peptides having a common amino terminal, and a herterogeneas carboxyterminal.

Unlike the "bead" embodiment, there is no need to sequence this amino terminal, as the sequence of the terminal is deducible from the coordinates of the assay-positive synthesis area. The active peptides within the known family may be determined by synthesizing a secondary library, corresponding to the family of the primary library, and assaying for binding. If a support is divided into 50 micron square synthesis areas, each area is expected to display 100,000 - 1,000,000 peptide molecules. If 100 molecules per 50 micron square synthesis area are required for detection, each such synthesis area may present 1,000 - 10,000 different peptide sequences. Thus, our adaptation of Fodor, et al.'s method allows it to explore a 1,000 - 10,000 fold more diverse universe of peptides.

### Screening

The peptide library is screened by exposing the peptide-bearing beads to an affinity reagent as previously described. The reagent will become bound to beads bearing peptides having an affinity for the reagent. Excess reagent is removed and a signal, e.g., fluorescence or a color change, is produced to distinguish the interacting beads from the passive beads.

The interacting beads are then removed, either manually, or by other means which detect either the presence of the reagent, or the generation of the signal. In one embodiment, a sorting reagent is employed which comprises magnetic beads coupled to antibodies (or other binding molecules) which bind the affinity reagent. For example, if the affinity reagent is a rabbit polyclonal antibody, the magnetic beads could be coupled to goat anti-rabbit antibodies. Preferably, the magnetic beads are of a mass substantially lower than that of the library beads, so as to reduce the risk of shearing the complex. However, the less massive the bead, the greater the magnetic field required for efficient separation. Preferably the beads have a mass of about 10⁻¹¹g to 10⁻¹³g. The diameter of the magnetic beads matters as well. Large and light magnetic beads would suffer higher shear forces a compared to small and compact beads having the same mass. This is due to larger drag forces affecting the larger beads from fluid movements. Most of the magnetic beads available on the market are composed of derivatives of polystyrene. They do not have similar densities since they vary in the amount of metal in the bead. The most advanced magnetic sorter available, the MACS from Beckton and Dickinsion, utilizes magnetic beads of about 0.1 micron. B & D use a very powerful magnet. Lesser machines, having less powerful magnets, use bigger beads. We believe however, that beads with about 1 micron diameter might be especially suitable.

Rather than use separate sorting and affinity reagents, it is possible to use a sorting reagent which mimics the target of interest and therefore binds the peptides directly. The "signal" is then the separation of the bead by the sorting reagent.

### Sequencing

"Positive" beads (those which carry binding peptides) are collected and individually sequenced. While a variety of sequencing techniques are known, all contemplate (a) cleaving off a single amino acid from one end of the peptide, (b) collecting and identifying the released amino acid, and (c) repeating steps (a) and (b) until the entire peptide has been sequenced, or further sequencing becomes impractical. Normally, sequencing is performed only on homogeneous peptide preparations. However, while a single bead bears a mixture of peptides, all of the peptides of that mixture have a common terminal portion, the "familial" portion, which is sequencable.

The "familial" portion of the peptides of the library may be the amino terminal portion, in which the sequential degradation must begin at the amino terminal, or it may be the carboxy terminal portion, in which case sequencing begins at the carboxyl end of the peptide.

The primary sequence of amino acids in a peptide or protein is commonly determined by a stepwise chemical degradation process in which amino acids are removed one-by-one from one end of the peptide, and identified. In the Edman degradation, the N-terminal amino acid of the peptide is coupled to phenylisothiocyanate to form the phenylthiocarbamyl (PTC) derivative of the peptide. The PTC peptide is then treated with strong acid, cyclizing the PTC peptide at the first peptide bond and releasing the N-terminal amino acid as the anilino-thiozolinoe (ATZ) derivative. The ATZ amino acid, which is highly unstable, is extracted and converted into the more stable phenylthiohydantoin (PTH) derivative and identified by chromatography. The residual peptide is then subjected to further stepwise degradation.

For carboxy terminal sequencing (of peptides synthesized with their amino terminal coupled to a support), the cleavage reagent may be a carboxypeptidase.

The present invention is not limited to any particular method of sequencing; however the method chosen must be reasonably capable of identifying the sequence of the familial portion of the family of peptides on a single bead.

### Sequencing of branching peptides

The N-terminal sequencing procedure is standard. The difficulty is in identification of the correct structure. If we know that a certain peptide is branched and we know the degree of branching (biantenary, triantenary, etc.) and its location relative to the sequence, we would be able to deduce the correct structure based upon data collected from the N-terminal sequencing and our knowledge of the secondary synthesis approach. However, if we allow a library to contain both linear and branched peptides, or if in a branched library we would allow random branching, it would be very difficult to guess the structure based solely upon sequence information coupled with a limited number of secondary libraries.

The best approach would probably be to design independent linear or branching libraries and to design each branching library with a single architecture.

The utilization of the full potential of the branching approach would be dependent upon use of N-terminal orthogonal protection of each branch. Otherwise, many possible structures would fail to appear within the library.

### Sequencing of cyclic peptides

Sequencing of cyclic peptide produced by intra-chain cystine formation is straightforward, following reduction of the disulfide bonds. With some forms of cyclization, determination of N-terminal sequence by Edmann degradation is not possible and thus the cyclization approach would have to be accompanied by an encoding procedure.

### Encoding

Certain amino acids (e.g., serine, histidine) are difficult to identify by the standard procedures, either because the AA are destroyed by the Edmann degradation procedure or because adequate references for their identification are not available. This problem may be overcome by any of several means:
(a) increasing the proportion of the difficult amino acids in mixed amino acid reagents used in the course of peptide synthesis;
(b) use of more than one sequencing procedure; an amino acid which is difficult to analyze by one procedure may be easier to detect by another.
(c) labeling difficult amino acids with a detectable but non-interfering label prior to adding them to the nascent peptide during peptide synthesis; and/or
(d) in the substitution set for a given residue position in the peptides of a single bead, partnering each difficult-to-sequence amino acid with a readily sequencable amino acid ("encoding").

Alternative (a), while conceivably workable, would create an imbalance in the structure of the intended library.

Alternative (b) requires splitting the bead in two and subjecting each half to a different sequencing procedure, since these procedures are destructive.

Alternative (c) is practical if the label is non-interfering and is not modified adversely by the synthesis or sequencing procedure.

Alternative (d) is preferred, and deserves further explanation. Consider a bead in a peptide library which bears peptides having the sequence
F₁ - F₂ - F₃ - I₄ - I₅ - I_{6,}
where Fₙ are "familial" residues and Iₙ are "individual" residues. Under normal usage, the "F" residues are unique for a given bead. Suppose, however, that one or more of Fₙ are difficult-to-sequence amino acids, such as Tryptophan. If so, then the sequencing of the peptides on this bead will not yield useful results, as the amino acid in that position would be undetermined. While one could exhaustively test all the possibilities, there is an alternative.

Suppose that only F₁ was Tryptophan. One could instead structure the library so that on this bead, F₁ was either Tryptophan or an easy-to-sequence amino acid, such as glycine. If this bead were found to be positive, amino-terminal sequencing would reveal the sequence
Gly - F₂ - F₃
for the first three amino acids of the peptides on the bead. The actual binding peptide could by Gly - F₂ - F₃, or it could be Trp- F₂ - F₃. The answer would be determined by screening a secondary library which corresponds to the family of peptides found on this positive bead.

A typical usage of "encoding" would be in screening D-amino acids, as conventional sequencing does not distinguish D- and L-forms of the same amino acid. Technically it is possible to separate using a proper column, between D and L amino acids, or in a pure chiral solution to determine their optical nature. However, all of the known methods need much larger amounts of material for analysis than are obtained when sequencing a single bead. Thus, it is impractical to determine the chiral identity of the sequenced amino acids from individual library beads. Another use for "encoding" would be to distinguish Glu from Gln, or Asp from Asn.

Thus, according to our strategy we incorporate in the "familial" positions (e.g., 1-3 from the N-terminus) of the primary library, two amino acids. The amino acid pairs are selected so that each "difficult" amino acid is paired with an "easy" one. When sequencing is performed, the signal generated by the "easy" amino acid indicates the existence of the "difficult" one even though it is not registered by the sequencer.

In short peptides, the N-terminal might influence the activity of the peptide. e.g. When the peptide is composed of mostly hydrophobic amino acid residues, the hydrophilic primary amine of the N-terminal might disrupt the peptide activity. However, it is not possible to sequence N-terminal blocked peptides by Edmann degradation.

The encoding strategy may be used to analyze the structure of N-terminal modified peptides. In the final synthesis cycle, we use a mixture of FMOC protected (α-amine blocked by FMOC) and blocked (α-amine blocked with acetic or benzoic acid) amino acids. Upon sequencing, the signal obtained from the N-terminal amino acid implies the existence of the blocked N-terminal amino acid which is stable to the Edmann degradation.

### Non-Peptide Libraries

Polymers other than peptides may be used in the structured libraries of the present invention, provided, (a) they can be synthesized, in a manner permitting "structuring", (b) when so presented, they are bindable by a target material, and (c) the polymer molecules on a single bead may be sequenced, at least partially. Suitable polymers include peptoids, nucleic acids, and carbohydrates.

It should be noted that if a particular type of polymer cannot be sequenced readily, it can be studied indirectly by means of an "encoding" strategy in which the beads carry both peptides and the non-peptide polymer. Cf. Brenner and Lerner, "Encoded Combinatorial Chemistry," PNAS (USA), 89:5381-83, (1992), who disclose chemically linking a "genetic tag" (amplifiable by PCR) to a polymer which is not itself genetically encodable. The peptide need not, however, be chemically linked to the non-peptide polymer in the present method. For example, a library may be structured so that on a given bead, there is a single peptide sequence, and a family of related nucleic acid sequences. Sequencing the peptide then identifies the nucleic acid family.

It may be desirable to present difficult-to-sequence polymer libraries by our adaptation of the light-directed, spatially addressable parallel chemical synthesis method of Fodor, et al., as the familial sequence is then indicated by the spatial address.

Peptoids are peptide analogues in which the peptide bond (-NHCO-) is replaced by an analogous structure, e.g., -NRCO-. See Simon, et al., P.A., "Peptoids: A modular approach to drug discovery." *Proc. Natl. Acad. Sci. USA* 89:9367-9371, 1992, and c.f. Gilon, et al, "Backbone cyclization: A new method for conferring conformation constraint on peptides." *Biopolymers* 31:745-750, 1991. When the polymer is a peptoid, it may be synthesized as described in the Reference Example. In general, these peptoids are sequenceable just as peptides are, though the sensitivity or accuracy may be different.

When the polymer is a nucleic acid, conventional DNA or RNA synthesis and sequencing methods may be employed. The usual bases are the purines adenine and guanine, and the pyrimidines thymidine (uracil for RNA) and cytosine. However, unusual bases, such as those listed below, may be incorporated into the synthesis or produced by post-synthesis treatment with mutagenic agents.
4-acetylcytidine.
5-(carboxyhydroxylmethyl)uridine.
2'-O-methylcytidine.
5-carboxymethylaminomethyl-2-thioridine.
5-carboxymethylaminomethyluridine.
dihydrouridine.
2'-O-methylpseudouridine.
beta,D-galactosylqueosine
2'-O-methylguanosine.
inosine.
N6-isopentenyladenosine.
1-methyladenosine.
1-methylpseudouridine.
1-methylguanosine.
1-methylinosine.
2,2-dimethylguanosine.
2-methyladenosine.
2-methylguanosine.
3-methylcytidine.
5-methylcytidine.
N6-methyladenosine.
7-methylguanosine.
5-methylaminomethyluridine.
5-methoxyaminomethyl-2-thiouridine.
beta,D-mannosylqueosine.
5-methoxycarbonylmethyluridine.
5-methoxyuridine.
2-methylthio-N6-isopentenyladenosine.
N-((9-beta-D-ribofuranosyl-2-mehtylthiopurine-6-yl)carbamoyl)threonine.
N-((9-beta-D-ribofuranosylpurine-6-yl)N-methylcarbamoyl)threonine.
uridine-5-oxyacetic acid methylester.
uridine-5-oxyacetic acid (v).
wybutoxosine.
pseudouridine.
queosine.
2-thiocytidine.
5-methyl-2-thiouridine.
2-thiouridine.
4-thiouridine.
5-methyluridine.
N-((9-beta-D-ribofuranosylpurine-6-yl)carbamoyl)threonine.2'-O-methyl-5-methyluridine.
2'-O-methyluridine.
wybutosine.
3-(3-amino-3-carboxypropyl)uridine.

DNA may be synthesized by the stepwise addition of nucleotides to a nascent chain. The first step of the synthesis may be the coupling of a nucleoside, via a succinyl linkage, to a suitable support, such as cellulose. This nucleoside represents the 3' end. Chain elongation proceeds from 3' to 5'; each cycle being composed (in one conventional method) of the following steps:
(1) Selective deprotection
   For example, if the 5'-hydroxyl is protected by a dimethoxytrityl group, it is removed with acid.
(2) Condensation
   A protected nucleotide is coupled to the exposed 5' end. The protected nucleotides may be 5'-O-dimethoxytrityl-N⁶-(benzoyl)-2'-deoxyadenosine, 5'-dimethoxytrityl-N⁴-(anisoyl)-2'-deoxycytidine, 5'-O-dimethoxytrityl-N⁶-(N',N',-di-n-butyl formadine)-2'-deoxyadenosine, and 5'-O-dimethoxytrityl-N²-(propionyl)-O⁶-(diphenylcarbamoyl)-2'-deoxyguanosine.
(3) Capping
   Unreacted 5'-hydroxyl groups are protected, e.g., by acylation.

The traditional method for DNA sequencing by chemical cleavage depends on the parallel execution of four base-specific or base-selective modification protocols and the parallel electrophoretic resolution of the hydrolysates in four lanes. It is also possible to analyze DNA based on a single base modification procedure, if it produces some degree of backbone cleavage at all bases in the DNA but the rates of cleavage at the four canonical bases (A, T, G, C) are clearly different. See Ambrose and Pless, Meth. Enzymol., 152:522 (1987) (modification with 0.5M aqueous piperidine, 0.3M NaCl, 90°C., pH > 12, 5 hrs). The single reagent method is faster but less accurate.

Polysaccharides are larger polymers of monosaccharides in a branched or unbranched chain. Oligosaccharides are shorter polymers of monosaccharides, such as di-, tri-, tetra-, penta-, and hexasaccharides. For the sake of convenience, the term "polymeric carbohydrate" will be used to cover both poly- and oligosaccharides.

Monosaccharides in a polymeric carbohydrate library may be aldoses, ketoses, or derivatives. They may be tetroses, pentoses, hexoses or more complex sugars. They may be in the D- or the L-form. Suitable D-sugars include D-glyceraldehyde, D-erythrose, D-threose, D-arabinose, D-ribose, D-lyxose, D-xylose, D-glucose, D-mannose, D-altrose, D-allose, D-talose, D-galactose, D-idose, D-gulose, D-rhamnose, and D-fucose. Suitable L-sugars include the L-forms of the aforementioned D-sugars.

A sugar hemiacetal may be reacted with a hydroxyl group of another sugar to form a disaccharide, and the reaction may be repeated. For carbohydrate synthesis methods, see Kanie, O. and Hindsgaul, O., "Synthesis of Oligosaccharides, Glycolipids and Glycopeptides," Curr. Opin Struc. Bio., 2:674-681, (1992). For sequencing, see Y.C. Lee, "Review: High-Performance Anion-exchange Chromatography for Carbohydrate Analysis," Anal. Biochem. 189:151-162, (1990); Maley, F., Trimble, R.B., Tarentino, A.L., Plummer, T.H., "Review: Characterization of Glycoproteins and Their Associated Oligosaccharides Through the Use of Endoglycosidases," Anal. Biochem., 180:195-204, (1989); and Spellman, M.W., "Carbohydrate Characterization of Recomibianant Glycoproteins of Pharmaceutical Interest," Anal. Chem. 62:1714-1722, (1990).
Special constructs which have been described recently include:
*Hybrid Polypeptide/Nucleic Acids*: In this type of amino acid derivative, the R group of the Cα is a nucleotide residue. The backbone may be a regular polypeptide and thus we assume that there should be no difficulty in synthesis and in Edmann degradation. See Meier, C. and Engels, J.W. Peptide nucleic acids (PNAs) -- "Unusual properties of nonionic oligonucleotide analogs," *Angew*. *Chem*. *(Engl)* 31:1008-1010, 1992; Egholm, M., Buchardt, O., Nielsen, P.E. and Berg, R.H., "Peptide nucleic acids (PNA). Oligonucleotide analogs with an achiral peptide backbone," *Journal of the American Chemical Society* 114:1895-1897,
1992. *Mixed" polymers of amino acids and other monomers:* A single chain comprising amino acids and other monomers (e.g., nucleic acids) may be prepared.

### Example 1

In this example, we describe a hexapeptide library in which each residue is chosen from a set of 25 amino acids. The library has a maximum possible "diversity" of 25⁶, or about 2.44 x10⁸ different peptide sequences.

While considering the library as a whole, each residue position of the hexapeptides may be any of the 25 residues of the set, the library is structured so residues 1-3 (numbered from the amino terminal) are familial residues, and residues 4-6 are individual residues. Unless "encoding" is needful, as explained previously, for the peptide family on a single bead, the first three residues will be the same, the diversity being apparent only in residues 4-6. (Of course, residues 1-3 will vary from bead to bead).

This library is constructed as follows. A mixture is prepared of 25 different, amino-protected amino acids. For three amino acid addition cycles, this mixture is reacted with all of the beads (i.e., an unstructured random cycle, abbreviated as "UR" in Table 1). As a result, each bead has a multitude of random tripeptides (positions 4-6 of the desired hexapeptides). Thus, there are 25³ possible different tripeptide sequences.

The remaining three cycles are structured random ("SR") cycles. In the fourth cycle, the beads are divided into 25 aliquots. The first aliquot is reacted with L-Ala, the second with L-Arg, the third with L-Asp, and so on through all 25 aliquots. We now have synthesized all 25⁴ possible different tetrapeptide sequences. However, the amino terminal amino acid of all of the peptides on the beads of the first aliquot is L-Ala, while the amino terminal amino acid of all of the peptides of the second aliquot is L-Arg. (This amino acid will be residue 3 of the final hexapeptide). All of the beads are now mixed together randomly. In the fifth cycle, the beads are once again divided into 25 aliquots, and each aliquot reacted with a particular amino acid to yield pentapeptides on the beads. This is residue 2 of the final hexapeptide. The beads are packed and "shuffled," and, in the sixth and last cycle, divided into 25 aliquots to receive the final amino acid (residue 1 of the hexapeptide). The beads of the library now bear all 25⁶ possible hexapeptides, but the synthesis has been structured so that residues 1-3 (counting from the amino terminal) of the peptides are identical for the peptides on a given bead.

The synthesis plan is summarized in the table below:

**Table 1**

| Cycle Type | Cycle | Residue | DF(cycle) | PF(cycle) |
|---|---|---|---|---|
| SR | 6 | 1 | 1 | 25 |
| SR | 5 | 2 | 1 | 25 |
| SR | 4 | 3 | 1 | 25 |
| UR | 3 | 4 | 25 | 1 |
| UR | 2 | 5 | 25 | 1 |
| UR | 1 | 6 | 25 | 1 |
| overall | | | 25³ | 25³ |

The sequence set statistics for this library appear below:

| | Size | Diversity | Sampling |
|---|---|---|---|
| bead | 6 x 10¹³ | ö 15,625 (25³) | = 4 x 10⁹ |
| library | 6 x 10²⁰ | ö 2.44 x 10⁸ (25⁶) | = 2.5 x 10¹² |

The safety factor analysis follows:

| Beads-in Library | Library PF | Assumed Detection Limit | Beads-in-Library Safety Factor |
|---|---|---|---|
| 10⁷ | ö 15,625 | ö 1 bead per library | = 640 |

| Sampling level Per Bead | Assumed Detection Limit | Peptide-on-Bead Safety Factor |
|---|---|---|
| 4 x 10⁹ (molecules per sequence per bead) | ö 10⁶ (binding molecules per bead) | = 4 x 10³ |

On a single bead, there are 4x10⁹ identical molecules of each different hexapeptide sequence, which is well above the assumed binding detection limit of 10⁷ molecules. For the first three residue positions, there are 100 picomoles of each amino acid, which comfortably exceeds the 10 picomoles assumed to be required for sequencing.

25³ (15,625) different beads would be required to have at least one bead for each of the 25³ possible permutations of the first three residue positions. If 10⁷ beads are employed, there will be 10⁷/25³ or about 640 beads in the library bearing the identical familial sequence.

If one of the beads was detected as "positive" when the library was assayed, the peptide molecules on it would be sequenced. All of them would have the same first three amino acids, in 100 pmole quantities, which is sufficient for sequencing. Thus, these three amino acid positions could be determined. However, it would not yet be known which of the 25³ different peptide sequences on that bead was responsible for the binding activity.

To find out, a secondary library is made. All peptides of this library have residues 1-3 in common. All peptides on a given bead have residues 4-6 in common. If the assay on the secondary library "marks" a bead, all of the peptide on this bead will have residues 1-6 in common, in 100 pmole quantities. Thus, sequencing is feasible, and the active peptide is then fully identified.

It should be evident that larger active peptides may be determined by further iterative steps.

### Example 2

In this example, a 10⁷ bead library of all of the 100⁶ hexapeptides formable from 100 different amino acids is prepared.

Suppose 10⁷ beads are subjected to three unstructured cycles, in each of which the beads are reacted with a mixture of 100 different amino acids, and three structured cycles in each of which the beads are divided into 100 aliquots, each aliquot is reacted with a single unique amino acid out of the set of 100 amino acids, and the aliquots are pooled back together after each cycle.

The synthesis plan this time is:

**Table 2**

| Cycle Type | Cycle # | Residue # | Cycle DF | Cycle PF |
|---|---|---|---|---|
| SR | 6 | 1 | 1 | 100 |
| SR | 5 | 2 | 1 | 100 |
| SR | 4 | 3 | 1 | 100 |
| UR | 3 | 4 | 100 | 1 |
| UR | 2 | 5 | 100 | 1 |
| UR | 1 | 6 | 100 | 1 |
| overall | | | 10⁶ | 10⁶ |

This results in the following sequence set statistics:

| | Size | Diversity | Sampling |
|---|---|---|---|
| Bead | 6 x 10¹³ | 10⁶ | 6 x 10⁷ |
| Library | 6 x 10²⁰ | 10¹² | 6 x 10⁸ |

The relationship of the sequence set statistics to the varying detection limits may be expressed through calculation of "safety factors", as follows: bead-in-library

| beads-in-library | library PF | safety factor |
|---|---|---|
| 10⁷ | ö 10⁶ | = 10 |

| sampling level per bead | assumed detection limit | peptide-on-bead safety factor |
|---|---|---|
| 6 x 10⁷ | ö 10⁶ | = 60 |

The derivation of these numbers is explained in greater detail, below.

The diversity of this library (D_{L}) is 100⁶ (10¹²). If there are 10⁷ beads in the library (B_{L}), and 6x10¹³ molecules/bead (M_{B}), there are 6x10²⁰ molecules in the library (M_{L}). It is therefore possible to have (6x10²⁰/10¹² = 6x10⁸) identical molecules of each unique peptide sequence.

Each bead has a diversity of 100³ (10⁶). With 6x10¹³ molecules on the bead, the expected number of identical molecules per bead is (6x10¹³/10⁶ = 6x10⁷), which is well above the 10⁶ believed necessary for detection. There are also 100³ (10⁶) different synthetic "paths" taken by the beads during the structured cycles, and therefore the expected number of beads which underwent any given path is (10⁷/100³ = 10), well above the desired minimum level of 1-2. Finally, 6x10¹³ hexapeptide molecules per bead is the equivalent of 100 picomoles/bead. Since all molecules on a single bead have the same first three amino acids, this initial tripeptide is present at a concentration of 100 picomoles, whereas 25 is deemed desirable for sequencing.

Thus, the foregoing demonstrates the practicality of screening hexapeptide library with each residue chosen from a set of 100 different amino acids.

### Example 3

Another way of synthesizing a library of all possible hexapeptides which could be prepared from 100 different amino acids is by a combination of six partially structured random cycles.

In cycles 1-3, residues 4-6 are provided. In each cycle, the beads are divided into four aliquots A, B, C and D. Aliquot A is reacted with amino acid mixture A' (AAs 1-25), aliquot B with mixture B' (AAs 26-50), C with C' (AAs 51-75), and D with D' (AAs 76-100). At the end of each cycle; the aliquots are repooled.

In cycles 4-6, residues 1-3 are added. In each cycle, the beads are divided into 50 different aliquots, and each aliquot is reacted with a unique mixture of two of the 100 different amino acids. Where possible, a difficult-to-sequence amino acid is paired with an easy-to-sequence amino acid.

This synthetic plan is summarized below:

**Table 3**

| Cycle Type | Cycle | Residue | Cycle DF | Cycle PF |
|---|---|---|---|---|
| SR | 6 | 1 | 2 | 50 |
| SR | 5 | 2 | 2 | 50 |
| SR | 4 | 3 | 2 | 50 |
| SR | 3 | 4 | 25 | 4 |
| SR | 2 | 5 | 25 | 4 |
| SR | 1 | 6 | 25 | 4 |
| overall | | | 50³ | 200³ |

The sequence set statistics and library safety factors are as follows:

| | Size | Diversity | Sampling Level |
|---|---|---|---|
| bead | 6 x 10¹³ | 125,000 | 4.8 x 10⁸ |
| library | 6 x 10²⁰ | 10¹² | 6 x 10⁸ |

| Beads-in-Library | Library PF | Bead-in-Library Safety Factor |
|---|---|---|
| 10⁷ | ö 8 x 10⁶ | = 1.25 |

| Sampling level Per Bead | Assumed Detection Limit | Peptide-on-Bead Safety Factor |
|---|---|---|
| 4.8 x 10⁸ | ö 10⁶ | = 480 |

If each bead takes up, in a given cycle, 100 pmoles of amino acid, residues 1-3 will be sequenceable, as each of two possible amino acids will be present in a concentration of (100/2=) 50 pmoles (five-fold more than the 10 pmoles assumed necessary for sequencing). The diversity per bead will be 2³x25³, or 125,000. The expected number of identical molecules of each sequence will be (6x10¹³/125,000), or 5x10⁸.

The partitioning factor of the library will be 50³x4³, or 8x10⁶. With 10⁷ beads in the library, the expected number of beads representing each possible partitioning permutation will be (10⁷/8x10⁶=) 1.25.

### Example 3A: Screening of the primary library for TNF binding.

To illustrate the technique, the above library is screened for beads which specifically bind TNF as follows: The beads are mixed with a solution containing TNF (Tumor Necrosis Factor) at a concentration of 10 µg/ml in 5% low fat milk buffer. Following washing with phosphate-buffered saline (PBS), the beads are incubated with rabbit antibodies specific for TNF, washed as before and incubated with antibodies specific to rabbit immunoglobulin which are conjugated with alkaline phosphatase (anti-rabbit Ig-alkaline phosphatase). After washing, the beads are exposed to the substrate BCIP (5-bromo-4-chloro-3-indolyl phosphate). Those beads which bound the ligand and/or the immunoglobulins are stained blue.

The stained beads are collected with a micropipetor, under a microscope and destained by DMF treatment. The bound proteins (TNF and antibodies) are removed by washes with 0.1N HCl. The destained beads are reacted with the anti-TNF and anti-rabbit IG-alkaline phosphatase conjugate without preincubation with TNF. Some of the beads, which are stained by the antibodies are removed. The peptides on the beads stained at this stage apparently bind to the antibodies or to the alkaline phosphatase and do not bind TNF. (Of course, a different target could be substituted for TNF).

### Example 3B: Screening the selected TNF-binding beads of the primary library for TNF-TBP1-complexes binding.

The unstained beads from the previous stage are reacted with the TNF as before and then reacted with TBP1 (TNF Binding Protein p55, or soluble TNF receptor type 1). The beads are then reacted with rabbit antibodies specific to TBP1 and with the anti-rabbit Ig antibodies conjugated with alkaline phosphatase as before. The beads are then stained by reaction with BCIP. The blue beads apparently bind TNF in an orientation which allows the bound TNF to bind TBP1. The unstained beads apparently bind TNF in an orientation which blocks the active site. The beads which were unstained when exposed to the TBP1 and its antibodies, are then reacted with antibodies to TNF as before in order to verify that they still bind the TNF. The stained beads are then subjected to N-terminal sequencing. The peptides on these beads inhibit TNF activity by inhibiting its binding to the soluble Type 1 receptor, the TBP1. Since the soluble receptor is the extracellular portion of the cell surface receptor, the peptide, by binding also to the latter, should inhibit the binding of TNF to the receptor and thereby ameliorate the harmful influence of TNF.

### Example 3C: The secondary library

The sequencing of each bead selected from the primary library yields two amino acids for each of the 1-3 positions. In the current example, each position contains 2 amino acids. Therefore, there are 8 different possible tripeptides. For each three amino acids sequence obtained from analysis of the selected beads, we synthesize 8 different secondary libraries, each expressing a single tripeptide of the possible 8. Positions 4-6 of each of the 8 secondary libraries contain two amino acids per position, as performed for positions 1-3 in the initial library. each secondary library contains 10⁶ beads in order to allow full expression of all possible hexapeptides from the set of 100 different amino acids used to construct the library.

The 8 secondary libraries, based upon the results of the sequencing of the first 3 positions in a selected bead obtained from the primary library, are synthesized according to the following table:

**Table 3c**

| Cycle | Residue | Cycle DF | Cycle PF |
|---|---|---|---|
| 6 | 1 | 1 | 1 |
| 5 | 2 | 1 | 1 |
| 4 | 3 | 1 | 1 |
| 3 | 4 | 2 | 50 |
| 2 | 5 | 2 | 50 |
| 1 | 6 | 2 | 50 |
| overall | | 8 | 50³ |

The partitioning factor for the library is 50³ (125,000), for which 10⁶ beads are adequate. The diversity of each bead is only 8, residues 4-6 are sequenceable (residues 1-3 are identical throughout a given secondary library). The diversity of the secondary library is 100³.

Each of the secondary libraries thus synthesized is probed as described above for probing of the primary library. The identity of the most highly stained library indicates the exact sequence of the tripeptide in positions 1-3 from the N-terminal. The most darkly stained beads from the library are selected and subjected to N-terminal sequencing. The sequence of the first 3 positions should be known from the synthesis of this library (one of the 8 possible peptides), and the sequence information for positions 4-6 (two possible amino acids in each), is the basis for the tertiary library which is described below.

### Example 3D: The tertiary library

Since the diversity of each bead in the secondary library is only 8, the sequencing of the peptides on an active bead will reveal that one or more of the 8 possible sequences is an active sequence. The tertiary library described in this example has a dual purpose: identification of the exact sequence in residues 4-6; and exploration of those nonapeptides, formed of the same 100 amino acids, which begin with the active hexapeptide sequence.

There are eight tertiary libraries, each representing one of the eight possible sequences at residues 4-6 for the active bead from one of the secondary libraries of the last example. for a given tertiary library, the synthetic plan is as follows:

**Table 3d**

| Cycle | Residue | DF(bead) | PF(library) |
|---|---|---|---|
| 9 | 1 | 1 | 1 |
| 8 | 2 | 1 | 1 |
| 7 | 3 | 1 | 1 |
| 6 | 4 | 1 | 1 |
| 5 | 5 | 1 | 1 |
| 4 | 6 | 1 | 1 |
| 3 | 7 | 2 | 50 |
| 2 | 8 | 2 | 50 |
| 1 | 9 | 2 | 50 |
| overall | | 8 | 50³ |

This will yield a bead diversity of 8 and a library diversity of 100³. If there is an active bead, residues 4-6 will be known (by virtue of knowing which tertiary library the bead was in), and residues 7-9 will be limited to one of eight possibilities (as shown by sequencing those residues of the bead).

It should be apparent that it would be possible to synthesize the next eight quaternary dodecapeptide libraries to identify residues 7-9 while exploring possibilities at positions 9-12, and so forth through further generations of libraries.

### Example 4

In this example, an octapeptide library is presented. For the purpose of this example, we assume that there are 10⁸ beads in the library, 6 x 10¹³ peptides (100 pmoles) per bead, a detection limit of one active bead with 10⁶ active molecules thereon, and a sequence limit of 10 pmole/residue per bead. The library is constructed as follows:

**Table 4**

| Type | Cycle | Residue | DF | PF |
|---|---|---|---|---|
| SR | 8 | 1 | 1 | 40 |
| SR | 7 | 2 | 1 | 40 |
| SR | 6 | 3 | 1 | 40 |
| SR | 5 | 4 | 1 | 40 |
| UR | 4 | 5 | 40 | 1 |
| UR | 3 | 6 | 40 | 1 |
| UR | 2 | 7 | 40 | 1 |
| UR | 1 | 8 | 40 | 1 |

This library therefore has the following statistics:

| | Size | Diversity | Sampling | |
|---|---|---|---|---|
| | | | Level | PF |
| Library statistics | 6 x 10²¹ | 6.55 x 10¹² | 9 x 10⁸ | 2.56 x 10⁶ |
| Bead statistics | 6 x 10¹³ | 2.56 x 10⁶ | 2.4 x 10⁷ | |

| | Safety Factor |
|---|---|
| detect bead-in-library | 400 |
| detect peptide-on-bead | 24 |
| sequence residues 1-4 | 10 |

In general, if the number of beads in the library (B_{L}) is within one or two orders of magnitude of the ratio (M_{B}/M_{B}') where M_{B} is the number of molecules per bead and M_{B}' is the molecule-per-bead detection limit, the best strategy is to employ equal numbers of structured and unstructured random cycles, e.g. 4 and 4 for an octapeptide library.

However, this assumption may not always be valid. Suppose that there are 10⁹ beads in the library, but the detection limit is 10⁸ molecules per bead.

A four-and-four strategy would lead to these statistics:

| | Size | Diversity | Safety Level | PF |
|---|---|---|---|---|
| library | 6 x 10²² | 6.55 x 10¹² | 9 x 10⁹ | 2.56 x 10⁹ |
| bead | 6 x 10¹³ | 2.56 x 10⁶ | 2.47 x 10⁷ | - |

| | Safety Factor |
|---|---|
| detect bead-in-library | 400 |
| detect peptide-on-bead | .24 |
| sequence residues 1-4 | 10 |

It would be safer to employ 5 structured and three unstructured random cycles:

| | Size | Diversity | Sampling Level | PF |
|---|---|---|---|---|
| library | 6 x 10²² | 10¹² | 9 x 10⁹ | 2.56 x 10⁹ |
| bead | 6 x 10¹³ | 6.4 x 10⁴ | 9 x 10⁸ | - |

| | Safety Factor |
|---|---|
| detect bead-in-library | 10 |
| detect peptide-in-bead | 9 |
| sequence residues 1-4 | 10 |

### EXPERIMENTAL EXAMPLE A

1. **Synthesis of a model peptide: N-Ala-Leu-Pro (PLA) on Eupergit C resin.** Using the conventional solid phase synthesis with Fmoc protected amino acid we synthesized the PLA sequence on epoxy activated Eupergit C (Rohm, Darmstadt, Germany; alternatively, Spectra/Cryl, Spectrum, Houston, Texas, USA) resin (bead diameter 250µ), after introduction of cystamine as linker, and evaluated the peptide yield per bead by amino acids microsequencing. By this we confirmed our synthesis method and reagents and demonstrated that we can determine the amino terminal sequence of one bead (about 20-40 pmole amino acid per bead). The ability to sequence one bead is essential for the peptide library approach. The average yield per bead of each amino acid from the sequences, based on sequencing 20-30 beads, was as follows:

| Cycle | Amino Acid | Yield (pmole) |
|---|---|---|
| 1 | Ala | 63.9 |
| 2 | Leu | 45.1 |
| 3 | Pro | 31.1 |

2. **Synthesis of a peptide library on the Eupergit C beads.** The library was prepared from 37 different amino acids (the genetically encoded amino acids, except for L-cysteine; their D-forms, except for D-isoleucine; plus L-Norleucine) and constructed from six random (entire library receives a mixture of all the amino acids) steps followed by three structured (each 1/37 of the library gets a single amino acid) steps. This library was used first to test, by sequencing, the introduction of all the different amino acids, when alone or in a mixture. We demonstrated by sequencing that all of the amino acids were represented in the library. The following table sets forth the average yields, in pmoles per bead, obtained by sequencing the beads.

| Cycle & Type | Ala | Arg | Asn | Asp | Cys | Glu | Gln | Gly | His | Ile |
|---|---|---|---|---|---|---|---|---|---|---|
| structured | 133 | 151 | 80 | 244 | N/A | 138 | 106 | 188 | 77 | 195 |
| random | 582 | 125 | 113 | 338 | N/A | 214 | 234 | 253 | 48 | 29 |

| Leu | Lys | Met | Phe | Pro | Ser | Thr | Trp | Tyr | Val |
|---|---|---|---|---|---|---|---|---|---|
| 217 | 191 | 294 | 263 | 131 | 21 | 83 | 78 | 62 | 249 |
| 244 | 155 | 313 | 408 | 234 | 21 | 29 | 27 | 233 | 52 |

In the structured region, most of the amino acids were represented in equimolar amounts (some as Serine gave very low peak due to the sequencing problem), while in the random region the distribution was nonequimolar. There was a deviation from random representation of about an order of magnitude from the best to the worst represented amino acids. The favored amino acids were Alanine, Aspartic acid, Phenylalanine, Methionine and Leucine. The worst represented amino acids were Serine, Threonine, Isoleucine and Tryptophan and Valine. For Isoleucine and Valine we observed that in the first 3 positions, where each amino acid was incorporated alone, they were highly represented, but were less well represented in positions 4-6 where they were incorporated from a mixture. Thus we could distinguish between difficulty in relative coupling efficiency and difficulty in sequencing. Since the N-terminus of the peptides that contains the structured part is unique and is the important part in this type of library, we were not concerned by the differences between the presentation rates of the amino acids at the random part.
3. **Screening of the Eupergit C peptide library with Rhodamine labeled TBP1**. Purified TBP1 (TNF binding protein; soluble extracellular domain of TNF receptor type 1, p55) (recombinant human, CHO produced, affinity purified TBP1 was obtained from InterPharm Laboratories, Ltd.) was labeled with rhodamine and applied to the peptide library. The result was a background pink staining of the beads which probably resulted from the high hydrophobicity of the Eupergit C matrix. We also observed fragility of the beads. Because of these two reasons we decided not to use this resin further and to try a "classical" solid phase synthesis resin, namely polystyrene cross linked with divinyl benzene (PS-DVB).
4. **Synthesis of the PLA model peptide on aminomethylated polystyrene 1% DVB resin**. The tripeptide was synthesized on the DVB-polystyrene resin (bead diameter 150µ) as in paragraph 1, with similar results.
5. **Surface staining and ELISA model experiments with TBP1-conjugated beads**. Purified TBP1 was immobilized onto aminomethylated polystyrene beads (1.5 ml of packed resin was reacted with 1.5 ml of 10% glutaraldehyde for 30 minutes and 1 mg of TBP1 was added conjugated for 1 hour) and the beads were immunostained with an alkaline phosphatase labeled monoclonal antibody (see EP Appl. 412, 486) or with polyclonal rabbit antibodies to TBP1 followed by alkaline phosphatase labeled anti-rabbit IgG.
Two signal generation systems, both mediated by alkaline phosphatase, were tested: surface staining of the beads with the insoluble product produced by the substrate system 5-bromo-4-chloro-indolylphosphate/nitroblue tetrazolium (BCIP/NBT), and the soluble color produced from the para-nitrophenyl phosphate (pNPP) substrate.
In the first system, when staining with monoclonal antibodies, beads (about 50 µl packed) were incubated with 5% FBS (fetal bovine serum) in PBS and then incubated with monoclonal antibody against TBP1 conjugated with alkaline phosphatase at a concentration of 1-10 µg/ml (in FBS/PBS buffer) for 30 minutes. After washes with PBS containing 0.05% Tween-20, 330 µl per tube of BCIP/NBT substrate (Bio Rad kit catalogue number 170-6432, diluted 1:100/1:100 according to the manufacturer's instruction in 0.1 M Tris pH 9.5) were added. When staining with polyclonal antisera, beads (about 50 ul packed) were incubated with 5% FBS (fetal bovine serum) in PBS and then incubated with rabbit polyclonal serum against TBP1 diluted 1:2000 (in FBS/PBS buffer) for 30 minutes. After washes with PBS containing 0.05% Tween 20, alkaline phosphatase conjugated to goat anti rabbit IgG (Bio Makor catalogue number 3471) diluted 1:1000 (in FBS/PBS buffer) was added, for 30 minutes incubation and then washes and addition of substrate as above.
In the second system, the procedure was as above for polycolonal antisera, but the substrate added was para-nitrophenyl phosphate (pNPP, Sigma 104™, tablet of 5 mg in 10 ml of 10mM diethanolamine, pH 9.5 containing 0.5 mM MgCl₂), instead of BCIP/NBT, and the soluble color was produced at 37°C. The resulted O.D. of each sample were monitored at 405 nm. in microtiter plate wells.
The results showed high specific positive surface staining (with BCIP/NBT) of the TBP1-coupled beads as compared to staining of control beads or staining of TBP labeled beads with control antibodies. The background surface staining was essentially nonexistent. We could easily identify and pick up one stained bead from among thousands of unstained beads under a transmission microscope or a reflectance stereo microscope (dissecting microscope). While the soluble substrate detection system was not sensitive enough to allow detection of a single stained bead, it could detect as few as ten positively stained beads in one microliter well, as compared to the control.
Other signal generating systems that were tested included the use of chemiluminescence substrate, staining of the beads with colloidal gold-labeled second antibodies or use of ¹²⁵I-labeled probes. However, none of these systems were sensitive enough to allow detection of signal from single beads. While such a system is not required for the present invention, it does permit one to increase the diversity of the library.
6. **Synthesis of a peptide library on aminomethylated polystyrene beads**. The library was synthesized using the 37 amino acids and constructed of six "semi random" steps and three structured steps to yield: NH₂-1-1-1-2-3-4-5-6-7-COO-NH-CH₂-bead (each number represents the number of different amino acids added to each portion of the beads at given coupling step). This strategy was used in order to increase the number of presented peptides without increasing the library size (volume or number of beads). Each bead is therefore unique in the 3 positions starting at the amino terminus end, contains two different amino acids at position 4, three at position 5 and so on. We believe that there is a need for at least pentapeptide in order to achieve a binding affinity sufficient for immunodetection of the beads.
The amino acids actually used in the semi-random steps were as given below, with "position" being measured from the amino terminal. In the subheadings, such as "9-3", the first number is the residue position and the second number is the group number. Each group is a mixture of amino acids with which an aliquot of beads is reacted. The number of groups is equal to the partitioning factor. Group 9-3 is one of five groups, and each of the groups for position 9 is composed of 7 or 8 amino acids.

| Position 9: | | | | |
|---|---|---|---|---|
| 9-1 | 9-2 | 9-3 | 9-4 | 9-5 |
| L-Ala | L-His | L-Pro | D-Arg | D-Met |
| L-Arg | L-Ile | L-Ser | D-Asn | D-Phe |
| L-Asn | L-Leu | L-Thr | D-Asp | D-Pro |
| L-Asp | L-Lys | L-Trp | D-Gln | D-Ser |
| L-Gln | L-Met | L-Tyr | D-Glu | D-Thr |
| L-Glu | L-Nle | L-Val | D-His | D-Trp |
| Gly | L-Phe | D-Ala | D-Leu | D-Tyr |
| | | | D-Lys | D-Val |

| Position 8: | | | | | |
|---|---|---|---|---|---|
| 8-1 | 8-2 | 8-3 | 8-4 | 8-5 | 8-6 |
| L-Ala | Gly | L-Nle | L-Tyr | D-Gln | D-Phe |
| L-Arg | L-His | L-Phe | L-Val | D-Glu | D-Pro |
| L-Asn | L-Lle | L-Pro | D-Ala | D-His | D-Ser |
| L-Asp | L-Leu | L-Ser | D-Arg | D-Leu | D-Thr |
| L-Gln | L-Lys | L-Thr | D-Asn | D-Lys | D-Trp |
| L-Glu | L-Met | L-Trp | D-Asp | D-Met | D-Tyr |
| | | | | | D-Val |

| Position 7: | | | | | |
|---|---|---|---|---|---|
| 7-1 | 7-2 | 7-3 | 7-4 | 7-5 | 7-6 |
| L-Ala | L-Glu | L-Lys | L-Ser | D-Ala | D-Glu |
| L-Arg | Gly | L-Met | L-Thr | D-Arg | S-His |
| L-Asn | L-His | L-Nle | L-Trp | D-Asn | D-Leu |
| L-Asp | L-Ile | L-Phe | L-Tyr | D-Asp | D-Lys |
| L-Gln | L-Leu | L-Pro | L-Val | D-Gln | D-Met |
| | | | | | D-Phe |

| 7-7 | | | | | |
|---|---|---|---|---|---|
| D-Pro | | | | | |
| D-Ser | | | | | |
| D-Thr | | | | | |
| D-Trp | | | | | |
| D-Tyr | | | | | |
| D-Val | | | | | |

| Position 6: | | | | | |
|---|---|---|---|---|---|
| 6-1 | 6-2 | 6-3 | 6-4 | 6-5 | 6-6 |
| L-Ala | L-Gln | L-Ile | L-Nle | L-Thr | D-Ala |
| L-Arg | L-Glu | L-Leu | L-Phe | L-Trp | D-Arg |
| L-Asn | Gly | L-Lys | L-Pro | L-Tyr | D-Asn |
| L-Asp | L-His | L-Met | L-Ser | L-Val | D-Asp |

| 6-7 | 6-8 | 6-9 | | | |
|---|---|---|---|---|---|
| D-Gln | D-Lys | D-Ser | | | |
| D-Glu | D-Met | D-Thr | | | |
| D-His | D-Phe | D-Trp | | | |
| D-Leu | D-Pro | D-Tyr | | | |
| | | D-Val | | | |

| Position 5: | | | | | |
|---|---|---|---|---|---|
| 5-1 | 5-2 | 5-3 | 5-4 | 5-5 | 5-6 |
| L-Ala | L-Asp | Gly | L-Leu | L-Nle | L-Ser |
| L-Arg | L-Gln | L-His | L-Lys | L-Phe | L-Thr |
| L-Asn | L-Glu | L-Ile | L-Met | L-Pro | L-Trp |

| 5-7 | 5-8 | 5-9 | 5-10 | 5-11 | 5-12 |
|---|---|---|---|---|---|
| L-Tyr | D-Arg | D-Gln | D-Leu | D-Phe | D-Thr |
| L-Val | D-Asn | D-Glu | D-Lys | D-Pro | D-Trp |
| L-Ala | D-Asp | D-His | D-Met | D-Ser | D-Tyr |
| | | | | | D-Val |

| Position 4: | | | | | |
|---|---|---|---|---|---|
| 4-1 | 4-2 | 4-3 | 4-4 | 4-5 | 4-6 |
| L-Ala | L-Asn | L-Gln | Gly | L-Ile | L-Lys |
| L-Arg | L-Asp | L-Glu | L-His | L-Leu | L-Met |

| 4-7 | 4-8 | 4-9 | 4-10 | 4-11 | 4-12 |
|---|---|---|---|---|---|
| L-Nle | L-Pro | L-Thr | L-Tyr | D-Ala | D-Asn |
| L-Phe | L-Ser | L-Trp | L-Val | D-Arg | D-Asp |

| 4-13 | 4-14 | 4-15 | 4-16 | 4-17 | 4-18 |
|---|---|---|---|---|---|
| D-Gln | D-His | D-Lys | D-Phe | D-Ser | D-Trp |
| D-Glu | D-Leu | D-Met | D-Pro | D-Thr | D-Tyr |
| | | | | | D-Val |

7. **First screening of the polystyrene peptide library with TBP1.**
One quarter (400 µl packed) of the total number of beads were blocked with 5% FBS in PBS (1 ml for 45 minutes), to avoid nonspecific binding, and incubated with TBP1 (1 ml at a concentration of 10µg/ml in FBS/PBS for 45 minutes) followed by polyclonal rabbit anti-TBP1 (1:500 in FBS/PBS, 1 ml for 1 hour) and alkaline phosphatase conjugated goat anti-rabbit Ig antibodies (1:2000 in FBS/PBS, 1 ml for 40 minutes). The signal was generated by BCIP/NBT substrate (12 ml in 9 cm petri dish; further details as in 5 above). More than 50 stained beads were selected and three of them were subjected to sequencing. Two additional beads that were selected from a screening of a second quarter of the library with 100 ng/ml TBP1 (signal generated with FAST-RED (4-chloro-2-methylbenzyl diazonium salt supplied by Sigma, St. Louis, Missouri, USA, used in combination with naphtol AS-MX, 3-hydroxy-2-naphtoic acid 2, 4-dimethyl anilide) substrate were also sequenced. The obtained sequences of the five beads are:
1. N-Ala-Cly-Met-(Pro, Phe, Ser?)-(Phe, Leu, Met)
2. N-Ala-Glu-Met-Ser?-Ser?
3. N-Val-Gln-Pro
4. N-Trp-Glu-Pro-Glu
5. N-Ser-Lys-Val-Leu-(Phe, Pro)

8. **Testing the specificity of the selected beads.** The beads that were selected were stained because of their ability to bind either the TBP1, the antibodies to TBP1, the goat antibodies against rabbit IgG or the alkaline phosphatase. It was therefore necessary to distinguish the beads that bind antibodies and alkaline phosphatase from those that bind the TBP1. One way to verify the bead specificity is to destain, and then restain the beads with all the components but without the target ligand, TBP1. The beads that would be stained by the antibodies alone would be removed as non-specific binders.
9. **Destaining and restaining**. The BCIP/NBT substrate that we used in order to stain the beads could not be destained by any treatment that we tried (organic solvents, urea, SDS, NaOH, boiling, sonication and combinations of all of the above), and we therefore decided to use another alkaline phosphatase substrate which could be readily destained. The use of the substrate FAST RED resulted in satisfactory staining, destaining and restaining of model beads (absorbed with alkaline phosphatase labeled IgG). For reasons unknown, stained library beads could not be restained. Replacing the NBT by MTT gave the desired results and beads that were stained could be destained with short DMF wash.
10. **Selection methods**. When screening a small volume (<5 ml packed) of beads library, where the number of stained beads is relatively small, it is possible to pick up the selected stained beads with a forceps or a micropipetor. When a large number of stained beads should be picked up, manual handling might take too much time. We therefore tested several methods for faster and more convenient sorting. We succeeded in separation of positive model bead (TBP1 immobilized covalently) from negative beads by reacting them with smaller magnetic beads coupled to anti-rabbit antibodies. The anti-rabbit antibodies on the magnetic beads bind the TBP carrying beads which were previously reacted with rabbit anti-TBP1 antibodies. The magnetically labeled beads were separated with a magnet. The separation conditions were as follows:
TBP1-beads - as in 5.
control beads-aminomethylated polystyrene beads without modification.
A mixture of 1% TBP1-beads in control beads (total 50 µl beads).
Blocking-1 ml of 5% low fat milk in PBS + 0.05% Tween 20.
Purified rabbit polyclonal serum against TBP1 6 µg/ml in
FBS/PBS - 1 ml for 30 minutes.
Washes with PBS containing 0.05% Tween 20.
Sheep anti rabbit IgG coupled to magnetic beads - (Dynabeads™ M-280, Dynal catalogue number 112.03, 2.8 µm diameter), diluted 1:100 in blocking buffer - 1 ml for 30 minutes.
Magnetic separation + 2 washes using the "Magnetic particle concentrator" MPC®-1 (Dynal catalogue number 120.01).

Using such a system we demonstrated our ability to fish out all of the TBP1 coated beads from an excess of control beads. 99.9% of the beads at the bound fraction were TBP1-beads and only 0.1% were control beads.
11. **Confirmation of the sequence**. The sequencer can not distinguish between L- and D-amino acids. Thus, the sequence information is not complete. Information about the first three amino acids allow for eight (2³) possible sequences and we need to find out which is the correct one. From the first 2 beads which were sequenced we obtained 2 partially overlapping sequences: N-Ala-Cly-Met and N-Ala-Glu-Met. The 12 possible peptides obtained from the sequences of the first two selected beads were synthesized on beads containing random collections of all the amino acids at positions 1-6 from the C-terminal. The 12 peptides (each synthesized on around 100,000 beads) were tested for TBP1 binding by the regular procedure but with the soluble substrate. The peptide N-DAla-DGlu-LMet gave a high OD (ten times higher than background) and the other 11 peptides were lower (between background and three times the background). The specificity of the peptide to TBP1 was also confirmed by lack of response with antibodies alone. The resulting OD in presence of the TBP1 was four times higher than with the antibodies only.
12. **Synthesis of secondary libraries**. The sequence found from the first analyzed bead was used for synthesis of 37 peptides on beads (150 µl packed; about 200,000 beads) primed with 5 random steps. At the fourth place from the amino terminus the beads were divided into 37 groups and each group got a different amino acid. The three terminal amino acids were N-DAla-DGlu-LMet. In this way we got 37 peptides which differ at the fourth position. Staining of the 37 peptides (separately, using soluble substrate) indicated that the L-Serine is the desired amino acid at the fourth position. The process was repeated in order to find the amino acid at position five from the N terminal but at this point we realized some problems, described in the next paragraph, so we are not sure about the results obtained by the soluble substrate approach.
13. **Screening with soluble substrate (ELISA)**. ELISA assays were performed in mini-columns made from polypropylene syringes supported with polyethylene frit. Equal amounts of beads of each tested group are inserted to each column. Reagents and general procedure were as described for the model beads at five for the second system, 200-500 µl of reagent were added to each column at each step. After adding substrate, the columns were placed at 37°C for about 60 minutes and then 200 µl substrate of each column (without the beads) were transfered to a microtiter plate well for O.D. reading at 405 nm.
The use of a soluble staining product for monitoring differences between peptides seems very useful especially since the use of ELISA reader enables discrimination of small differences between staining intensities. However, when we further used it for secondary libraries, results indicated that it is not possible to obtain a positive binding response based upon contribution of only 3 specific amino acids (as compared to contribution of 5-6 amino acids in the original library). Further experiments indicated existence of artifacts resulting from high background observed when using the soluble substrate. The background is apparently attributable to the container, and not the beads. The problem, we believe, results from absorption of proteins to the polyethylene frits of the columns used as reaction vessels. It may be possible to block this absorption and thereby improve results.
14. **Secondary library and sequence confirmation for the sequence**: **N-Ser-Lys-Val**. Eight secondary libraries based on the sequence obtained from screening of the library with TBP1, 100 ng/ml (see 7) were synthesized with the general structure: N-Ser-Lys-Val-1-1-1-R-R-R-Bead, R represents random step, 1 is one of 37 amino acids added to each bead in each step and at the three N positions, each of the eight libraries got one of the possible peptides (combination of the L and D isomers). Each secondary library contained about 50,000 beads intended to include all the possible combinations of the structured three amino acids at positions 4-6 from the N-terminal. The eight libraries were screened with surface staining. The results indicated that for the N terminal sequence: N-DSer-DLys-LVal (group no. 4) bead of the strongly stained beads of group 4 was analyzed and the sequence obtained was: N-Ser-Lys-Val-Lys-Lys.
15. **Specificity of the N-DSer-DLys-LVal peptide**. Several staining steps were performed with the library beads containing the N-DSer-DLys-LVal sequence in order to verify its specificity. Immunostaining performed with and without TBP1 revealed specificity to the antibodies. Further analysis revealed that the above sequence specifically recognized the rabbit immunoglobulins from both rabbit anti-TBP1 antiserum and from normal rabbit serum. Immunoglobulins from other animal species and from humans were not recognized.
16. **Sensitivity of the surface staining**. We experienced some difficulties in obtaining reproducible high surface staining results. No false positive results were ever experienced. We assume that the difficulty resulted from the nature of the substrate used. We attempted to increase the concentration of reagents up to a level which we believed would ensure reproducible results, with low background. Control (without peptides) beads were tested with increasing TBP1 and antibodies concentrations and with different blockers. We have shown that background staining was negligible even with high concentration (e.g., above 10 mg/ml of monoclonals or purified polyclonals, and dilutions of below 1:100 of crude polyclonals) of antibodies when using 5% low fat milk powder instead of 5% FBS, or when TBP1 concentration was 100 ug/ml (when using diluted antibodies, even with FBS blocker). We prefer to perform screening using high ligand concentration (10-100 ug/ml) and to use diluted antibodies in order to select as few as possible immunoglobulin and alkaline phosphatase binding peptides.
17. **Application of the selection procedures in screening for TBP1 binding peptides**. The currently available peptide library (described in par. 6), was stained for TBP1 and antibodies and stained beads were retrieved. The beads were destained and reacted with the antibodies without TBP1. Beads stained with the antibodies were removed. The beads were then reacted with TBP1, followed by reaction with TNF and staining with antibodies to TNF. Those beads that bound TNF were left aside as TBP1-TNF complex recognizers. Such peptides could probably be used for assaying of TBP1-TNF complex formation. The beads were then restained with TBP1 and anti-TBP1 antibodies and the highest stained beads were selected. This staining procedure was repeated a second time in order to ensure specificity. A total of about 15 beads were recovered. Apparently, these beads bind TBP1 at a site that is close enough to the TNF binding site to inhibit the binding of TNF or else the binding of the peptide inhibits TNF binding by an allosteric mechanism. These peptides are candidates for TBP replacement therapy. In diagnostic they can be used in immunoassays for measuring only the free TBP1 (an information that can be very useful) and in production for affinity purification of TBP1 from production fluids.

### EXPERIMENTAL EXAMPLE B

### Model Staining of Peptide Library with Monoclonal Antibody to Human β-Endorphin

This peptide library is a heptapeptide library in which the C-terminal amino acid is constant. The other six positions were varied.

### Materials

### Beads:

a. Beads of high density peptide library #4 which was constructed according to the following parameters:
   Resin Type: polystyrene/divinyl benzene.
   Amino acids used =73
   Total hexapeptides diversity: 1.5 x10¹¹
   Structure:N-2.28-2.28-2.28-5-24-73-L-L-L-Bead(L=linker)(Numbers represent the statistically expected average number of different amino acids incorporated on each bead in the indicated position. Since the groups incorporated in a given cycle may be of different sizes, the average number is not always a whole number. For example, 2.28 is the weighted average of 23 groups with 2.a.a./group and 9 with 3 a.a./group).
   All beads mixed together in one pool.
   Total peptides per bead = About 70,000
   Only about 0.2 repeat of hexapeptide per sequence were used on this experiment, other parts were used in previous experiments.
   In library 4, the groups were as follows (with amino acids identified by ID number): Position 6 (one group)
   Group 1: 1-3, 5, 7-12, 14-23, 28, 29, 31-39, 42-48, 51-66, 68, 70-82, 85-89.
   Position 5 (3 groups)
   Group 1: 3, 7, 8, 9, 14, 15, 18-23, 28, 29, 31-33, 36, 38, 39, 54, 73-76.
   Group 2: 1, 2, 5, 11, 16, 17, 52, 53, 55, 59, 60-62, 65, 71, 72, 77-80, 85-89.
   Group 3: 10, 12, 34, 35, 37, 42-48, 51, 56-58, 63, 64, 66, 68, 70, 81, 82.
   Position 4 (15 groups)
   - Group 1:: 9, 18, 19, 20, 21
   - Group 2:: 28, 29, 58, 73, 74
   - Group 3:: 3, 23, 33, 75, 76
   - Group 4:: 31, 32, 38, 39, 54
   - Group 5:: 7, 8, 14, 15, 36
   - Group 6:: 16, 17, 37, 70, 89
   - Group 7:: 2, 5, 11, 79, 80
   - Group 8:: 1, 71, 72, 65
   - Group 9:: 52, 53, 55, 61, 62
   - Group 10:: 43, 85-88
   - Group 11:: 22, 59, 60, 77, 78
   - Group 12:: 63, 64, 66, 68
   - Group 13:: 34, 35, 42, 48, 51
   - Group 14:: 44-47, 82
   - Group 15:: 10, 12, 56, 57, 81
   Positions 1-3 (32 groups)
   - Group 1:: 11, 20, 21
   - Group 2:: 18, 19
   - Group 3:: 73, 74
   - Group 4:: 28, 29
   - Group 5:: 75, 76
   - Group 6:: 31, 32
   - Group 7:: 38, 39
   - Group 8:: 7, 8
   - Group 9:: 14, 15
   - Group 10:: 16, 17
   - Group 11:: 79, 80
   - Group 12:: 71, 72
   - Group 13:: 52, 53
   - Group 14:: 85, 86
   - Group 15:: 87, 88, 37
   - Group 16:: 59, 60
   - Group 17:: 77, 78
   - Group 18:: 61, 62
   - Group 19:: 63, 64
   - Group 20:: 48, 51, 43
   - Group 21:: 44, 45
   - Group 22:: 56, 57
   - Group 23:: 58, 42
   - Group 24:: 3, 33
   - Group 25:: 68, 66, 1
   - Group 26:: 22, 10, 12
   - Group 27:: 2, 65, 81
   - Group 28:: 46, 47, 23
   - Group 29:: 55, 82
   - Group 30:: 70, 5
   - Group 31:: 89, 34, 35
   - Group 32:: 36, 54
b. Beads of high density peptide library #6 which was constructed according to the parameters of library #4 with the following changes:
   Structure: N-(1-2)-(2-3)-4-5-8-10-Asn-AcaE-Bead
   Total peptides per bead = About 7,500
   The beads of this library were not pooled after the last synthesis cycle (position 1 from the N-terminal) but were left grouped according to their position 1 for the final deprotection and screening steps. Groups containing the N-terminal Tyr (group 11) and Pro (group 12) were checked in this experiment).
The library may be further characterized as follows:
   Position 6 (7 groups)
   - Group 1:: 5, 18-22, 28, 29, 31, 32
   - Group 2:: 1-3, 33, 38, 39, 73-76
   - Group 3:: 9-12, 77-82
   - Group 4:: 43, 56-60, 85-88
   - Group 5:: 23, 44-48, 51, 61-64
   - Group 6:: 34, 35, 52-55, 69, 66, 68, 71, 72
   - Group 7:: 7, 8, 14-17, 36, 37, 42, 70, 89
   Position 5 (9 groups)
   - Group 1:: 18, 19, 28, 29, 73-76
   - Group 2:: 7, 8, 14, 15, 20, 21, 31, 32
   - Group 3:: 63, 64, 66, 68, 71, 72, 77, 78
   - Group 4:: 44-47, 85-88
   - Group 5:: 16, 17, 38, 39, 52-55
   - Group 6:: 10, 12, 34, 35, 56, 57, 59, 60
   - Group 7:: 48, 51, 61, 62, 79-82
   - Group 8:: 22, 37, 42, 43, 58, 65, 70, 89
   - Group 9:: 1-3, 5, 9, 11, 23, 33, 36
   Position 4 (15 groups)
   - Group 1:: 20, 21, 18, 19, 9
   - Group 2:: 73, 74, 28, 29, 58
   - Group 3:: 75, 76, 23, 3, 33
   - Group 4:: 31, 32, 38, 39, 54
   - Group 5:: 7, 8, 36, 14, 15
   - Group 6:: 89, 16, 17, 70, 37
   - Group 7:: 11, 79, 80, 5, 2
   - Group 8:: 52, 53, 55, 61, 62
   - Group 9:: 43, 85-88
   - Group 10:: 22, 59, 60, 77, 78
   - Group 11:: 34, 35, 42, 48, 51
   - Group 12:: 44-47, 82
   - Group 13:: 10, 12, 56, 57, 81
   - Group 14:: 1, 65, 71, 72
   - Group 15:: 63, 64, 66, 68
   Position 3 (18 groups)
   - Group 1:: 18, 19, 28, 29
   - Group 2:: 73-76
   - Group 3:: 20, 21, 31, 32
   - Group 4:: 7, 8, 14, 15
   - Group 5:: 71, 72, 77, 78
   - Group 6:: 63, 64, 66, 68
   - Group 7:: 44-47
   - Group 8:: 85-88
   - Group 9:: 52-55
   - Group 10:: 16, 17, 38, 39
   - Group 11:: 56, 57, 59, 60
   - Group 12:: 10, 12, 34, 35
   - Group 13:: 48, 51, 61, 62
   - Group 14:: 79-82
   - Group 15:: 22, 42, 43, 56, 57
   - Group 16:: 37, 65, 70, 89
   - Group 17:: 1, 5, 9, 11
   - Group 18:: 2, 3, 23, 33, 36
   Position 2 (24 groups)
   - Group 1:: 11, 20, 21
   - Group 2:: 18, 19, 54
   - Group 3:: 28, 29, 36
   - Group 4:: 5, 75, 76
   - Group 5:: 31, 32, 70
   - Group 6:: 38, 39, 42
   - Group 7:: 7, 8
   - Group 8:: 14, 15, 82
   - Group 9:: 9, 77, 78
   - Group 10:: 23, 46, 47
   - Group 11:: 63, 64, 81
   - Group 12:: 48, 51, 43
   - Group 13:: 44, 45, 65
   - Group 14:: 56, 57, 89
   - Group 15:: 68, 66, 22
   - Group 16:: 79, 80, 33
   - Group 17:: 52, 53, 1
   - Group 18:: 16, 17, 55
   - Group 19:: 71, 72, 3
   - Group 20:: 85, 86, 2
   - Group 21:: 87, 88, 37
   - Group 22:: 59, 60
   - Group 23:: 10, 12, 34, 35
   - Group 24:: 73, 74, 61, 62
   Position 1 (40 groups)
   - Group 1:: 20, 21
   - Group 2:: 18, 19
   - Group 3:: 73, 74
   - Group 4:: 28, 29
   - Group 5:: 75, 76
   - Group 6:: 31, 32
   - Group 7:: 38, 39
   - Group 8:: 7, 8
   - Group 9:: 14, 15
   - Group 10:: 16, 17
   - Group 11:: 79, 80
   - Group 12:: 71, 72
   - Group 13:: 52, 53
   - Group 14:: 85, 86
   - Group 15:: 87, 88
   - Group 16:: 59, 60
   - Group 17:: 77, 78
   - Group 18:: 61, 62
   - Group 19:: 63, 64
   - Group 20:: 48, 51
   - Group 21:: 44, 45
   - Group 22:: 56, 57
   - Group 23:: 58, 42
   - Group 24:: 3, 33
   - Group 25:: 68, 66
   - Group 26:: 10, 12
   - Group 27:: 34, 35
   - Group 28:: 46, 47
   - Group 29:: 55, 82
   - Group 30:: 70, 5
   - Group 31:: 11, 23
   - Group 32:: 36, 54
   - Group 33:: 37, 81
   - Group 34:: 1
   - Group 35:: 9
   - Group 36:: 89
   - Group 37:: 65
   - Group 38:: 2
   - Group 39:: 43
   - Group 40:: 22
c. Model beads (TentaGel beads, RAPP Polymere, Germany) carrying the sequences n-Tyr-Gly-Gly-Phe-Leu and n-His-Pro-Tyr-Pro-Pro.
   Monoclonal antibody to human β-endorphin, (Boehringer Mannheim, Germany. Cat. No. 1089 170)¹, reacts with the N-terminal Tyr-Gly-Gly-Phe sequence of human β-endorphin.
   Polyclonal rabbit antibodies against mouse IgG conjugated with alkaline phosphatase (BioMakor, Israel. Cat. No. 3465).
   Blocking Buffer: 1% I-Block (Tropix, MA, USA) in PBS containing 0.05% NaN₃, 0.1% Tween 20, 0.133 g/L CaC1₂.2H₂O and 0.1 g/L MgC1₂.6H₂O.
   Wash buffer: PBS containing 0.05% NaN₃ and 0.1 Tween 20. BCIP, 5-Bromo-4 Chloro-3-Indolyl Phosphate (Sigma, Cat. No. B-0274) 25 mg dissolved in 0.5 ml dimethylformamide.

### Method:

The peptide libraries were synthesized essentially as previously described.

Staining of the beads was performed using the following steps (all incubation steps were performed with continuous mixing of the beads):
a. Washes of the beads with wash buffer.
b. Incubation of the beads with blocking buffer for 45 minutes to block non-specific interactions.
c. Incubation of the beads with the monoclonal antibody to human β-endorphin diluted to 200 ng/ml in blocking buffer for 40 minutes.
d. Six washes with wash buffer.
e. Incubation of the beads with the polyclonal rabbit antibodies against mouse IgG conjugated with alkaline phosphatase, diluted 1:1000 in blocking buffer for 40 minutes.
f. Six washes with wash buffer and one wash with Tris (25mM), NaC1 (125 mM), Tween (0.1%).
g. Incubation of the beads with BCIP diluted to 500 ug/ml in 0.1M Tris pH 9.0, for 2 hours.
h. Two washes with H₂O, transfer of the beads to petri dishes and observation for blue stained beads (using a stereomicroscope).

### Results:

The staining results are given in Table B-1:

**Table B-1**

| Staining of beads with anti-endorphin McAb clone 3-E7 | |
|---|---|
| **Bead Source** | **Number of Blue-stained Beads** |
| Library 6: Tyr at N-terminal | about 100 beads |
| Library 6: Pro at N-terminal | about 50 beads |
| Library 4 | about 15 beads |
| Model YGGFL | most of the beads |
| Model HPYPP | none of the beads |

Some of the stained beads were submitted for N-terminal sequencing using gas phase peptide microsequencer (model 475A, Applied Biosystems) and the results are summarized in Table B-2:

**Table B-2**

| Sequence Results of Blue-stained Beads | | | | | |
|---|---|---|---|---|---|
| No. | Bead Source | Position 1 | Position 2 | Position 3 | Sequence |
| 1 | Lib. 6:Tyr at n-terminal | Tyr (Y) | Gly (G) | Gly (G) | nYGG |
| 2 | Lib. 6:Tyr at n-terminal | Tyr (Y) | Met (M) | | nYM |
| 3 | Lib. 6:Pro at n-terminal | | | | no sequence yield |
| 4 | Lib. 4 | Lys (K) + Tyr (Y) | Phe (F) + Thr (T) | Thr (T) + Leu (L) + Gly (G) | n-K/Y-F/T-T/L/G |
| 5 | Lib. 4 | Gln (Q) | Gly (G) | Tyr (Y) | nQGY |
| 6 | Lib. 4 | Tyr (Y) | Gly (G) | | nYG |

### Discussion

As can be seen the sequence in nYGG, that is part of the peptide sequence to which the antibody was raised, was identified by library 6 (70,000 hexapeptides per bead). These results prove our basic hypothesis about the ability to detect specific sequences synthesized on beads carrying many hexapeptides per bead.

The other sequences obtained nYM and nQGY were not reported in the literature but could be correct due to the fact that those sequences may contain some non-conventional amino acids (that were not used in previous reports) in positions closer to the C-terminal and D isomers of the YGGF amino acids at any position, that can influence the binding to the antibody epitope. Furthermore, the amino acids Gln (Q) and Met (M) recognized by as were also common in some of the literature reports but in other positions of the tetrapeptide sequence. The sequence nQGY seem to contain the reverse of nYG and might be recognized by the antibody.

### References:

1. Gramsch, C. et al. (1983) J. Neurochem. 40, 1220-1226
2. Kassarjian, A., Schellenberger, V., and Turck, C.W. Screening of Synthetic Peptide Libraries with Radiolabeled Acceptor Molecules. Peptide Res. 6:129-133, 1993.
3. Lam, K.S., Salmon, S.E., Hersh, E.M., Hruby, V.J., Kazmierski, W.M., and Knapp, R.J. A New Type of Synthetic Peptide Library for Identifying Ligand-binding Activity, Nature 354:82-84, 1991.
4. Lam, I.S., Hruby, V.J., Lebl, M., Knapp, R.J., Kazierski, W.W., Hersh, E.M., and Salmon, S.E. The Chemical Synthesis of Large Random Peptide Libraries and Their Use for the Discovery of Ligands for Macromolecular Acceptors, Bioorganic and Medicinal Chemistry Lett 3:419-424, 1993.
5. Lam, K.S., Lebl, M., Krchnak, V., Wade, S., Abdul-Latif, F., Ferguson, R., Cuzzocrea, C., and Wertman, K. Discovery of D-Amino-Acid-Containing Ligands with Selectide Technology, Gene 137:13-16, 1993.
6. Nikolaiev, V., Stierandov, A., Krchnak, V., Seligmann, B., Lam, K.S., Salmon, S.E., and Lebl, M. Peptide-Encoding for Structure Determination of Nonsequenceable Polymers Within Libraries Synthesized and Tested on Solid-Phase Supports, Peptide Res. 6:161-170, 1993.
7. Pinilla, C., Appel, J.R., and Houghten, R.A. Synthetic Peptide Combinatorial Libraries (SPCLs): Identification of the Antigenic Determinant of β-Endorphin Recognized by Monoclonal Antibody 3E7, Gene 128:71-76, 1993

### Working Example C

This section describes the synthesis and screening of a library prepared from glass beads.

The glass beads were obtained from Potters-Balotini in France. Type 5000 beads were used. The claimed diameter of the beads is 0-30 microns. The beads were separated by 1G precipitation and a subtraction was obtained. The average diameter of the separated beads is about 7 microns.

The capacity of the beads is ∼ 1µmole/ml, which corresponds to about 300,000 peptides per bead. Thus, assuming there are 1000 different peptides per bead, each of them will be represented by 300 peptides.

The beads were washed with about 60% nitric acid for 5 hours. The acid washed beads were aminated with aminopropyltriethoxysilan (2% in ethanol for 1 hour at 95°C). Coupling of amino acids was performed as follows: FMOC protected amino acid 25 mM in DMF.
- PyBOP: 25 mM in DMF.
- HObt: 25 mM in DMF.
- NMM: 42 mM
Coupling was performed for 1 hour with continuous.

The library was built from the following 74 α-amine FMOC protected amino acids:
1-3, 5, 7-12, 14-23, 28, 29, 31-39, 42-48, 51-66, 68, 70-82, 85-89, 120.
The first incorporated amino acid (at the C-terminal) was β-Ala.
For the second amino acid the amino acids were grouped as shown in Table C-1:

**Table C-1**

| **Steps 2, 3, 4, 5 only: Total 18 groups** | | | | | |
|---|---|---|---|---|---|
| **1** | **4** | **7** | **10** | **13** | **16** |
| 10AlaC-D | 31Glu-D(Obut) | 58NleM-L | 46LeuM-D | 56Nle-D | 79Tyr-D(But) |
| 12AlaC-L | 32Glu-L(Obut) | 87ValM-D | 47LeuM-L | 57Nle-L | 80Tyr-L(But) |
| 7Ala-D | 20Asp-D(OBut) | 88ValM-L | 14AlaM-D | 59Nva-D | 77Trp-D |
| 8Ala-L | 21Asp-L(OBut) | 36GlyM | 15AlaM-L | 60Nva-L | 78Trp-L |
| 5Aib-L | | | | | |

| **2** | **5** | **8** | **11** | **15** | **17** |
|---|---|---|---|---|---|
| 16Arg-D(Mtr) | 18Asn-D | 70PhepNt-L | 48Lys-D(Boc) | 61Orn-D(BOC) | 73Ser-D(But) |
| 17Arg-L(Mtr) | 19Asn-L | 120PhepNt-D | 51Lys-L(Boc) | 62Orn-L(BOC) | 74Ser-L(But) |
| 44Leu-D | 34GlyC-L | 37GlyP-L | 52Met-D | 38His-D(Trt) | 75Thr-D(But) |
| 45Leu-L | 35GlyC-D | 89Hyp-L(Tbu) | 53Met-L | 39His-L(Trt) | 76Thr-L(But) |
| 9AlaB | | | | | |

| **3** | **6** | **9** | **12** | **15** | **18** |
|---|---|---|---|---|---|
| 11AcaE | 29Gln-L | 42Ile-L | 66PheM-D | 63Phe-D | 71Pro-D |
| 1Aba-L | 28Gln-D | 85Val-D | 68PheM-L | 64Phe-L | 72Pro-L |
| 2Abu-L | 22Ava5 | 86Val-L | 43IleM-L | 54MetS-L | 81Tyr2,5II-L |
| 3AbuG-L | 65Phe-4Cl-L | 33Gly | | 55MetSO-L | 82Tyr3,5Br-L |
| 23Cit-L(Bos) | | | | | |

An individual bead in the library would display mixture of peptides which, at the second amino acid position, would feature only amino acids from a single group. Different beads could display amino acids from different groups. The same groups were used for synthesis steps 2, 3, 4, 5.

For step 6 (position 2 from the N-terminal) the amino acids were initially grouped as in Table C-2:

**Table C-2**

| **1** | **7** | **13** | **19** | **25** | **31** | **37** |
|---|---|---|---|---|---|---|
| 10AlaC-D | 31Glu-D(Obut) | 58NleM-L | 46LeuM-D | 56Nle-D | 79Tyr-D(But) | 5Aib-L |
| 12AlaC-L | 32Glu-L(Obut) | 87ValM-D | 47LeuM-L | 57Nle-L | 80Tyr-L(But) | 9AlaB |

| **2** | **8** | **14** | **20** | **26** | **32** | |
|---|---|---|---|---|---|---|
| 7Ala-D | 20Asp-D(OBut) | 88ValM-L | 14AlaM-D | 59Nva-D | 77Trp-D | |
| 8Ala-L | 21Asp-L(OBut) | 36GlyM | 15AlaM-L | 60Nva-L | 78Trp-L | |

| **3** | **9** | **15** | **21** | **27** | **33** | |
|---|---|---|---|---|---|---|
| 16Arg-D(Mtr) | 18Asn-D | 70PhepNt-L | 48Lys-D(Boc) | 61Orn-D(BOC) | 73Ser-D(But) | |
| 17Arg-L(Mtr) | 19Asn-L | 120PhepNt-D | 51Lys-L(Boc) | 62Orn-L(BOC) | 74Ser-L(But) | |

| **4** | **10** | **16** | **22** | **28** | **34** | |
|---|---|---|---|---|---|---|
| 44Leu-D | 34GlyC-L | 37GlyP-L | 52Met-D | 38His-D(Trt) | 75Thr-D(But) | |
| 45Leu-L | 35GlyC-D | 89Hyp-L(Tbu) | 53Met-L | 39His-L(Trt) | 76Thr-L(But) | |

| **5** | **11** | **17** | **23** | **29** | **35** | |
|---|---|---|---|---|---|---|
| 11AcaE | 29Gln-L | 42Ile-L | 66PheM-D | 63Phe-D | 71Pro-D | |
| 1Aba-L | 28Gln-D | 85Val-D | 68PheM-L | 64Phe-L | 72Pro-L | |

| **6** | **12** | **18** | **24** | **30** | **36** | |
|---|---|---|---|---|---|---|
| 2Abu-L | 22Ava5 | 86Val-L | 43IleM-L | 54MetS-L | 81Tyr2,5II-L | |
| 3AbuG-L | 65Phe-4Cl-L | 33Gly | 23Cit-L(Bos) | 55MetSO-L | 82Tyr3,5Br-L | |

For reasons of convenience, the initial 37 step 6 groups were merged into 12 larger groups, labeled A-L in Table C-3. Thus step 6 groups 1-3 went into tube A, groups 4-6 into tube B, etc.

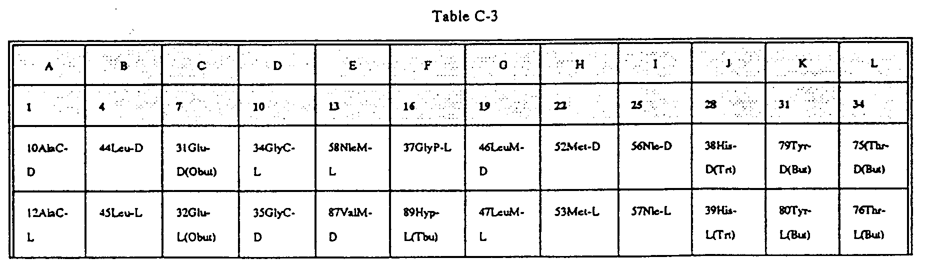

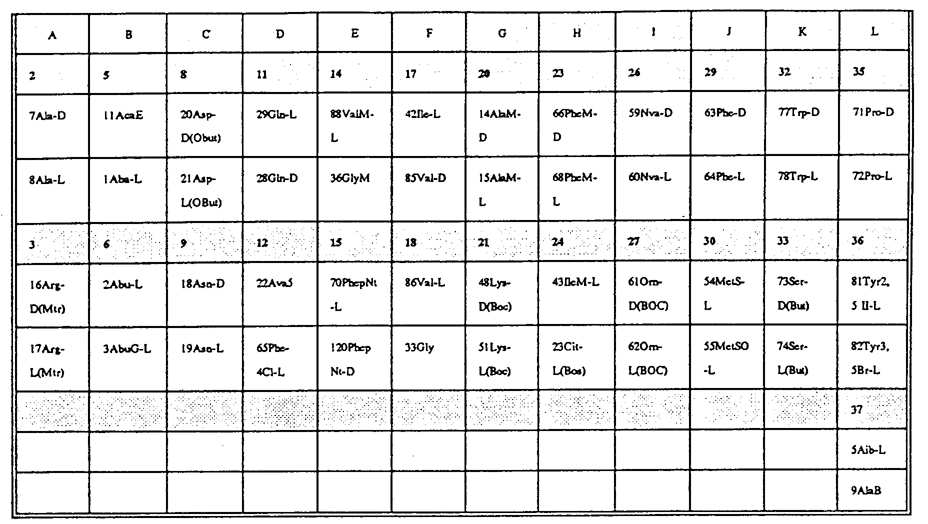

In Table C-4, the amino acids listed in the column headings in C-4 are those inserted at the second position from the N-terminal. Either of two different amino acids are found at this position in the peptides on a given bead. Since Tube A comprises beads from three different step 6 groups, there are six possibilities for this position for the beads of a given tube A-L.

The contents of tube A were then distributed into 37 micronic tubes to be placed in column "A" of a microtiter plate. The contents of each of these 37 tubes was reacted with the corresponding two amino acid mixture set forth on the left side of Table C-4, thus supplying the amino terminal amino acid. The contents of tube B went into the 37 tubes of the next column, which were similarly reacted, and so on for a total of 12x37=444 tubes, as shown in Table C-4.

The library was screened for binding IL6 that was labeled with fluorescent label Cy3. No staining was observed in any of the wells.

The library was stained with TBP1 that was labeled with tetramethyl rhodamine. Staining was observed in the wells marked with "1" in Table C-3 below. Positive staining means that the well contained at least 4 stained beads.

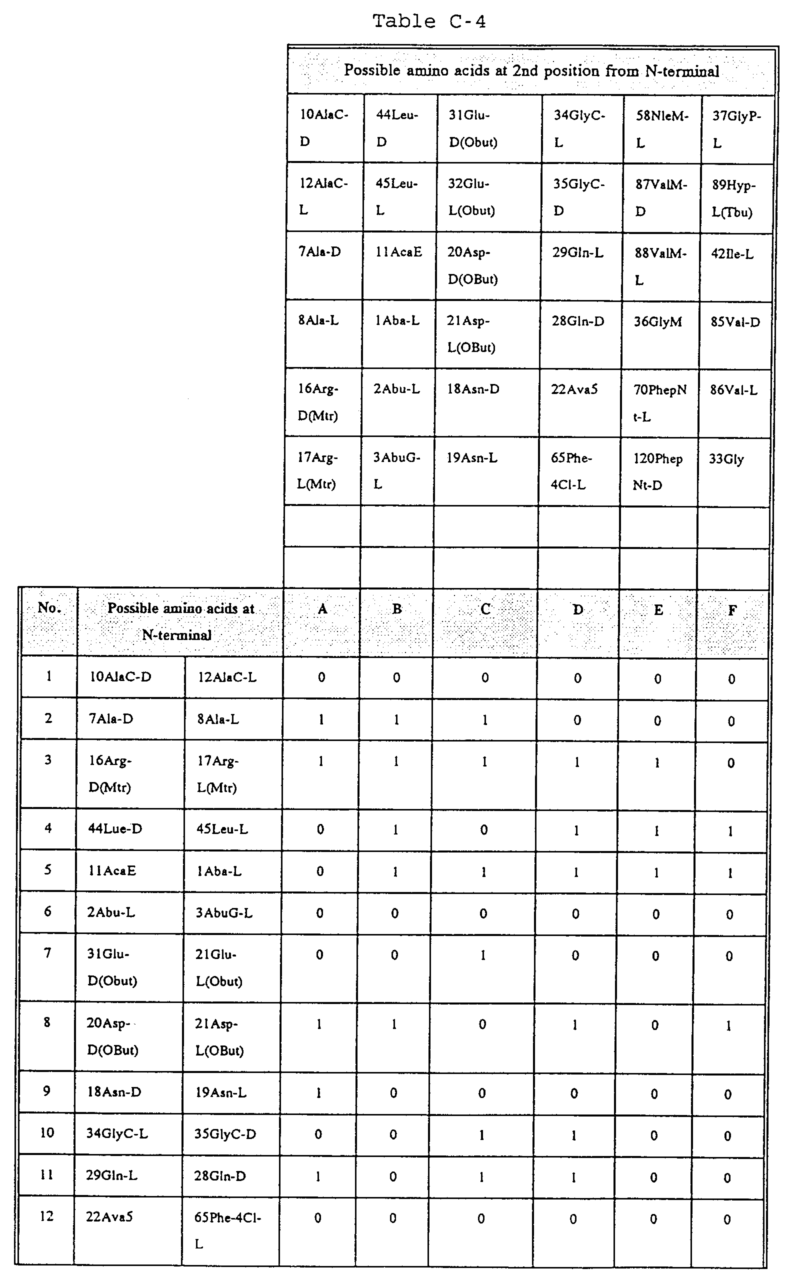

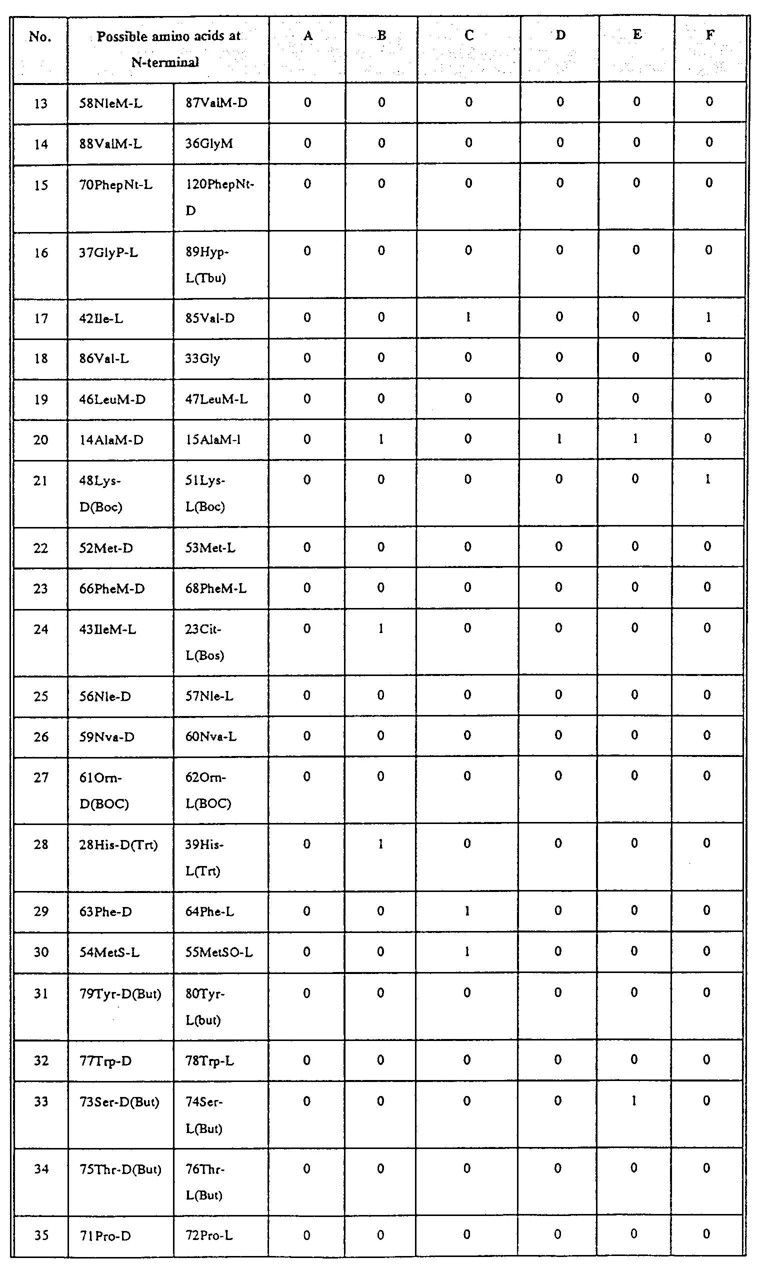

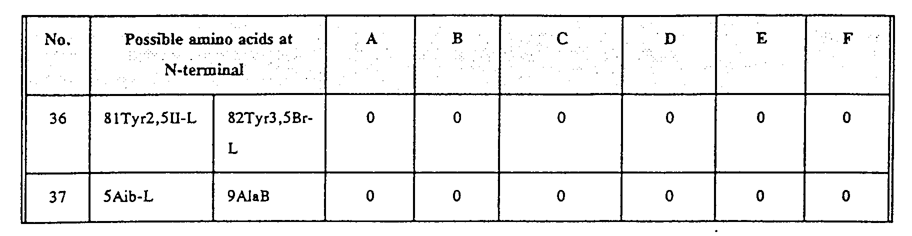

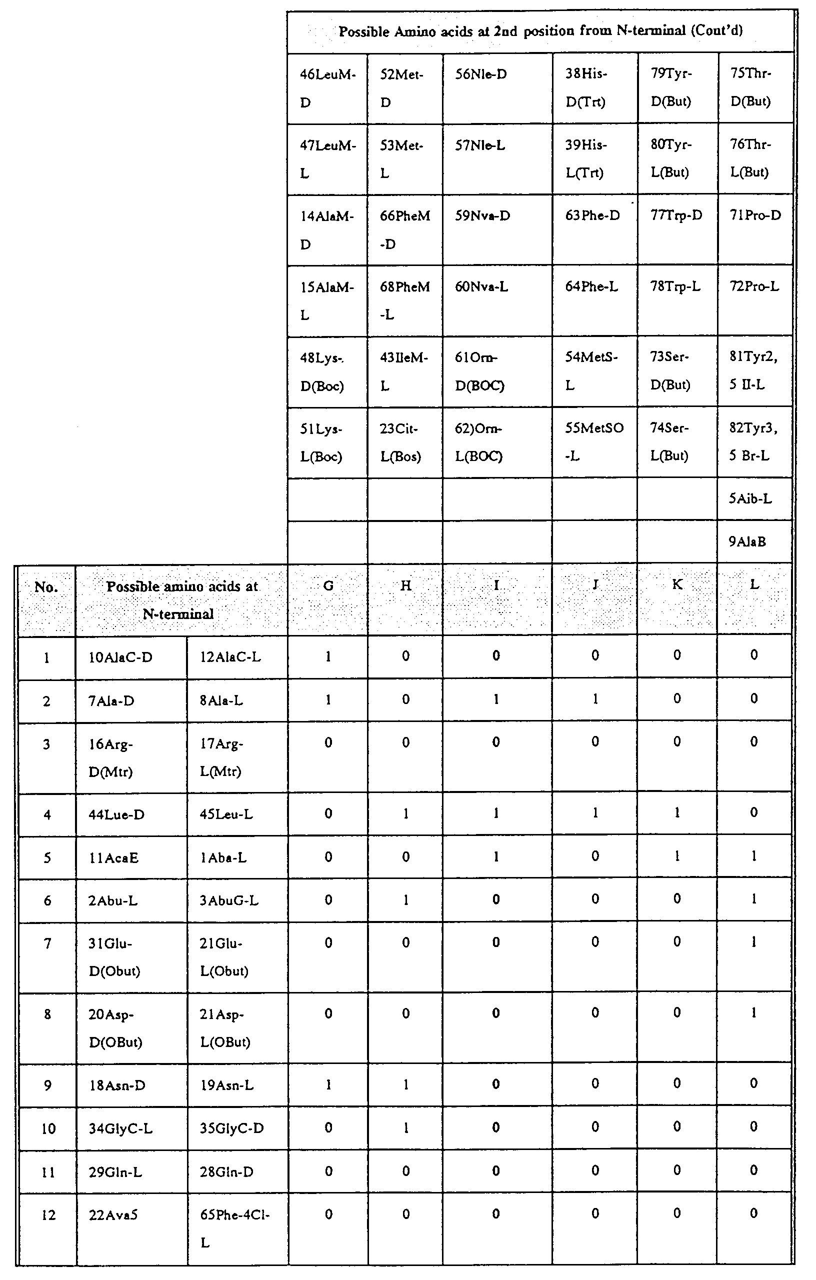

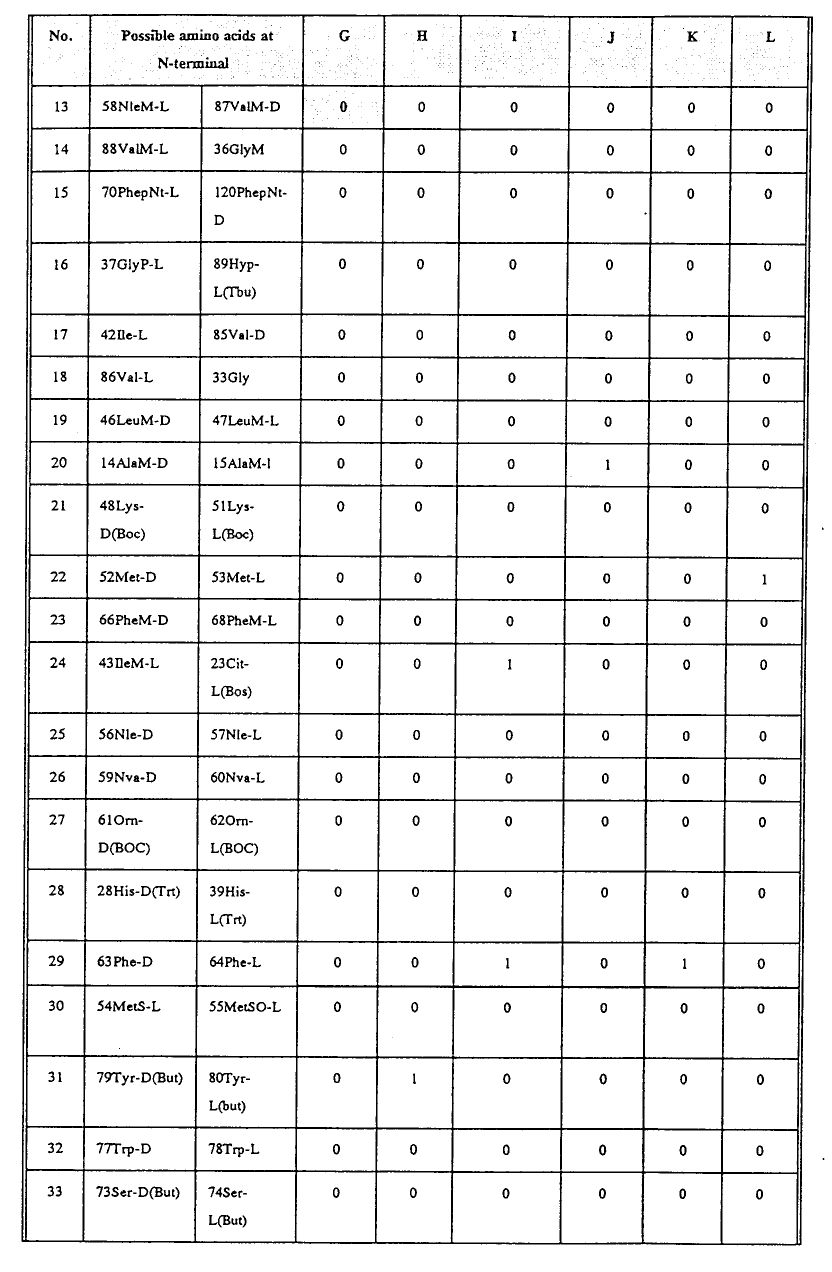

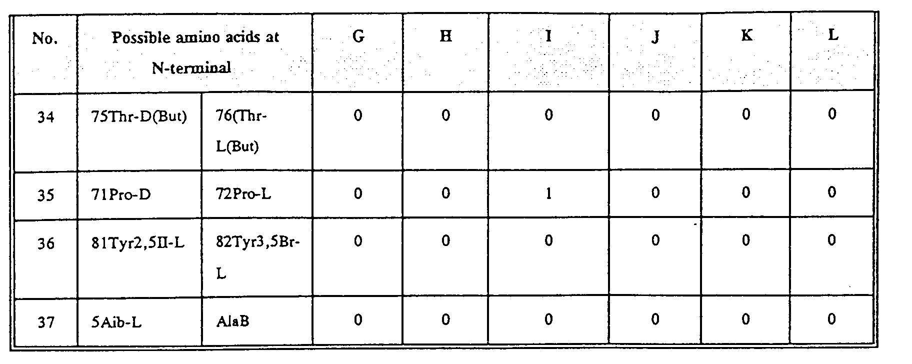

### Staining procedure

The beads are adsorbed in 6 well plates. To the wells is added PBS containing 0.1% Tween 20 and 0.1% sodium azide (wash buffer). The wash buffer was removed by suction. To the wells was added 200µl of blocking buffer (PBS + 1mg/ml of I-block + Ca⁺⁺ 0.9 mM + Mg⁺⁺ 0.5 mM) containing 5 µg/ml of TBP1 (over 99% pure) labeled with tetramethyl rhodamine. The solution was incubated in the well for 30 minutes with gentle agitation. The wells were filled up (about 5 ml) with wash buffer. The wash buffer was removed by suction.

### Analysis procedure

The wells were observed by fluorescence inverted microscope under 100x magnification. Some fluorescent debris were observed. Thus, suspected positive beads were verified by observation at 400x magnification.

### Reference Example: Peptide and Peptoid Synthesis

### Supports:

The most common support material for solid phase peptide synthesis consists of beads made of polystyrene cross linked with 1-4% of divinylbenzene (DVB). Other supports which have been used include, among other, modified paper (cellulose) either as sheet or as Perloza beaded cellulose, polyamide based resins some of which are based on polyacrylamide, grafted polyethylene; Polyethylene glycol modified polystyrene/1% DVB which is commercially available as Tentagel from several companies; Modified glass either as sheets (as used by Affimax), or as beads. Virtually, any material stable to the solvents and chemicals used in peptides synthesis might be used as a support. The preferred support is Tentagel.

Some reference for novel supports are cited below:
1. Mendre, C., Sarrade, V. and Calas, B. Continuous flow synthesis of peptides using a polyacrylamide gel resin (Expansin™). *International Journal of Peptide and Protein Research* 39:278-284, 1992.
2. Kanda, P., Kennedy, R.C. and Sparrow, J.T. Synthesis of polyamide supports for use in peptide synthesis and as peptide-resin conjugates for antibody production. *Int. J. Pept. Protein Res*. 38:385-391, 1991.
3. Kiederowski, G. Light-directed parallel synthesis of up to 250,000 different oligopeptides and oligonucleotides. *Angew*. *Chem. Int. Ed. Engl*. 30:822, 1991. Note: Synthesis on modified glass. This is the technology used by Affimax.
4. Valerio, R. M., Benstead, M., Bray, A. M., Campbell, R. A. and Maeji, N.J. Synthesis of peptide analogues using the multipin synthesis method. *Anal.Biochem* 197:168-177, 1991. Note: Synthesis on grafted polyethylene rods.
5. Calas, B., Mery, J., Parello, J. and Cave, A. Solid-phase synthesis using a new polyacrylic resin. *Tetrahedron* 41:5331-5339, 1985.
6. Englebresten, D.R. and Harding, D.R.K. Solid phase peptide synthesis on hydrophilic supports. Part II-- Studies using Perloza beaded cellulose. *Int. J. Pept. Protein Res*. 40:487-496, 1992.

### Protecting groups:

The two most common protecting groups for the α-amino group are tert-butyloxycarbonyl (Boc) or 9-Flourenylmethoxycarbonyl (Fmoc). The Boc protecting group is removed by acid conditions, e.g. by 100% TFA. The Fmoc protecting groups are removed under basic conditions e.g. by 20% piperidine in DMF. We prefer to use the Fmoc strategy.

Side chains of some of the amino acids also need to be protected to avoid damage during synthesis or to avoid formation of branched peptides by incorporation of amino acids at free amino group appearing on the side chain of a few amino acids (e.g. lysine, ornithine). The common protecting groups are as follows:
For free amino groups: Boc; Fmoc; Benzyloxycarbonyl (CBZ);
For free carboxy: tert. butyl ester; Benzyl ester; Cyclohexyl ester;
For Arginine: Nirto (NO₂); Mesitylenesulfonic acid; Tosyl; Aspargine: Xanthyl; Methoxy-2,3,6-trimethylsulfone (Mtr); For Cystein: Acetamidomethyl; Benzyl thiether; tert. butyl thiether;
Methylbenzyl; Methobenzyl; 3-Nitro-2-pyridinesulfonyl (Npys); For Glutamine: Trityl; Xanthyl (Xan);
Hisidine: Tosyl; Trityl
For free hydroxyls: Benzyl ether; tert butyl ether;
Tryptophan: N-Formyl; N-Bromobenzoxycarbonyl: Nitrophenylsulfonyl (Nps);
Tyrosine: O-Benzyl; 0-2,6-dichlorobenzyl;
We prefer the following protecting groups:
For free amino groups: BOC
For free carboxyl groups: tert. butyl ester.
For Cystein: Note used.
For Glutamine and Aspargine: None.
For Histidine: Trityl.
For free hydroxyl groups: tert. butyl ether.
For Tryptophan: None
For Arginine: Methoxy-2,3,6-trimethylsulfone (Mtr)

### Coupling reagents:

The coupling of amino acids in solid phase peptide synthesis has been under study since the introduction of the technique by Merrifield in 1967. Today, the synthesis of short peptides composed of the 20 common L-amino acids can be performed by any of a large selection of methods. Recent reports in the field describe novel methods intended to:
1. Shorten synthesis time.
2. Reduce racemization during synthesis.
3. Enable synthesis of long peptides.
4. Enable synthesis of "difficult" sequences.
5. Enable incorporation of unnatural amino acids such as N-substituted amino acids, or α-carbon di-substituted amino acids where the free H is replaced with another group.
6. Improve the automation of peptide synthesis.
7. Improve large scale peptide synthesis.
8. Produce peptides with different types of pseudopeptide bonds such as:
   Carba Ψ(CH2);
   Depsi Ψ(CO-O);
   Hydroxyethylene Ψ(CHOH-CH2);
   Ketomethylene Ψ(CO-CH2);
   Methylene-oxy CH2-O-;
   Reduced CH2-NH; Retro inverso NH CO;
   Thiomethylene CH₂-S-;
   Thiopeptide CS-NH.
9. Improve production of peptides having constrained conformations such as cyclic peptides or multiple antigen peptides (MAPs).
Several recent examples for studies of peptide synthesis are cited below:
1. Schnolzer, M., Alewood, P., Jones, A., Alewood, D. and Kent, S.B.H. *In situ* neutralization in Boc-chemistry solid phase peptide synthesis. Rapid, high yield assembly of difficult sequences. *Int. J. Pept. Protein Res*. 40:180-193, 1992.
2. Chen, S. and Xu, J. A new coupling reagent for peptide synthesis. Benzotriazolyloxy-bis (pyrrolidino)-carbonium hexafluorophosphate (BBC). *Tetrahedron Letters* 33:647-650, 1992.
3. Spencer, J.R., Antonenki, V.V., Delaet, N.G.J. and Goodman, M. Comparative study of methods to couple hindered peptides. *Int. J. Pept. Protein Res*. 40:282-293, 1992
4. 1. Kiso, Y., Fujiwara, Y., Kimura, T., Nishitani, A. and Akaji, K. Efficient solid phase peptide synthesis. Use of methanesulfonic acid α-amino deprotecting procedure and new coupling reagent, 2-(benzotriazol-1-ul)oxy-1,3-dimethylimidazolidinium hexafluorophosphate (BOI). *Int. J. Pept*. *Protein Res*. 40:308-314, 1992.

### Materials:

Solvent: We prefer to use dimethylformamide (DMF) throughout the synthesis. We sometimes add some dichloromethane (DCM) in order to enable easier collection of the beads: The density of the DCM is very high and beads float in mixtures of DMF and DCM.

It should be understood that the present invention is not limited to the use of a particular support, protective group, or solvent.

### Preferred Synthetic Cycle

One cycle of the synthesis consists of the following operations:

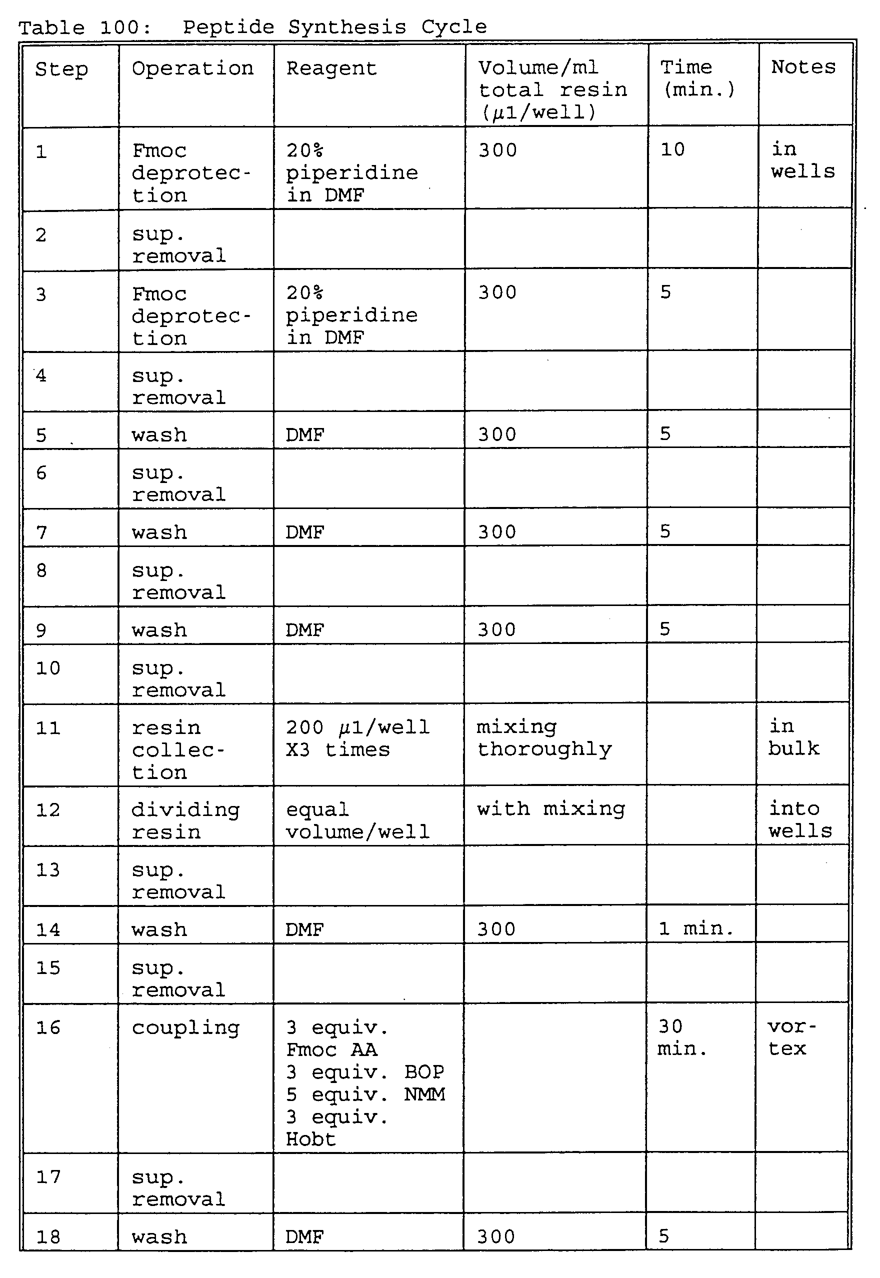

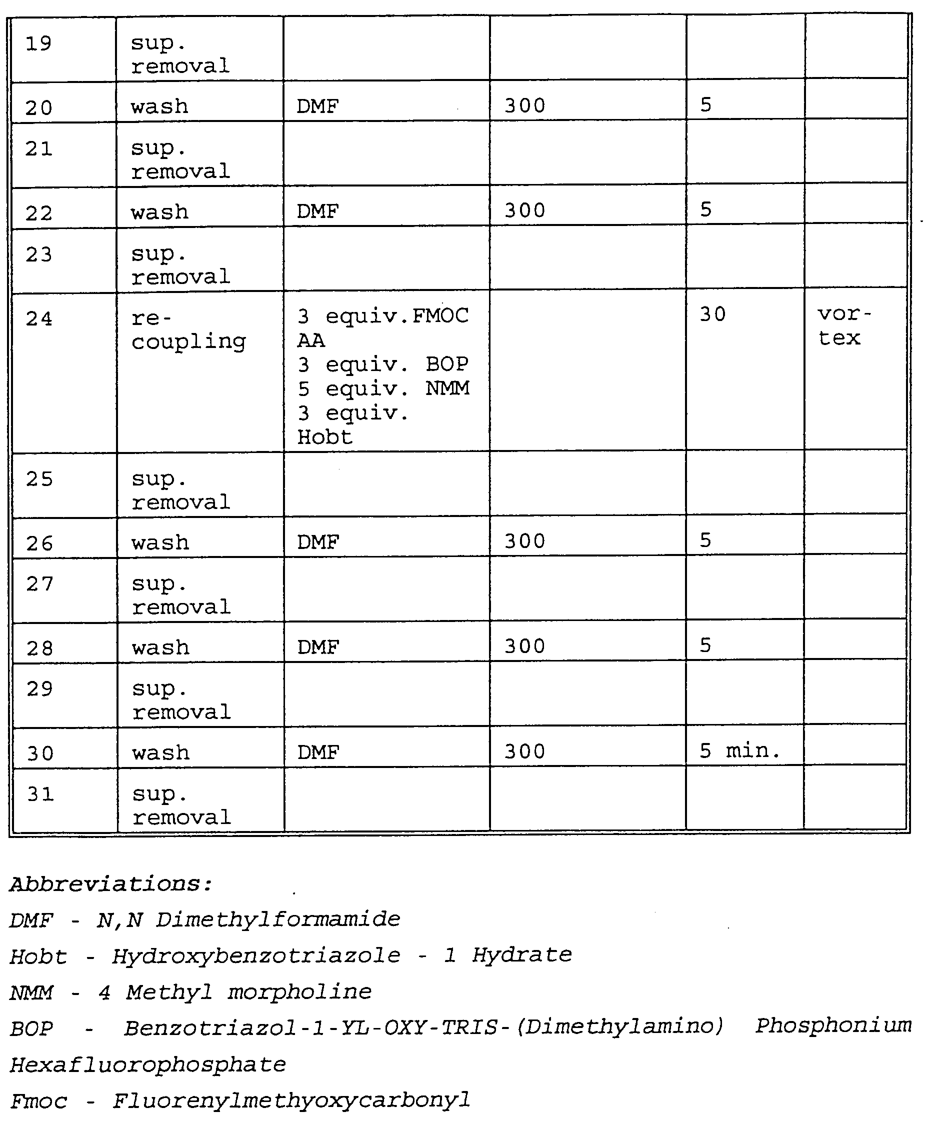

**Global deprotection**: of side chain protecting groups is performed at the end of the synthesis by twice 40 minutes incubations in: 25% TFA in DCM + 5% anisol and 5% thioanisol.

### Peptides including Unusual Amino Acids

The current trend in peptide synthesis, especially in the approach of irrational drug design calls for the incorporation of many different types of building blocks. Many of the new building blocks used are amino acids which are not genetically encoded, e.g., glycosylated amino acids, or various unnatural amino acids. The references cited below indicate some of these recent efforts:
1. Bielfeldt, T., Peters, S., Meldal, M., Bock, K. and Paulsen, N.A. new strategy for solid-phase synthesis of *O*-glycopeptides. *Angew. Chem. (Engl)* 31:857-859, 1992.
2. Gurjar, M.K. and Saha, U.K. Synthesis of the glycopeptide-O-(3,4-di-O-methyl-2-0-[3,4-di-O-methyl-α-L-rhamnopyranosyl]-α-L-rhamnophyranosyl)-L-alanilol: An unusual part structure in the glycopeptidolipid of Mycobacterium fortuitum. *Tetrahedron* 48:4039-4044, 1992.
3. Kessler, H., Wittmann, V., Kock, M. and Kottenhahn, M. Synthesis of *C*-glycopeptides via free radical addition of glycosyl bromides to dehydroalanine derivatives. *Angew*. *Chem*. *(Engl.)* 31:902-904, 1992.
4. Kraus, J.L. and Attardo, G. Synthesis and biological activities of new *N*-formylated methionyl peptides containing an α-substituted glycine residue. *European Journal of Medicinal Chemistry* 27:19-26, 1992.
5. Mhaskar, S.Y. Synthesis of N-lauroyl dipeptides and correlation of their structure with surfactant and antibacterial properties. *J. Am. Oil Chem. Soc*.69:647-652, 1992.
6. Moree, W.J., Van der Marel, G.A. and Liskamp, R.M.J. Synthesis of peptides containing the β-substituted aminoethane sulfinamide or sulfonamide transition-state isostere derived from amino acids. *Tetrahedron Lett*. 33:69-6392, 1992.
7. Paquet, A. Further studies on the use of 2,2,2-trichloroethyl groups for phosphate protection in phosphoserine peptide synthesis. *International Journal of Peptide and Protein Research* 39:82-86, 1992.
8. Sewald, N., Riede, J., Bissinger, P. and Burger, K. A new convenient synthesis of 2-trifluoromethyl substituted aspartic acid and its isopeptides. Part 11. *Journal of the Chemical Society*. *Perkin Transactions 1* 1992:267-274, 1992.
9. Simon, R.J., Kania, R.S., Zuckermann, R.N., Huebner, V.D., Jewell, D.A., Banville, S., Ng, S., Wang, L., Rosenberg, S., Marlowe, C.K., Spellmeyer, D.C., Tan, R., Frankel, A.D., Santi, D.V., Cohen, F.E. and Bartlett, P.A. Peptoids: A modular approach to drug discovery. *Proc. Natl, Acad. Sci. USA* 89:9367-9371, 1992.
10. Tung, C.-H., Zhu, T., Lackland, H. and Stein, S. An acridine amino acid derivative for use in Fmoc peptide synthesis. *Peptide Research* 5:115-118, 1992.
11. Elofsson, M. Building blocks for glycopeptide synthesis: Glycosylation of 3-mercaptopropionic acid and Fmoc amino acids with unprotected carboxyl groups. *Tetrahedron Lett*. 32:7613-7616, 1991.
12. McMurray, J.S. Solid phase synthesis of a cyclic peptide using Fmoc chemistry. *Tetrahedron Letters* 32:7679-7682, 1991.
13. Nunami, K.-I., Yamazaki, T. and Goodman, M. Cyclic retro-inverso dipeptides with two aromatic side chains. I. Synthesis. *Biopolymers* 31:1503-1512, 1991.
14. Rovero, P, Synthesis of cyclic peptides on solid support. *Tetrahedron Letters* 32:2639-2642, 1991.
15. Elofsson, M., Walse, B. and Kihlberg, J. Building blocks for glycopeptide synthesis: Glycosylation of 3-mercaptopropionic acid and Fmoc amino acids with unprotected carboxyl groups. *Tetrahedron Letter*, 32:7613-7616, 1991.
16. Bielfeldt, T., Peter, S., Meldal, M., Bock, K. and Paulsen, H. A new strategy for solid-phase synthesis of O-glycopeptides. *Agnew*. *Chem (Engl)* 31:857-859, 1992.
17. Luning, B., Norberg, T. and Tejbrant, J. Synthesis of glycosylated amino acids for use in solid phase glycopeptide synthesis, par 2:N-(9-fluorenylmethyloxycarbonyl)-3-O-[2,4,6-tri-O-acetyl-α-D-sylopyranosyl)-β-D-glucopyranosyl]-L-serine. J. *Carbohydr*. *Chem*. 11:933-943, 1992.
18. Peters, S., Bielfeldt, T., Meldal, M., Bock, K. and Paulsen, H. Solid phase peptide synthesis of mucin glycopeptides. *Tetrahedron Lett*. 33:6445-6448, 1992.
19. Urge, L., Otvos, L., Jr., Lang, E., Wroblewski, K., Laczko,I. and Hollosi, M. Fmoc-protected, glycosylated asparagines potentially useful as reagents in the solid-phase synthesis of N-glycopeptides, *Carbohydr*. *Res*. 235:83-93, 1992.
20. Gerz, M., Matter, H. and Kessler, H., S-glycosylated cyclic peptides, *Angew. Chem*. *(Engl.)* 32:269-271, 1993.

### Branched Peptides

One of the advantages of the chemical approach to peptide libraries (as opposed to libraries expressed by biological means, e.g. filamentous phages) is the ability to produce and test branching peptides. Early examples in the literature for such structures are found in the use of multiple antigenic peptides (MAP) as immunogens, in MAPs, peptide haptens are attached to a branching "tree" of lysines.
1. Baleux, F. and Dubois, P. Novel version of Multiple Antigenic Peptide allowing incorporation on a Cysteine functionalized lysine tree. *Int. J. Pept. Protein Res*. 40:7-12, 1992.
2. Munesinghe, D.Y., Clavijo, P., Calle, M.C., Nussenzweig, R.S. and Nardin, E. Immunogenicity of multiple antigen peptides (MAP) containing T and B cell epitopes of the repeat region of the P. *falciparum* circumsporozoite protein. *Eur. J. Immunol.* 21:3015-3020, 1991.

In the current MAP system identical peptide sequences are repeated several time in the MAP structure. Such an arrangement apparently stabilizes the peptide conformation, allowing for better presentation of the antigenic structure and hence better immunogenicity. The use of approaches similar to the MAP could enable better biological activity of peptides due to stabilization of conformation.

The interaction of short linear peptides with their targets occurs along the peptide length. Formation of branching peptides may enable interaction of the peptide with the target throughout a surface and thus mimic the type of interaction of some antibodies with their target antigens (as observed by X-ray crystalography and analysis of antibody-antigen complexes). This type of interaction opens up new possibilities for small peptide-ligand interactions which are non-existent for linear peptides but existent for protein-ligand interactions.

### Cyclic Peptides

Many naturally occurring peptide are cyclic. Cyclization is a common mechanism for stabilization of peptide conformation thereby achieving improved association of the peptide with its ligand and hence improved biological activity. Cyclization is usually achieved by intra-chain Cystine formation, by formation of peptide bond between side chains or between N- and C-terminals. Cyclization was usually achieved by peptides in solution, but several publications have appeared recently that describe cyclization of peptides on beads (see references below). These published techniques may be directly applicable to our library approach.
1. Spatola, A.F., Anwer, M.K. and Rao, M.N. Phase transfer catalysis in solid phase peptide synthesis. Preparation of cycle [Xxx-Pro-Gly-Yyy-Pro-Gly] model peptides and their conformational analysis. *Int. J. Pept. Protein Res*. 40:322-332, 1992
2. Tromelin, A., Fulachier, M.-H., Mourier, G. and Menez, A. Solid phase synthesis of a cyclic peptide derived from a curaremimetic toxin. *Tetrahedron Lett*. 33:5197-5200, 1992.
3. Trzeciak, A. Synthesis of 'head-to-tail' cyclized peptides on solid supports by Fmoc chemistry. *Tetrahedron Lett*. 33:4557-45560, 1992.
4. Wood, S. J. and Wetzel, R. Novel cyclization chemistry especially suited for biologically derived, unprotected peptides, *Int. J. Pept. Protein Res*. 39:533-539, 1992.
5. Gilon, C., Halle, D., Chorev, M., Selinger, Z. and Byk, G. Backbone cyclization: A new method for conferring conformational constraint on peptides. *Biopolymers* 31:745-750, 1991.
6. McMurray, J. S. Solid phase synthesis of a cyclic peptide using Fmoc chemistry. *Tetrahedron Letters* 32:7679-7682, 1991.
7. Rovero, P. Synthesis of cyclic peptides on solid support. *Tetrahedron Letters* 32:2639-2642, 1991.
8. Yajima, X. Cyclization on the bead via following Cys Acm deprotection. *Tetrahedron* 44:805, 1988.

### Peptoid Synthesis

Most, if not all of the materials containing at least 1 free amino and 1 free carboxyl group might be used for synthesis of polypeptide polymers. Most if not all the materials having only a single type of groups (i.e. free amino or free carboxyl), can be incorporated at the C-or N- terminals respectively, or at appropriate side chains. Most if not all materials having a groups reactive with free carboxyl or free amino, free bydroxyl or free thio groups could be used for modification of terminal or the side chains of appropriate amino acids. So far only a small variety of such compounds have actually been used for synthesis. Some of the reported structures are described below:
1. Modification of the R group in single Cα substituted amino acids. Modified R groups that have been reported are: Glycosylated, phosphorylated, sulfated, metal chelators, nucleotide residues, and many others.
2. Modification of the peptide bonds into pseudopeptide bonds: The pseudopeptide bonds are usually incorporated into di-pseudopeptides which are then incorporated into peptides. It is not possible to sequence such pseudopeptides and thus sequence determination would have to rely on encoding. Following is a list of most of the pseudopeptide bonds which were described in the literature.
   Carba Ψ(CH₂-CH₂)
   Depsi Ψ(CO-O)
   Hydroxyethylene Ψ(CHOH-CH₂)
   Ketomethylene Ψ(CO-CH₂)
   Methylene-ocy CH₂-O-
   Reduced CH₂-NH
   Retro inverso NH CO
   Thiomethylene CH₂-S-
   Thiopeptide CS-NH
3. Backbone modifications: Use of non-α amino acids e.g., β-Alanine.
4. α-amino acids with 2 R groups of the Cα. The R groups may be similar or different.
5. Dehydroamino acids (see below). 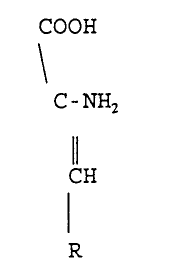
6. N-modified amino acid of the general structure:

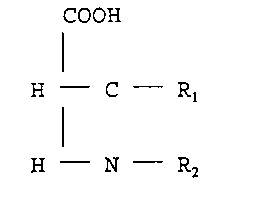

### References:

1. Corringer, P.J., Weng, J.H., Ducos, B., Durieux, C., Boudeau, P., Bohme, A. and Roques, B.P. CCK-B agonist or antagonist activities of structurally hindered and peptidase-resistant Boc-CCK₄ derivatives. *J. Med. Chem*. 36:166-172, 1993. Amino acids reported: aromatic naphthylalaninimide (Nal-NH2); N-methyl amino acids.
2. Beylin, V.G., Chen, H.G., Dunbar, J., Goel, O.P., Harter, W., Marlatt, M. and Topliss, J.G. Cyclic derivatives of 3,3-diphenylalanine (Dip) (II), novel α-amino acids for peptides of biological interest. *Tetrahedron Lett*. 34:953-956, 1993.
3. Garbay-Jaureguiberry, C., Ficheux, D. and Roques, B.P. Solid phase synthesis of peptides containing the non-gydrolysable analog of (O)phosphotyrosine, *p*(CH₂PO₃H₂)Phe. Application to the synthesis of 344-357 sequences of the β₂ adrenergic receptor. *Int. J. Pept. Protein Res*. 39:523-527, 1992.
4. Lüning, B., Norberg, T. and Tejbrant, J. Synthesis of glycosylated amino acids for use in solid phase glycopeptide synthesis, part 2: *N*-(9-fluorenylmethyloxycarbonyl)-3-*O*-[2,4,6-tri-*O*-acetyl-3-*O*-(2,3,4-tri-*O*-acetyl-α-D-xylopyranosyl)-β-D-glucopyranosyl]-L]serine. *J. Carbohydr. Chem*. 11:933-943, 1992.
5. Tung, C.H., Zhu, T., Lackland, H. and Stein, S. An acridine amino acid derivative for use in Fmoc peptide synthesis. *Peptide Research* 5:115-118, 1992.
6. Eric Frerot, PyBOP and PyBroP: Two reagents for the difficult coupling of the alpha,alpha-dialkyl amino acid Aib. *Tetrahedron* 47:259-270, 1991.
7. Moree, W.J., Van der Marel, G.A. and Liskamp, R.M.J. Synthesis of peptides containing the β-substituted aminoethane sulfinamide or sulfonamide transition-state isostere derived from amino acids. *Tetrahedron Lett*. 33:6389-6392, 1992.
8. Rana, T.M., Synthesis of a metal-binding amino acid suitable for solid phase assembly of peptides. *Tetrahedron Lett*. 33:4521-4524, 1992.
9. Urge, L., Otvos, L., Jr., Lang, E., Wroblewski, K., Laczko, I. and Hollosi, M. Fmoc-protected, glycosylated asparagines potentially useful as reagents in the solid-phase synthesis of *N*-glycopeptides. *Carbohydr. Res*. 235:83-93, 1992.
10. Pavone, V., DiBlasio, B., Lombardi, A., Maglio, O., Isernia, D., Pedone, C., Benedette, E., Altmann, E. and Mutter, M. Non coded C^{α,α}-disubstituted amino acids. X-ray diffraction analysis of a dipeptide containing (S)-α-methylserine. *Int. J. Pept. Protein Res*. 41:15-20, 1993.
11. Nishino, N., Mihara, H., Kiyota, H., Kobata, K. and Fujimoto, T. Aminoporphyrinic acid as a new template for polypeptide design. *J. Chem. Soc. Chem. Commun*. 1993:162-163, 1993.
12. Sosnovsky, G., Prakash, I. and Rao, N.U.M. In the search for new anticancer drugs. XXIV: Synthesis and anticancer activity of amino acids and dipeptides containing the 2-chloroethyl- and [*N*'-nitroso]-aminocarbonyl groups. *J. Pharm. Sci*. 82:1-10, 1993.
13. Berti, F., Ebert, C. and Gardossi, L. One-step stereospecific synthesis of α,β-dehydroamino acids and dehydropeptides. *Tetrahedron Lett*. 33:8145-8148, 1992.

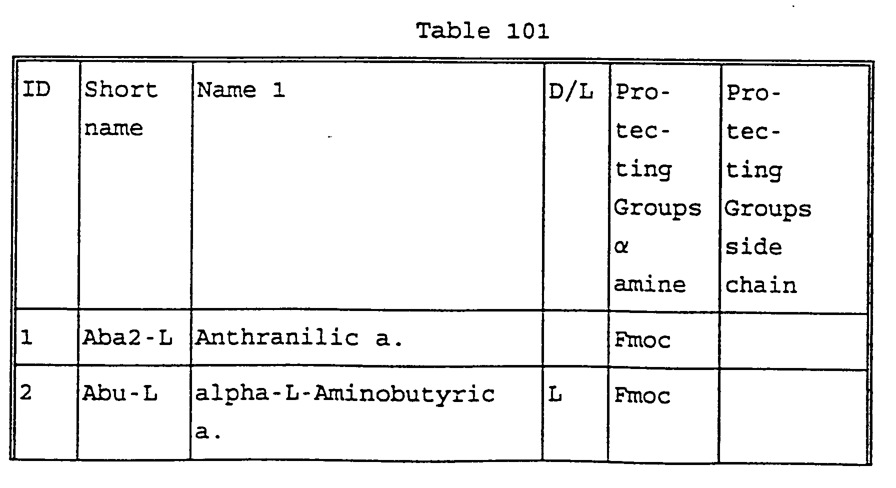

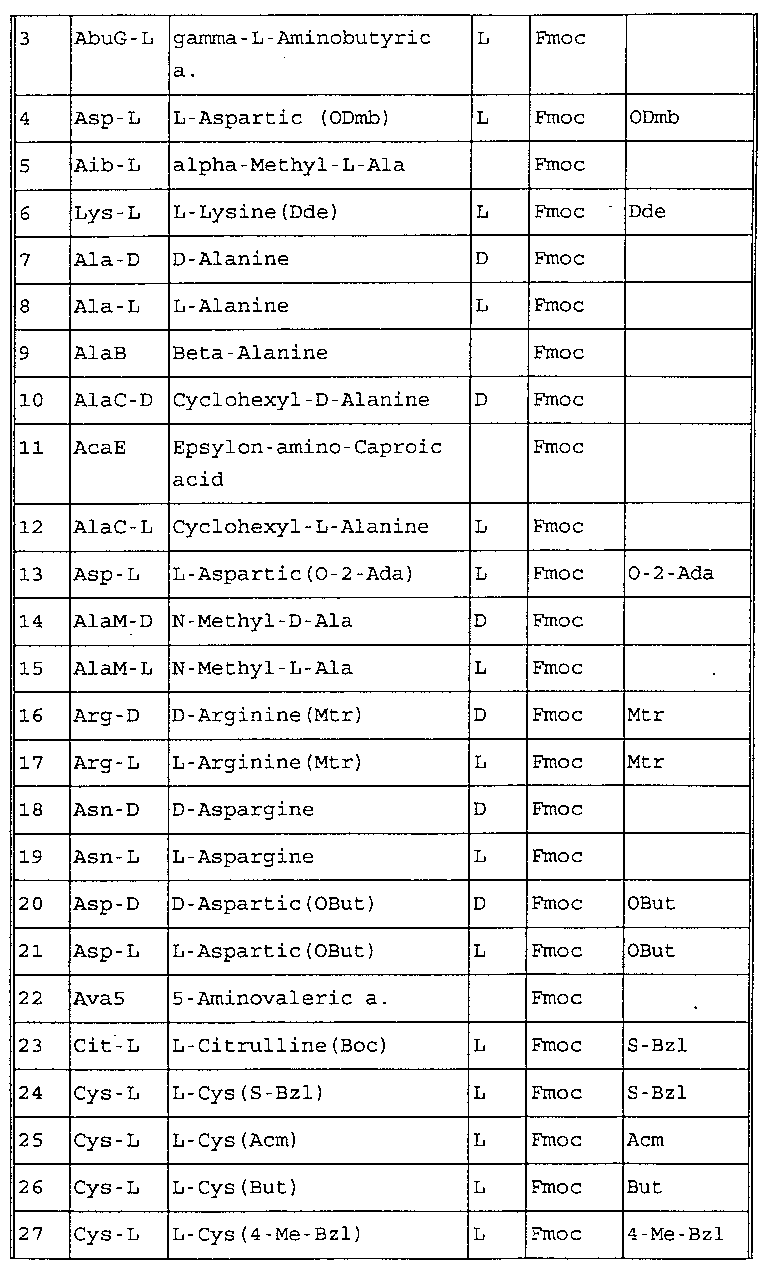

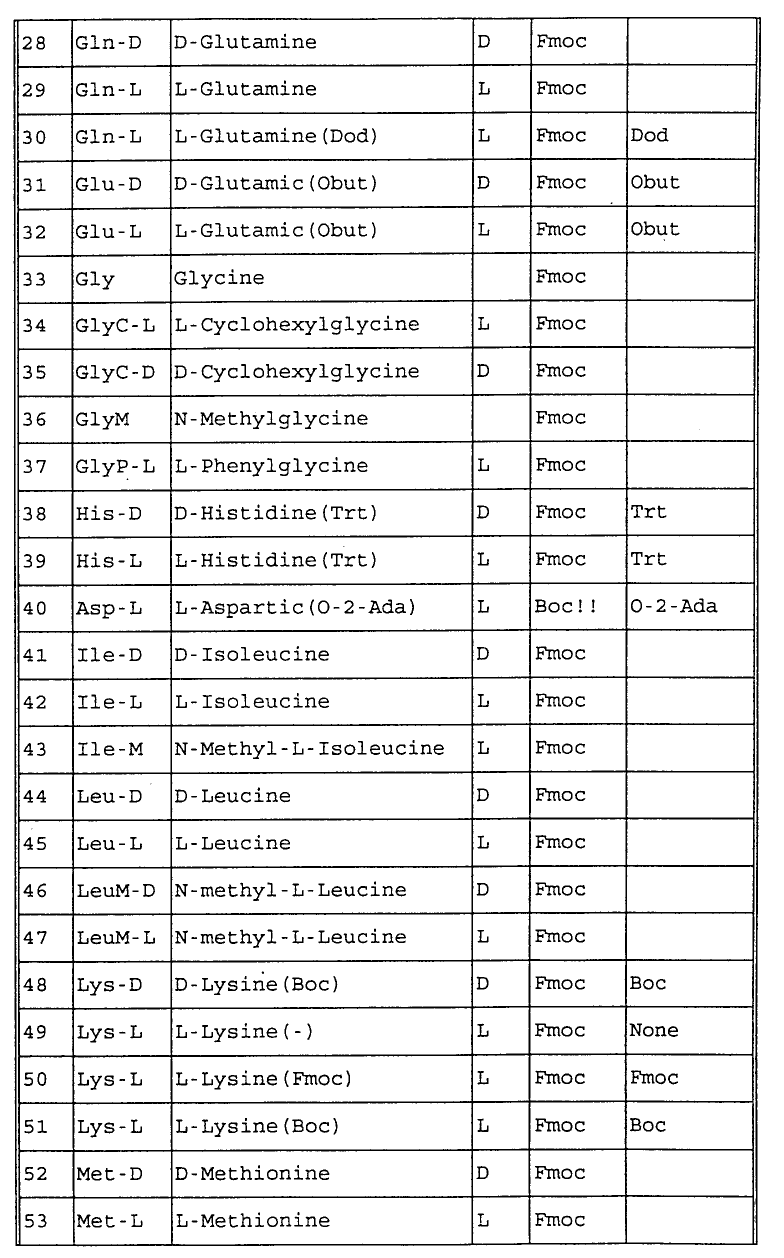

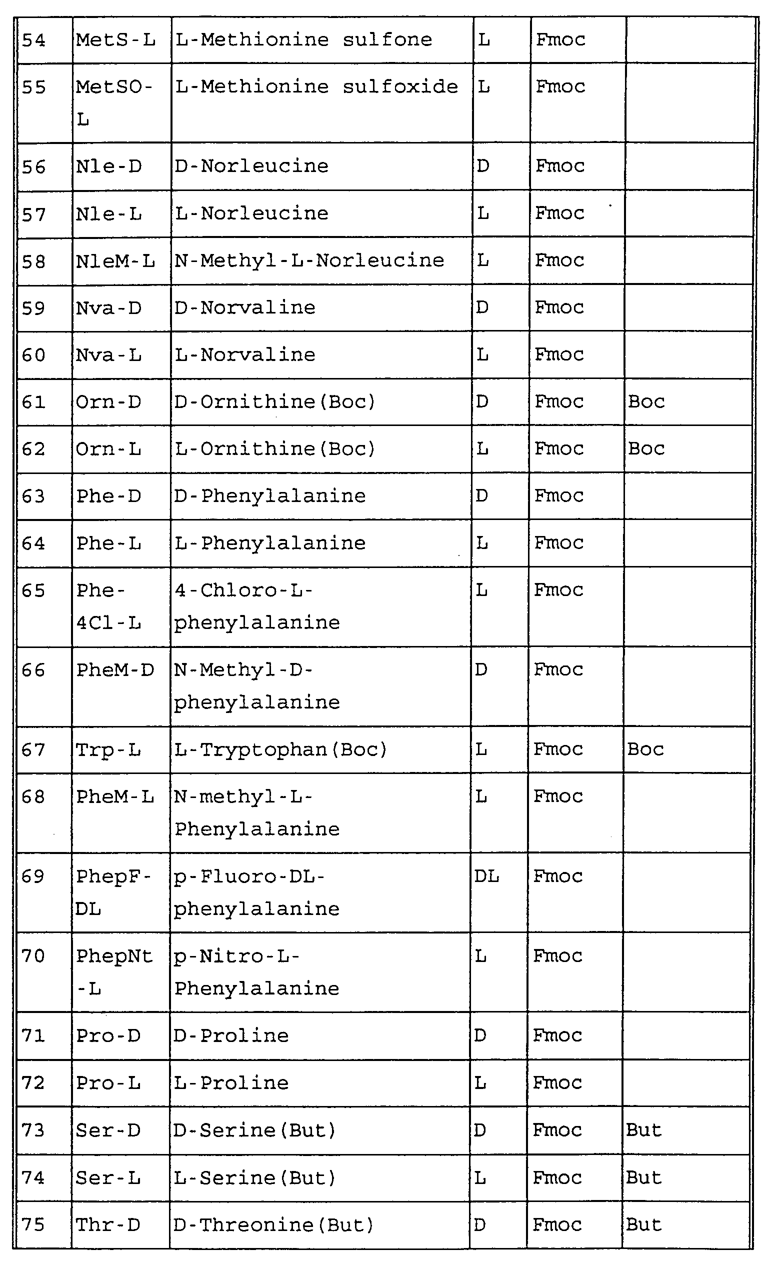

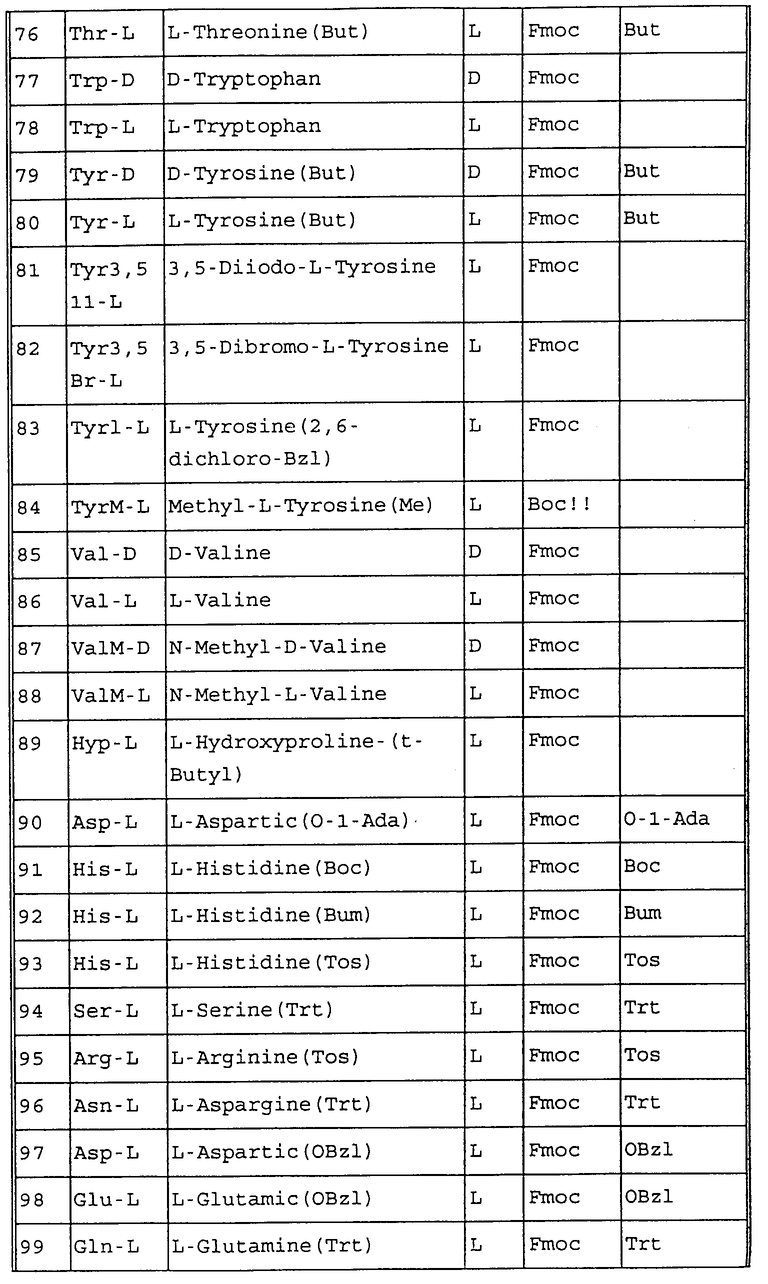

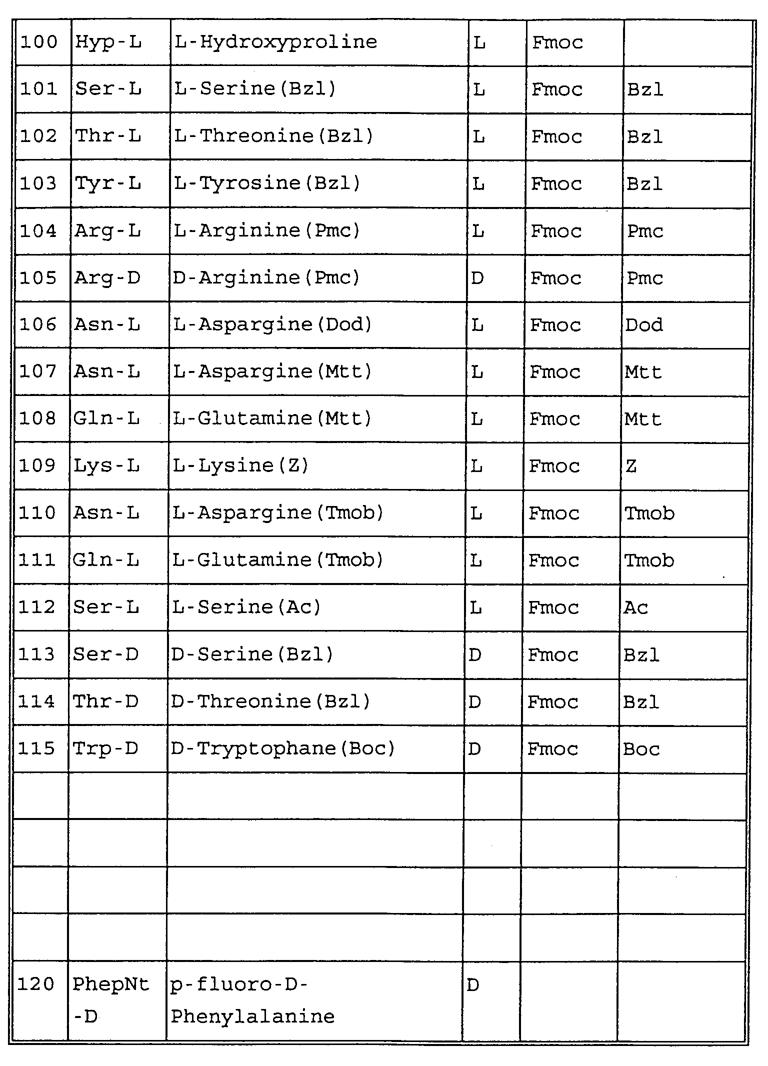

### General References:

Amato I. (1992) Speeding up a chemical game of chance. Science 257:330.
**Baumbach** G. A. and Hammond D. J. (1992) Protein Purification using affinity ligands deduced from peptide libraries. BioPharm May 1992, Page 24.
**Birnbaum** S and Mosbach K. (1992) Current Opinion in Biotechnology 3:49.
**Bischoff** S. C., Weck A. L. and Dahinden C. A. (1992) Peptide analogous of consensus receptor sequence inhibit the action of cytokines on human basophils. Lymphokine and cytokine research 11:33.
**Cwirla** S. E. et al. (1990) Peptides on phage: a vast library of peptides for identifying ligands. Proc. Natl. Acad. USA 87:6378.
**Devlin** J. J. et al. (1990) Random peptide libraries: a source of specific protein binding molecules. Science 249:404.
**Fodor**, P. A., Read, J. L., Pirrung, M.C., Stryer, L., Lu, A. T., and Solas, D. (1991). Light-directed spatially addressable parallel chemical synthesis. Science, 251,767.
**Furka**, A., Sebestyen F., Asgedon M. and Dibo G. (1988) Abst. 14th Int. Congr. Biochem. Prague, Czechoslovakia, Vol. 5, p.47.
**Furka**, A., Sebestyen F., Asgedon M. and Dibo G. (1991) General method for rapid synthesis of multicomponent peptide mixtures. Int. J. Peptide Protein Res. 37:487.
**Houghten** A.R., Pinilla, C., Blondelle, S. E., Appel, J. R., Dooley, C. T., and Cuervo, J. H. (1991). Generation and use of synthetic peptide combinatorial libraries for basic research and drug discovery. Nature, 354,84.
**Lam** K. S., Salmon, S.E., Hersh, E. M., Hruby, V.J., Kazmiesky, W. M., and Knapp R. J. (1991) A new type of synthetic peptide library for identifying ligand-binding activity. Nature 354:82.
**Magazine** H. I. and Johnson, H. M. (1991) Characterization of a synthetic peptide corresponding to a receptor binding domain of a mouse Interferon γ. Biochemistry 30:5784.
**Scott**, J. K., and Smith, G. P. (1990) Searching for peptide ligands with an epitope library. Science 249:386.

## Claims

1. A library of polymeric molecules, each consisting of a plurality of monomeric units, said library comprising a plurality of different families of different sequences of said monomeric units, said molecules being immobilized upon beads, each bead means carrying a plurality of different sequences belonging to one sequence family of the polymeric molecule, the expected amount of each such sequence on each beads being sufficient for detection of whether a molecule having that sequence will bind to a target of interest, each sequence comprising a familial portion and an individual portion, the familial portion having a lesser degree of diversity among the molecules carried by a single bead than among the molecules of the library as a whole, such familial portion thereby being sequenceable upon retrieval of the molecules carried by a single bead.

2. A method of constructing a library of polymeric molecules which may be synthesized by stepwise conjugation of monomeric or oligomeric reactants which comprises:
(a) providing a plurality of beads;
(b) in a plurality of synthetic cycles of which at least one is not a fully structured cycle, stepwise conjugating a monomeric or oligomeric reactant to said beads or to a nascent polymeric molecule thereon where for one or more "structured random" synthetic cycles the following steps are carried out,
(i) dividing the beads into N aliquots,
(ii) reacting each aliquot with one and only one of a set of N different predetermined monomeric or oligomeric reactants, where the value of N and the reactants of said set may be the same or different for each cycle, and (iii) pooling said reacted aliquots said synthetic cycles stepwise forming said molecules through the coupling of a reactant of one cycle to the reactant of another cycle.

3. The method of claim 2, further comprising one or more "structured random" synthetic cycles in each of which all beads are reacted with a single predetermined mixture of monomeric or oligomeric reactants, where said mixture may be the same or different for each such cycle.

4. The method of claim 3, further comprising one or more "nonrandom" synthetic cycles in which all beads are reacted with a purified reactant so as to introduce a constant element into said molecule.

5. A method of identifying polymeric molecules which bind to a target of interest which comprises:
(a) providing a first polymeric molecule library according to claim 1;
(b) contacting the first library with the target of interest, under conditions permitting the detection of the binding of the target to polymeric molecules carried by a bead of the libraries and selecting beads carrying polymeric molecules to which said target binds;
(c) determining at least the familial portion of the sequences of the polymeric molecules carried by a selected bead of the first library to which the target binds,
(d) providing a second polymeric molecule library according to claim 1 said second library being such that essentially all sequences expected to be carried on said selected bead of the first library are represented in the molecules of the second library, said second library essentially omitting sequences of the first library which were not expected to be carried on the selected bead;
(e) contacting the second library with the target of interest, under conditions permitting the detection of the binding of the target to molecules carried by a bead of the libraries and selecting bead carrying peptides to which said target binds;
(f) determining at least the familial portion of the sequences of molecules carried by a selected bead of the second library to which the target binds,
whereby a sequence corresponding to a target binding molecule of the first library which was carried by said first selected bead is further determined.

6. The library of claim 1 or the method of any of claims 2-5 wherein the library comprises at least 10⁷ beads.

7. The library or method of any of claims 1-6 wherein each bead carries at least 10³ molecules.

8. The library or method of any of claims 1-7 wherein the assay requires no more than 10⁷, more preferably no more than 10⁶, binding molecules per bead for the detection of binding to target, and the average sampling level per bead for the sequences on the bead is at least about equal to said peptide-per-bead detection limit.

9. The library or method of any of claims 1-8 wherein the assay requires no more than ten more preferably two, still more preferably one bead carrying target binding molecules for detection, and the ratio of the number of beads in the library to the library partitioning factor is at least about equal to said bead-per-library detection limit.

10. The library or method of any of claims 1-9 wherein the size of the library is at least 10⁸, more preferably at least 10¹⁰, still more preferably at least 10¹², and most preferable at least 10¹⁴ molecules.

11. The library or method of any of claims 1-10 wherein the diversity of the library is at least 10⁸, more preferably at least 10⁹, still more preferably at least 10¹⁰, and most preferably at least 10¹¹ unique sequences.

12. The library or method of any of claims 1-11 wherein during at least one random cycle at least forty different units are coupled to the nascent molecules of the library.

13. The library or method of any of claims 1-12 wherein during each random cycle at least forty different units are coupled to the nascent molecules of the library.

14. The library or method of any of claims 1 to 13 wherein the molecules are peptides, peptoids, nucleic acids or carbohydrates, preferably peptides.

15. The library or method of claims 1-14 in which the polymers have a length of at least five units.

16. The library or method of any of claims 1-14 in which the familial portion is one to four units in length.

17. The library or method of any of claims 1-16 in which the familial portion is identical for all molecules on a bead.

18. The library or method of any of claims 1-16 in which, at at least one monomer position in the familial portion, a more difficult-to-sequence monomer unit in a first molecule on a bead is paired with a less difficult-to-sequence monomer unit at the corresponding monomer position in a second molecule on the same bead.

19. A method of constructing a library of polymeric molecules which may be synthesized by stepwise conjugation of monomeric or oligomeric reactants which comprises
(a) providing a support having a surface which is dividable into a plurality of individually selectable zones,
(b) for one or more rounds,
(i) reacting a first selected zone of the surface of said support with a first selected monomeric or oligomeric reactant, so that said reactant is coupled with said support, or bound nascent polymeric molecules, essentially only within said first selected zone
(ii) reacting a second selected zone of the surface of said support to a second selected monomeric or oligomeric reactant, so that said reactant is coupled with said support, or bound nascent polymeric molecules, essentially only within said second selected zone, said first and second zones being nonoverlapping and said first and second reactants being different; and
(c) for one or more rounds, reacting the entire support surface with a mixture of two or more selected monomeric or oligomeric reactants.

20. The method of claim 19 in which a zone is selected by exposing the surface to radiation through mask means directing the radiation onto and only onto the selected zone, thereby activating the zone by removal of photolabile protecting groups from the irradiated zone.

## Patentansprüche

1. Bank von Polymermolekülen, die jeweils aus einer Vielzahl von Monomereinheiten bestehen, wobei die Bank eine Vielzahl verschiedener Familien von unterschiedlichen Sequenzen der Monomereinheiten umfaßt, und die Moleküle an Kügelchen immobilisiert sind, wobei jedes Kügelchen eine Vielzahl verschiedener Sequenzen trägt, die zu einer Sequenzfamilie der Polymermoleküle gehören, und die erwartete Menge einer jeden solchen Sequenz auf jedem Kügelchen für den Nachweis ausreichend ist, ob ein Molekül, das diese Sequenz besitzt, an ein Zielmolekül von Interesse bindet, wobei jede Sequenz einen familienspezifischen und einen individuellen Teil umfaßt, wobei der familienspezifische Teil einen geringeren Grad an Verschiedenartigkeit unter den Molekülen, die von einem einzelnen Kügelchen getragen werden, besitzt als unter den Molekülen der gesamten Bank, und ein solcher familienspezifischer Teil nach Wiedergewinnung der Moleküle, die von einem einzelnen Kügelchen getragen werden, dadurch sequenzierbar ist.

2. Verfahren zur Herstellung einer Bank von Polymermolekülen, die durch schrittweise Konjugation von monomeren oder oligomeren Reaktionspartnern synthetisiert werden können, das folgende Schritte umfaßt:
(a) Bereitstellung einer Vielzahl von Kügelchen;
(b) schrittweise Konjugation in einer Vielzahl von Synthesezyklen, von denen mindestens einer nicht ein voll strukturierter Zyklus ist, eines monomeren oder oligomeren Reaktionspartners an die Kügelchen oder an ein darauf entstehendes Polymermolekül, wobei für einen oder mehrere "strukturierte zufällige" Synthesezyklen die folgenden Schritte durchgeführt werden:
(i) Aufteilung der Kügelchen in N Aliquots;
(ii) Umsetzung eines jeden Aliquots mit einem und nur einem Satz von N verschiedenen vorbestimmten monomeren oder oligomeren Reaktionspartnern, wobei der Wert von N und der Reaktionspartner dieses Satzes gleich oder unterschiedlich für jeden Zyklus sein kann; und
(iii) Vereinigung dieser umgesetzten Aliquots, wobei die Synthesezyklen durch die Verknüpfung eines Reaktionspartners eines Zyklus mit einem Reaktionspartner eines anderen Zyklus diese Moleküle schrittweise erzeugen.

3. Verfahren nach Anspruch 2, das zusätzlich einen oder mehrere "strukturierte zufällige" Synthesezyklen umfaßt, in denen jeweils alle Kügelchen mit einem einzigen vorbestimmten Gemisch von monomeren oder oligomeren Reaktionspartnern umgesetzt werden, wobei das Gemisch in jedem solchen Zyklus gleich oder verschieden sein kann.

4. Verfahren nach Anspruch 3, das zusätzlich einen oder mehrere "nicht-zufällige" Synthesezyklen umfaßt, in denen alle Kügelchen mit einem gereinigten Reaktionspartner umgesetzt werden, wodurch ein konstantes Element in das Molekül eingeführt wird.

5. Verfahren zur Identifizierung von Polymermolekülen, die an ein Zielmolekül von Interesse binden, das folgende Schritte umfaßt:
(a) Bereitstellung einer ersten Polymermolekül-Bank nach Anspruch 1;
(b) Inkontaktbringen der ersten Bank mit dem Zielmolekül von Interesse unter Bedingungen, die den Nachweis der Bindung des Zielmoleküls an Polymermoleküle erlauben, die von einem Kügelchen der Banken getragen werden, und Auswählen von Kügelchen, die Polymermoleküle tragen, an die das Zielmolekül bindet;
(c) Bestimmung zumindest des familienspezifischen Teils der Sequenzen der Polymermoleküle, die von einem ausgewählten Kügelchen der ersten Bank getragen werden und an die das Zielmolekül bindet;
(d) Bereitstellung einer zweiten Polymermolekül-Bank nach Anspruch 1, wobei die zweite Bank derart beschaffen ist, daß im wesentlichen alle Sequenzen, die erwartungsgemäß von dem ausgewählten Kügelchen der ersten Bank getragen werden, in den Molekülen der zweiten Bank vorhanden sind, wobei die zweite Bank im wesentlichen die Sequenzen der ersten Bank nicht enthält, von denen nicht erwartet wurde, daß sie von dem ausgewählten Kügelchen getragen werden;
(e) Inkontaktbringen der zweiten Bank mit dem Zielmolekül von Interesse unter Bedingungen, die den Nachweis der Bindung des Zielmoleküls an Moleküle erlauben, die von einem Kügelchen der Banken getragen werden, und Auswählen der Kügelchen, die Peptide tragen, an die das Zielmolekül bindet;
(f) Bestimmung zumindest des familienspezifischen Teils der Sequenzen der Moleküle, die von einem ausgewählten Kügelchen der zweiten Bank getragen werden und an die das Zielmolekül bindet,
wobei eine Sequenz, die einem Molekül der ersten Bank entspricht, das das Zielmolekül bindet und von dem ersten ausgewählten Kügelchen getragen wurde, weiter bestimmt wird.

6. Bank nach Anspruch 1 oder Verfahren nach einem der Ansprüche 2 bis 5, wobei die Bank mindestens 10⁷ Kügelchen umfaßt.

7. Bank oder Verfahren nach einem der Ansprüche 1 bis 6, wobei jedes Kügelchen mindestens 10³ Moleküle trägt.

8. Bank oder Verfahren nach einem der Ansprüche 1 bis 7, wobei der Test nicht mehr als 10⁷, stärker bevorzugt nicht mehr als 10⁶ gebundene Moleküle pro Kügelchen für den Nachweis der Bindung an ein Zielmolekül benötigt, und die durchschnittliche Anzahl der Moleküle mit identischer Sequenz ("average sampling level") pro Kügelchen mindestens etwa der Peptid-pro-Kügelchen Nachweisgrenze entspricht.

9. Bank oder Verfahren nach einem der Ansprüche 1 bis 8, wobei der Test nicht mehr als zehn, stärker bevorzugt zwei, noch stärker bevorzugt ein Kügelchen benötigt, das für den Nachweis Moleküle trägt, die das Zielmolekül binden, und das Verhältnis der Anzahl von Kügelchen in der Bank zu dem Bank-Verteilungs-Faktor mindestens ungefähr der Kügelchen-pro-Bank-Nachweisgrenze entspricht.

10. Bank oder Verfahren nach einem der Ansprüche 1 bis 9, wobei die Bank mindestens 10⁸, stärker bevorzugt mindestens 10¹⁰, noch stärker bevorzugt mindestens 10¹² und am stärksten bevorzugt mindestens 10¹⁴ Moleküle enthält.

11. Bank oder Verfahren nach einem der Ansprüche 1 bis 10, wobei die Mannigfaltigkeit der Bank mindestens 10⁸, stärker bevorzugt mindestens 10⁹, noch stärker bevorzugt mindestens 10¹⁰ und am stärksten bevorzugt 10¹¹ einzigartige Sequenzen umfaßt.

12. Bank oder Verfahren nach einem der Ansprüche 1 bis 11, wobei während mindestens eines zufälligen Zyklus mindestens 40 verschiedene Einheiten an die entstehenden Moleküle der Bank gekoppelt werden.

13. Bank oder Verfahren nach einem der Ansprüche 1 bis 12, wobei während jedes zufälligen Zyklus mindestens 40 verschiedene Einheiten an die entstehenden Moleküle der Bank gekoppelt werden.

14. Bank oder Verfahren nach einem der Ansprüche 1 bis 13, wobei die Moleküle Peptide, Peptoide, Nucleinsäuren, oder Kohlenhydrate, vorzugsweise Peptide sind.

15. Bank oder Verfahren nach einem der Ansprüche 1 bis 14, wobei die Polymere eine Länge von mindestens fünf Einheiten haben.

16. Bank oder Verfahren nach einem der Ansprüche 1 bis 14, wobei der familienspezifische Teil ein bis vier Einheiten lang ist.

17. Bank oder Verfahren nach einem der Ansprüche 1 bis 16, wobei der familienspezifische Teil für alle Moleküle eines Kügelchens identisch ist.

18. Bank oder Verfahren nach einem der Ansprüche 1 bis 16, wobei, zumindest an einer Monomerposition in dem familienspezifischen Teil, eine Monomereinheit, die schwieriger zu sequenzieren ist, in einem ersten Molekül auf dem Kügelchen mit einer Monomereinheit, die weniger schwierig zu sequenzieren ist, an der entsprechenden Monomerposition in einem zweiten Molekül auf demselben Kügelchen gepaart wird.

19. Verfahren zur Konstruktion einer Bank von Polymermolekülen, die durch schrittweise Konjugation von monomeren oder oligomeren Reaktionspartnern synthetisiert werden können, das folgende Schritte umfaßt:
(a) Bereitstellung eines Trägers, der eine Oberfläche hat, die in eine Vielzahl von individuell auswählbaren Bereichen unterteilbar ist,
(b) für eine oder mehrere Runden
(i) Umsetzung eines ersten ausgewählten Bereiches der Oberfläche des Trägers mit einem ersten ausgewählten monomeren oder oligomeren Reaktionspartner, so daß der Reaktionspartner im wesentlichen nur innerhalb des ersten ausgewählten Bereiches an den Träger oder an gebundene entstehende Polymermoleküle gekoppelt wird,
(ii) Umsetzung eines zweiten ausgewählten Bereiches der Oberfläche des Trägers mit einem zweiten ausgewählten monomeren oder oligomeren Reaktionspartner, so daß der Reaktionspartner im wesentlichen nur innerhalb des zweiten ausgewählten Bereiches an den Träger oder an gebundene entstehende Polymermoleküle gekoppelt wird, wobei der erste und zweite Bereich nicht überlappend sind, und der erste und zweite Reaktionspartner unterschiedlich sind; und
(c) Umsetzung der gesamten Trägeroberfläche mit einem Gemisch von zwei oder mehreren ausgewählten monomeren oder oligomeren Reaktionspartnern für eine oder mehrere Runden.

20. Verfahren nach Anspruch 19, wobei ein Bereich dadurch ausgewählt wird, daß die Oberfläche einer Strahlung durch eine Maske ausgesetzt wird, die eine Bestrahlung nur des ausgewählten Bereiches zuläßt, wodurch der Bereich durch Entfernung von photolabilen Schutzgruppen aus dem bestrahlten Bereich aktiviert wird.

## Revendications

1. Bibliothèque de molécules polymériques, chacune se composant d'une pluralité d'unités monomères, ladite bibliothèque comprenant une pluralité de différentes familles de différentes séquences de ces unités monomères, lesdites molécules étant immobilisées sur des perles, chaque moyen de perle comportant une pluralité de différentes séquences appartenant à une famille de séquences de la molécule polymérique, la quantité attendue de chacune de ces séquences sur chacune des perles étant suffisante pour détecter si une molécule ayant cette séquence se liera à une cible d'intérêt, chaque séquence comprenant une partie familiale et une partie individuelle, la partie familiale ayant un degré de diversité moindre parmi les molécules supportées par une seule perle que parmi les molécules de la bibliothèque dans son ensemble, la partie familiale étant ainsi séquençable lors de la récupération des molécules supportées par une seule perle.

2. Procédé de construction d'une bibliothèque de molécules polymériques qui peuvent être synthétisées par une conjugaison graduelle de réactifs monomères ou oligomères, qui comprend :
(a) la présence d'une pluralité de perles;
(b) dans une pluralité de cycles de synthèse dont au moins un n'est pas un cycle totalement structuré, la conjugaison graduelle d'un réactif monomère ou oligomère à ces perles ou à une molécule polymérique naissante sur celles-ci, où pour un ou plusieurs cycles de synthèse "désordonnés structurés" les étapes suivantes sont réalisées : (i) la division des perles en N fractions égales, (ii) la réaction de chaque fraction égale avec un et seulement un d'une série de N réactifs monomères ou oligomères prédéterminés différents, où la valeur de N et les réactifs de cette série peuvent être identiques ou différents pour chaque cycle et (iii) le rassemblement de ces fractions mises en réaction, les cycles de synthèse susdits formant graduellement les molécules susdites par le couplage d'un réactif d'un cycle au réactif d'un autre cycle.

3. Procédé suivant la revendication 2, comprenant de plus un ou plusieurs cycles de synthèse "désordonnés structurés" dans chacun desquels toutes les perles sont mises en réaction avec un seul mélange prédéterminé de réactifs monomères ou oligomères, ledit mélange pouvant être identique ou différent pour chacun de ces cycles.

4. Procédé suivant la revendication 3, comprenant de plus un ou plusieurs cycles de synthèse "non désordonnés" dans lesquels toutes les perles sont mises en réaction avec un réactif purifié de manière à introduire un élément constant dans la molécule précitée.

5. Procédé d'identification de molécules polymériques qui se lent à une cible d'intérêt qui comprend :
(a) la présence d'une première bibliothèque de molécules polymériques suivant la revendication 1;
(b) la mise en contact de la première bibliothèque avec la cible d'intérêt, sous des conditions permettant la détection de la liaison de la cible aux molécules polymériques supportées par une perle des bibliothèques et la sélection des perles supportant les molécules polymériques auxquelles ladite cible se lie;
(c) la détermination d'au moins la partie familiale des séquences des molécules polymériques supportées par une perle sélectionnée de la première bibliothèque à laquelle la cible se lie,
(d) la présence d'une seconde bibliothèque de molécules polymériques suivant la revendication 1, cette seconde bibliothèque étant telle qu'essentiellement la totalité des séquences attendues comme devant être supportées sur ladite perle sélectionnée de la première bibliothèque sont représentées dans les molécules de la seconde bibliothèque, la seconde bibliothèque omettant essentiellement les séquences de la première bibliothèque qui n'étaient pas attendues comme devant être supportées sur la perle sélectionnée;
(e) la mise en contact de la seconde bibliothèque avec la cible d'intérêt, sous des conditions permettant la détection de la liaison de la cible aux molécules supportées par une perle des bibliothèques et la sélection de la perle supportant les peptides auxquels ladite cible se lie;
(f) la détermination d'au moins la partie familiale des séquences de molécules supportées par une perle sélectionnée de la seconde bibliothèque à laquelle la cible se lie,
de telle sorte qu'une séquence correspondant à une molécule liant la cible de la première bibliothèque qui était supportée par la première perle sélectionnée soit de plus déterminée.

6. Bibliothèque suivant la revendication 1 ou procédé suivant l'une quelconque des revendication 2 à 5, dans lequel la bibliothèque comprend au moins 10⁷ perles.

7. Bibliothèque ou procédé suivant l'une quelconque des revendications 1 à 6, dans lequel chaque perle comporte au moins 10³ molécules.

8. Bibliothèque ou procédé suivant l'une quelconque des revendications 1 à 7, dans lequel l'essai ne requiert pas plus de 10⁷, plus avantageusement pas plus de 10⁶ molécules de liaison par perle pour la détection de liaison à la cible et le niveau d'échantillonnage moyen par perle pour les séquences sur la perle est au moins environ égal à la limite de détection de peptides par perle précitée.

9. Bibliothèque ou procédé suivant l'une quelconque des revendications 1 à 8, dans lequel l'essai ne nécessite pas plus de dix, plus avantageusement deux encore plus avantageusement une perle comportant des molécules de liaison de cible pour la détection et le rapport du nombre de perles dans la bibliothèque au facteur de partage de bibliothèque est au moins environ égal à la limite de détection de perles par bibliothèque précitée.

10. Bibliothèque ou procédé suivant l'une quelconque des revendications 1 à 9, dans lequel la taille de la bibliothèque est d'au moins 10⁸, plus avantageusement d'au moins 10¹⁰, encore plus avantageusement d'au moins 10¹² et le plus avantageusement d'au moins 10¹⁴ molécules.

11. Bibliothèque ou procédé suivant l'une quelconque des revendications 1 à 10, dans lequel la diversité de la bibliothèque est d'au moins 10⁸, plus avantageusement d'au moins 10⁹, encore plus avantageusement d'au moins 10¹⁰ et le plus avantageusement d'au moins 10¹¹ séquences uniques.

12. Bibliothèque ou procédé suivant l'une quelconque des revendications 1 à 11, dans lequel pendant au moins un cycle désordonné au moins 40 unités différentes sont couplées aux molécules naissantes de la bibliothèque.

13. Bibliothèque ou procédé suivant l'une quelconque des revendications 1 à 12, dans lequel pendant chaque cycle désordonné au moins 40 unités différentes sont couplées aux molécules naissantes de la bibliothèque.

14. Bibliothèque ou procédé suivant l'une quelconque des revendications 1 à 13, dans lequel les molécules sont des peptides, des peptoïdes, des acides nucléiques ou des hydrates de carbone, avantageusement des peptides.

15. Bibliothèque ou procédé suivant l'une quelconque des revendications 1 à 14, dans lequel les polymères ont une longueur d'au moins cinq unités.

16. Bibliothèque ou procédé suivant l'une quelconque des revendications 1 à 14, dans lequel la partie familiale comporte une à quatre unités en longueur.

17. Bibliothèque ou procédé suivant l'une quelconque des revendications 1 à 16, dans lequel la partie familiale est identique pour toutes les molécules sur une perle.

18. Bibliothèque ou procédé suivant l'une quelconque des revendications 1 à 16, dans lequel, à au moins une position de monomère dans la partie familiale, une unité de monomère plus difficile à séquencer dans une première molécule sur une perle est appariée avec une unité de monomère moins difficile à séquencer à la position de monomère correspondante dans une seconde molécule sur la même perle.

19. Procédé de construction d'une bibliothèque de molécules polymériques qui peuvent être synthétisées par une conjugaison graduelle de réactifs monomères ou oligomères, qui comprend:
(a) la présence d'un support ayant une surface qui est divisable en une pluralité de zones sélectionnables individuellement,
(b) pour un ou plusieurs essais,
(i) la réaction d'une première zone sélectionnée de la surface dudit support avec un premier réactif monomère ou oligomère sélectionné, de telle sorte que ledit réactif soit couplé au support susdit ou à des molécules polymériques naissantes liées, essentiellement uniquement à l'intérieur de la première zone sélectionnée,
(ii) la réaction d'une seconde zone sélectionnée de la surface du support susdit à un second réactif monomère ou oligomère sélectionné, de telle sorte que ledit réactif soit couplé au support précité ou à des molécules polymériques naissantes liées, essentiellement uniquement à l'intérieur de la seconde zone sélectionnée, la première et la seconde zone ne se recouvrant pas et le premier et le second réactif étant différents; et
(c) pour un ou plusieurs essais, la réaction de la surface de support entière avec un mélange de deux ou plusieurs réactifs monomères ou oligomères sélectionnés.

20. Procédé suivant la revendication 19, dans lequel une zone est sélectionnée en exposant la surface à un rayonnement à travers un moyen formant masque dirigeant le rayonnement sur et uniquement sur la zone sélectionnée, en activant ainsi la zone par séparation des groupes de protection photolabiles de la zone irradiée.
